# EUROPEAN PATENT APPLICATION

(11) **EP 2 660 243 A2**
(43) Date of publication of application: **06.11.2013**
(21) Application number: 11853469.2
(22) Date of filing: 13.12.2011
(51) Int. Cl.: C07D 495/14, C07D 495/22, C07D 451/02, A61K 31/46, A61K 31/439, A61K 31/4365, A61P 25/24, A61P 25/28

(54) **SUBSTITUTED HYDROGENATED THIENO-PYRROLO[3,2-C]PYRIDINE, LIGANDS, A PHARMACEUTICAL COMPOSITION AND A METHOD FOR USING THE ABOVE**

(30) Priority: 27.12.2010 RU 2010153155
(71) Applicant: Ivachtchenko, Alexandre Vasilievich, Moskovskaya obl. 141700 (RU); Alla Chem, LLC., Carson City, NV 89701 (US)
(72) Inventor: MITKIN, Oleg Dmitrievich, Khimki Moskovskaya obl. 141400 (RU)
(74) Representative: Patentanwälte Zellentin & Partner
(86) International application number: PCT/RU2011/000980
(87) International publication number: WO 2012/091628

(57) **Abstract**

The present invention relates to novel substituted tetrahydro-4*H*-thieno-pyrrolo[3,2-c]pyridines of the general formula **1,** geometrical isomers, mixtures of geometrical isomers, and pharmaceutically acceptable salts thereof, 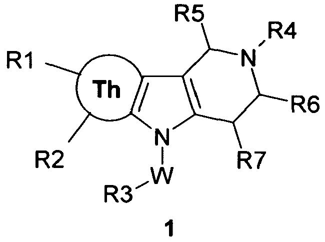 wherein:
**Th** represents annelated thienic cycle;
**W** represents odinary bond (in this case R3 is bound directly to N-atom of pyrrole cycle), methylene, 1,2-ethylene, 1,2-vinyl, 1,2-ethynylene, 1,3-propanediyl or 1,3-propenylene, optionally substituted with hydroxy group;
**R1** and **R2** represent hydrogen, C₁-C₄alkyl, halogen or -CH₂OH;
**R3** represents hydrogen, optionally substituted phenyl, optionally substituted azaheteroaryl;
**R4** represents C₁-C₄alkyl, CO₂C₂H₅ or CO₂C(CH₃)₃;
**R5, R6, R7** independently of each other represent hydrogen or C₁-C₄alkyl, or
**R5** and **R6** form together ethylene bridge, and R7 represents hydrogen, or R5 and **R7** form together ethylene bridge, and R6 represents hydrogen.

And also to synthesis of novel chemical compounds, novel physiologically active compounds, "molecular tools", to pharmaceutical composition, methods for preparation thereof and to method of treatment and prophylaxis of various diseases including diseases of central nervous system (CNS).

## Description

### Field of the invention

The present invention relates to synthesis of novel chemical compounds, searching for novel physiologically active compounds, leader-compounds, "molecular tools" ang drug candidates, and also to pharmaceutical composition, methods for preparation and use thereof.

### Background of the invention

The present invention relates to novel heterocyclic compounds, including templates 5,6,7,8-tetrahydro-4H-[2',3':4,5]pyrrolo[3,2-c]pyridine **A** and 4,5,6,7-tetrahydro-4H-[3',2':4,5]pyrrolo[3,2-c]pyridine **B,** pharmaceutically acceptable salts and/or hydrates thereof, to methods for their preparation, biologically active ligandes, "molecular tools", active components, pharmaceutical compositions, medicaments, and also to method for treatment and prophylaxis of various diseases, among them diseases of central nervous system (CNS).

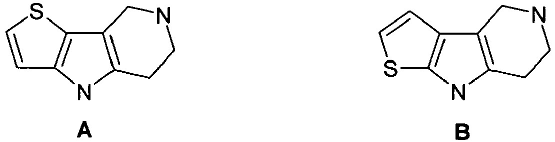

With the purpose of the development of novel biologically active compounds the authors of the invention carried out broad investigation in the field of synthesis of novel 5,6,7,8-tetrahydro-4H-thieno[2',3':4,5]pyrrolo[3,2-c]pyridines and 4,5,6,7-tetrahydro-4H-thieno[3',2':4,5]pyrrolo[3,2-c]pyridines, exhibiting a wide range of biological activity, comprising GPCR receptors (GPCR), ion channels and neurotransmitter transporters.

In literature the authors have not found examples of compounds including 4,5,6,7-tetrahydro-4H-thieno[3',2':4,5]perrolo[3,2-c]pyridine template **B,** and only one compound including template **A** has been described - ethyl 2,7-dimethyl-4,5,6,7-tetrahydro-4H-thieno[2',3':4,5]pyrrolo[3,2-c]pyridine-3-carboxylate **(A1**) [V.I.Shvedov, J.I. Trofimkin, V.K. Vasilieva. A.N. Griniev, "Functional derivates of thiophene", Khim.Geterotsikl.Soed.,1975, Nº10, 1324-1327]. Accordingly, any information concerning biological activity of these compounds is absent in scientific and patent literature.

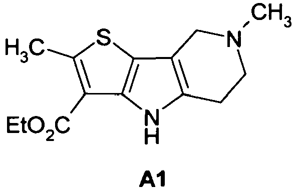

As a result of the accomplished investigations the inventors synthesized for the first time a large group of substituted 5,6,7,8-tetrahydro-4H-thieno[2',3':4,5]pyrrolo[3,2-c]pyridines and 4,5,6,7-tetrahydro-4H-thieno[3',2':4,5]pyrrolo[3,2-c]pyridines exhibiting physiological activity. The present invention relates to novel 5,6,7,8-tetrahydro-4H-thieno[2',3':4,5]pyrrolo[3,2-c]pyridines and 4,5,6,7-tetrahydro-4H-thieno[3',2':4,5]pyrrolo[3,2-c]pyridines; racemates, optical isomers, geometrical isomers, pharmaceutically acceptable salts and/or hydrates thereof, which represents one of the aspects of the present invention, to biologically active ligandes, "molecular tools", active components, pharmaceutical compositions, medicaments, and also to the method of treatment and prophylaxis of various CNS diseases.

### Disclosure of the invention

In context of the invention, terms are generally defined as follows:
**"Active component"** (drug-substance) means a physiologically active compound of synthetic or other (biotechnological, vegetable, animal, microbe and so on) origins exhibiting pharmacological activity which is an active ingredient of pharmaceutical composition employing in production and preparation of medicaments.
**"Aliphatic"** radical means a radical derived at removal of hydrogen atom from nonaromatic C-H bond. Aliphatic radical may additionally contain any substituens - aliphatic or aromatic radicals, the meanings of which are defined in this section. The representatives of aliphatic radicals include: alkyl, alkenyl, alkynyl, cycloalkyl, cycloalkenyl, heterocyclyl, heterocyclenyl, aralkenyl, aralkyloxyalkyl, aralkyloxycarbonylalkyl, aralkyl, aralkynyl, aralkyloxyalkenyl, heteroaralkenyl, heteroaralkyl, heteroaralkyloxyalkenyl, heteroaralkyloxyalkyl, heteroaralkenyl, annelated: arylcycloalkyl, heteroarylcycloalkyl, arylcycloalkenyl, heteroarylcycloalkenyl, arylheterocyclyl, heteroarylheterocyclyl, arylheterocyclenyl, annelated heteroarylheterocyclenyl.
**"Alkyl"** means an aliphatic hydrocarbon straight or branched chain with 1-12 carbon atoms. Branched means alkyl chain with one or more "lower alkyl" substituents. Alkyl group may have one or more substituents of the same or different structure ("alkyl substituent") including halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkoxycarbonyl, aralkoxycarbonyl, alkylthio, heteroarylthio, aralkylthio, arylsulfonyl, alkylsulfonylheteroaralkyloxy, annelated: heteroarylcycloalkenyl, heteroarylcycloalkyl, heteroarylheterocyclenyl, heteroarylheterocyclyl, arylcycloalkenyl, arylcycloalkyl, arylheterocyclenyl, heteroaralkyloxycarbonyl or Rₖ^{a}Rₖ₊₁^{a}N-, Rₖ^{a}Rₖ₊₁^{a}NC(=O)-, Rₖ^{a}Rₖ₊₁^{a}NC(=S)-, Rₖ^{a}Rₖ₊₁^{a}NSO₂-, where Rₖ^{a} and Rₖ₊₁^{a} independently of each other represent "amino group substituents" the meanings of which are defined in this section, for example, hydrogen, alkyl, aryl, aralkyl, heteroaralkyl, heterocyclyl or heteroaryl, or Rₖ^{a} and Rₖ₊₁^{a} together with the N-atom, they are attached to, form through Rₖ^{a} and Rₖ₊₁^{a} 4-7-membered heterocyclyl or heterocyclenyl. The preferred alkyl groups are methyl, trifluoromethyl, cyclopropylmethyl, cyclopentylmethyl, ethyl, *n*-propyl, *iso*-propyl, *n-*butyl, *tert*.-butyl, *n*-pentyl, 3-pentyl, methoxyethyl, carboxymethyl, methoxycarbonylmethyl, ethoxycarbonylmethyl, benzyloxycarbonylmethyl and pyridylmethyloxycarbonylmethyl. The preferred "alkyl substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, annelated arylheterocyclenyl, annelated arylheterocyclyl.
**"Aralkenyl"** means an aryl-alkenyl- group, for which the meanings of aryl and alkenyl are defined in this section. For example, 2-phenethyl is aralkenyl group.
**"Aralkyl"** means an alkyl group substituted with one or more aryl groups, for which the meanings of aryl and alkyl are defined in this section. Benzyl and 2,2-diphenylethyl are examples of aralkyl groups.
**"Aryl"** means an aromatic mono- or polycyclic system with 6-14 carbon atoms, predominantly 6-10 carbon atoms. Aryl may have one or more "cyclic system substituents" of the same or different structure. Phenyl, substituted phenyl, naphthyl, or substituted naphthyl are the representatives of aryl groups. Aryl could be annelated with nonaromatic cyclic system or heterocycle. The preferred aryl substituents are halogen, C₁-C₄alkyl, C₁-C₄alkoxy, CF₃, OCF₃.
**"Aromatic"** radical means a radical derived at removal of hydrogen atom from aromatic C-H bond. "Aromatic" radical implies aryl and heteroaryl cycles, the meaning of which are defined in this section. Aryl and heteroaryl cycles may additionally contain substituents, such as aliphatic and aromatic radicals, the meaning of which are defined in this section. Aryl, annelated cycloalkenylaryl, annelated cycloalkylaryl, annelated heterocyclylaryl, annelated heterocyclenylaryl, heteroaryl, annelated cycloalkylheteroaryl, annelated cycloalkenylheteroaryl, annelated heterocyclenylheteroaryl and annelated heterocyclylheteroaryl are the representatives of aromatic radicals.
**"1,2-Vinyl radical"** means -CH=CH- group, which comprises one or more "alkyl substituents" of the same or different structure, the meanings of which are defined in this section.
**"Halogen"** means fluorine, chlorine, bromine and iodine. Preference is given to fluorine, chlorine and bromine.
**"Heteroaryl"** means an aromatic mono- or polycyclic system with 5-14 carbon atoms, preferably from 5 to 10, wherein one or more carbon atoms are substituted by one or more heteroatoms, such as N, S or O. Prefix "aza", "oxa" or "thia" before "heteroaryl" means that N, O or S atoms are introduced in the appropriate cyclic fragment. N-Atom of heteroaryl cycle could be oxidized to N-oxide. Heteroaryl may have one or more "cyclic system sustituents" of the same or different structure. Pyrrolyl, furanyl, thienyl, pyridyl, pyrazinyl, pyrimidinyl, isoxazolyl, isothiazolyl, tetrazolyl, oxazolyl, thiazolyl, pyrazolyl, furazanyl, triazolyl, 1,2,4-thiadiazolyl, pyridazinyl, quinoxalinyl, phthalazinyl, imidazo[1,2-a]pyridinyl, imidazo[2,1-b]thiazolyl, benzofurazanyl, indolyl, azaindolyl, benzoimidazolyl, benzothiazenyl, quinolinyl, imidazolyl, thienopyridyl, quinazolinyl, thienopyrimidinyl, pyrrolopyridinyl, imidazopyridinyl, isoquinolinyl, benzoazaindolyl, 1,2,4-triazinyl, thienopyrrolyl, furopyrrolyl and others are the representatives of heteroaryl radicals. The preferred heteroaryl substituents are halogen, C₁-C₄alkyl, C₁-C₄alkoxy, CF₃, OCF₃.
**"Heterocyclenyl"** means a saturated monocyclic or polycyclic system with 3 - 13 carbon atoms, preferably from 5 to 13 carbon atoms, in which one or more carbon atoms are substituted by heteroatom such as N, O, or S, and which comprises at least one C=C double bond or C=N double bond. Prefix "aza", "oxa" or "thia" before "heterocyclenyl" means that N, O or S atoms are introduced in the cyclic system, respectively. Heterocyclenyl may have one or more "cyclic system substituents" of the same or different structure. N- and S- atoms of "heterocyclenyl" could be oxidized to N-oxide, S-oxide or S-dioxide. 1,2,3,4-Tetrahydropyridine, 1,2-dihydropyridine, 1,4-dihydropyridine, 2-pyrrolinyl, 3-pyrrolinyl, 2-imidazolyl, 2-pyrazolinyl, dihydrofuranyl, dihydrothiophenyl and others are the representatives of heterocyclenyls.
**"Heterocyclyl"** means an aromatic or saturated mono- or polycyclic system with 3-10 carbon atoms, preferably from 5 to 6, wherein one or more carbon atoms are substituted by one or more heteroatoms, such as N, S or O. Prefix "aza", "oxa" or "thia" before "heterocyclyl" means that N, O or S atoms are introduced in the cycle, respectively. Heterocyclyl may have one or more "cyclic system sustituents" of the same or different structure. Heterocyclyl may have one or more "cyclic system sustituents" of the same or different structure. N- and S- atoms of heterocyclic cycle could be oxidized to N-oxide, S-oxide or S-dioxide. Piperidinyl, pyrrolidinyl, piperazinyl, morpholinyl, thiomorpholinyl, thiazolidinyl, 1,4-dioxane-2-yl, tetrahydrofuranyl, tetrahydrothiophenyl and others are examples of heterocyclyl.
**"Hydrate"** means stoichiometric or nonstoichiometric compositions of compounds or their salts with water.
**"Hydroxyalkyl"** means HO-alkyl- group, for which alkyl is defined in this section.
**"Substituent"** means a chemical radical attached to a scaffold (fragment), for example, "alkyl substituent", "amino group substituent", "carbamoyl substituent", and "cyclic system substituent", meanings of which are defined in this section.
**"Amino group substituent"** means a substituent attached to amino group. Hydrogen, alkyl, cycloalkyl, aryl, heteroaryl, heterocyclyl, acyl, aroyl, alkylsulfonyl, arylsulfonyl, heteroarylsulfonyl, alkylaminocarbonyl, arylaminocarbonyl, heteroarylaminocarbonyl, heterocyclylaminocarbonyl, alkylaminothiocarbonyl, arylaminothiocarbonyl, heteroarylaminothiocarbonyl, heterocyclylaminothiocarbonyl, annelated heteroarylcycloalkenyl, annelated heteroarylcycloalkyl, annelated heteroarylheterocyclenyl, annelated heteroarylheterocyclenyl, annelated arylcycloalkenyl, annelated arylcycloalkyl, annelated arylheterocyclenyl, annelated arylheterocyclyl, alkoxycarbonylalkyl, aralkoxycarbonylalkyl, heteroaralkyloxycarbonylalkyl are amino group substituents. The meaning of "amino group substituents" is defined in this section.
**"Cyclic system substituent"** means a substituent attached to an aromatic or saturated cyclic system and implies hydrogen, alkylalkenyl, alkynyl, aryl, heteroaryl, aralkyl, heteroaralkyl, hydroxy, hydroxyalkyl, amino, aminoalkyl, alkoxy, aryloxy, acyl, aroyl, halogen, nitro, cyano, carboxy, alkoxycarbonyl, aryloxycarbonyl, aralkoxycarbonyl, alkyloxyalkyl, aryloxyalkyl, heterocyclyloxyalkyl, arylalkyloxyalkyl, heterocyclylalkyloxyalkyl, alkylsulfonyl, arylsulfonyl, heterocyclylsulfonyl, alkylsulfinyl, arylsulfinyl, heterocyclylsulfinyl, alkylthio, arylthio, heterocyclylthio, alkylsulfonylalkyl, arylsulfonylalkyl, heterocyclylsulfonylalkyl, alkylsulfinylalkyl, arylsulfinylalkyl, heterocyclylsulfinylalkyl, alkylthioalkyl, arylthioalkyl, heterocyclylthioalkyl, arylalkylsulfonylalkyl, heterocyclylalkylsulfonylalkyl, arylalkylthioalkyl, heterocyclylalkylthioalkyl, cycloalkyl, cycloalkenyl, heterocyclyl, heterocyclenyl, amidino, Rₖ^{a}Rₖ₊₁^{a}N-, Rₖ^{a}N=, Rₖ^{a}Rₖ₊₁^{a}N-alkyl-, Rₖ^{a}Rₖ₊₁^{a}NC(=O)- or Rₖ^{a}Rₖ₊₁^{a}NSO₂-, where Rₖ^{a} and Rₖ₊₁^{a} represent independently of each other "amino group substituents" the meanings of which are defined in this section, for example, hydrogen, optionally substituted alkyl, optionally substituted aryl, optionally substituted aralkyl, or optionally substituted heteroaralkyl, or a substituent Rₖ^{a}Rₖ₊₁^{a}N-, in which Rₖ^{a} may be acyl or aroyl, the meaning of Rₖ₊₁^{a} is defined above, or "cyclic system substituent" represent Rₖ^{a}Rₖ₊₁^{a}NC(=O)- or Rₖ^{a}Rₖ₊₁^{a}NSO₂-, in which Rₖ^{a} and Rₖ₊₁^{a} together with the N-atom they are attached to form through Rₖ^{a} and Rₖ₊₁^{a} 4-7 membered hererocyclyl or heterocyclenyl.
**"Protective group" (PG)** means a chemical radical attached to a scaffold or synthetic intermediate for temporary protection of amino group in multifunctional compounds, including, but not limited to: amide substituent, such as formyl, optionally substituted acetyl (for example, trichloroacetyl, trifluoroacetyl, 3-phenylpropionyl and others), optionally substituted benzoyl and others; carbamate substituent, such as optionally substituted C₁-C₇-alkoxycarbonyl, for example, methyloxycarbonyl, ethyloxycarbonyl, *tert*.-butyloxycarbonyl, 9-fluorenylmethyloxycarbonyl (Fmoc) and others; optionally substituted C₁-C₇-alkyl substituent, for example, *tert*.-butyl, benzyl, 2,4-dimethoxybenzyl, 9-phenylfluorenyl and others; sulfonyl substituent, for example, benzenesulfonyl, p-toluenesulfonyl and others. More specifically "Protective groups" are described in the book: Protective groups in organic synthesis, Third Edition, Green, T.W. and Wuts, P.G.M. 1999, p.494-653. Jon Wiley & Sons, Inc., New York, Chichester, Weinheim, Brisbane, Toronto, Singapore.
**"Inert substituent"** ("non-interfering substituent") means a low- or non-reactive radical, including, but not limited to: C₁-C₇ alkyl, C₂-C₇ alkenyl, C₂-C₇ alkynyl, C₁-C₇ alkoxy, C₇-C₁₂ aralkyl, substituted by inert substituents aralkyl, C₇-C₁₂ heterocyclylalkyl, substituted by inert substituents heterocyclylalkyl, C₇-C₁₂ alkaryl, C₃-C₁₀ cycloalkyl, C₃-C₁₀ cycloalkenyl, phenyl, substituted phenyl, toluyl, xylenyl, biphenyl, C₂-C₁₂ alkoxyalkyl, C₂-C₁₀ alkylsulfinyl, C₂-C₁₀ alkylsulfonyl, (CH₂)ₘ-O-(C₁-C₇ alkyl), -(CH₂)ₘ-N(C₁-C₇ alkyl)ₙ, aryl; aryl substituted by halogen or inert substituent; alkoxy group substituted by inert substituent; fluoroalkyl, aryloxyalkyl, heterocyclyl; heterocyclyl substituted by inert substituents and nitroalkyl; where m and n are varied from 1 to 7. C₁-C₇ Alkyl, C₂-C₇ alkenyl, C₂-C₇ alkynyl, C₁-C₇ alkoxy, C₇-C₁₂ aralkyl, C₇-C₁₂ alkaryl, C₃-C₁₀ cycloalkyl, C₃-C₁₀ cycloalkenyl, C₁-C₇ alkyl substituted by inert substituents, phenyl; phenyl substituted by inert substituents; (CH₂)ₘ-O-(C₁-C₇ alkyl), aryl; aryl substituted by inert substituents, heterocyclyl and heterocyclyl substituted by inert substituents are the preferred inert substituents.
**"Ligand"** (from Latin *ligo*) represents a chemical compound (small molecule, peptide, protein, inorganic ion, and so on) capable to interact with receptors which convert this interaction into specific signal.
**"Methylene"** radical means -CH₂- group, which comprises one or more "alkyl substituents" of the same or different structure, the meanings of which are defined in this section.
**"Lower alkyl"** means straight or branched alkyl with 1-4 carbon atoms.
**"1,3-Propylene"** radical means -CH₂-CH₂-CH₂- group, which comprises one or more "alkyl substituents" of the same or different structure, the meanings of which are defined in this section.
**"Template"** means the general structural formula of the group of compounds or compounds composing "combinatorial library".
**"Therapeutic kit"** is a simultaneously administered combination of two or more drug substances with different mechanism of pharmacological action and aimed at different biotargets taking part in pathogenesis of the disease.
**"Cycloalkyl"** means saturated monocyclic or polycyclic system with 3-10 carbon atoms. Cycloalkyl may have one or more "cyclic system substituents" of the same or different structure. Cyclopropyl, cyclobutyl, cyclopentyl, cyclohexyl, decalinyl, norbornyl, adamant-1-yl and others are the representatives of cycloalkyl groups. Cycloalkyl could be annelated with aromatic cycle or heterocycle. Alkyl, aralkoxy, hydroxy or Rₖ^{a}Rₖ₊₁^{a}N- are preferred "cyclic system substituents", the meanings of which are defined in this section.
**"Pharmaceutical composition"** means a composition comprising compound of the general formula I and at least one of components selected from the group consisting of pharmaceutically acceptable and pharmacologicaly compatible fillers, solvents, diluents, auxiliary, distributing and sensing agents, delivery agents, such as preservatives, stabilizers, disintegrators, moisteners, emulsifiers, suspending agents, thickeners, sweeteners, flavouring agents, aromatizing agents, antibacterial agents, fungicides, lubricants, and prolonged delivery controllers, choice and suitable proportions of which depend on nature and way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and their mixtures as well. Protection against action of microorganisms can be provided by various antibacterial and antifungal agents, such as, for example, parabens, chlorobutanole, sorbic acid, and similar compounds. Composition may also contain isotonic agents, such as, for example, sugar, sodium chloride, and similar compounds. Prolonged effect of composition may be achieved by agents slowing down absorption of active ingredient, for example, aluminum monostearate and gelatine. Examples of suitable carriers, solvents, diluents and delivery agents include water, ethanol, polyalcohols and their mixtures, natural oils (such as olive oil) and organic esters (such as ethyl oleate) for injections. Examples of fillers are lactose, milk-sugar, sodium citrate, calcium carbonate, calcium phosphate and the like. Examples of disintegrators and distributors are starch, alginic acid and its salts, and silicates. Examples of suitable lubricants are magnesium stearate, sodium lauryl sulfate, talc and polyethylene glycol of high molecular weight. Pharmaceutical composition for peroral, sublingval, transdermal, intramuscular, intravenous, subcutaneous, local or rectal administration of active ingredient, alone or in combination with another active compound may be administered to humans and animals in standard administration form, or in mixture with traditional pharmaceutical carriers. Suitable standard administration forms include peroral forms such as tablets, gelatin capsules, pills, powders, granules, chewing-gums and peroral solutions or suspensions, for example, therapeutic kit; sublingval and transbuccal administration forms; aerosols; implants; local, transdermal, subcutaneous, intramuscular, intravenous, intranasal or intraocular forms and rectal administration forms.
**"Pharmaceutically acceptable salt"** means relatively nontoxic both organic and inorganic salts of acids and bases disclosed in this invention. Salts could be prepared **in situ** in processes of synthesis, isolation or purification of compounds or they could be prepared specially. In particular, salts of bases could be prepared from purified base of the disclosed compound and suitable organic or mineral acid. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, *p*-toluenesulfonates, citrates, maleates, fumarates, succinates, tartrates, methane sulphonates, malonates, salicylates, propionates, ethane sulphonates, benzene sulfonates, sulfamates and the like (Detailed description of such salts properties is given in: Berge S.M., et al., "Pharmaceutical Salts" J.Pharm.Sci., 1977, 66: 1-19). Salts of the disclosed acids may also be prepared by the reaction of purified acids specifically with suitable base; moreover, metal salts and amine salts may be synthesized too. Metal salts are salts of sodium, potassium, calcium, barium, magnesium, lithium and aluminum, sodium and potassium salts being preferred. Suitable inorganic bases from which metal salts can be prepared are sodium hydroxide, carbonate, bicarbonate and hydride; potassium hydroxide, carbonate and bicarbonate, lithium hydroxide, calcium hydroxide, magnesium hydroxide, zinc hydroxide. Organic bases suitable for preparation of the disclosed acid salts are amines and amino acids of sufficient basicity to produce stable salt suitable for medical purposes use (in particular, they are to have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using some tetraalkylammonium hydroxides, such as holine, tetramethylammonium, tetraethylammonium, and the like. Aminoacids may be selected from the main aminoacids - lysine, ornithine and agrinine.
**"Fragment"** (scaffold) means a structural formula of the part of a molecule characteristic of group of compounds or molecular framework characteristic of group of compounds or compounds composing "combinatorial library".
**"1,2-Ethylene radical"** means -CH₂-CH₂- group carrying one or more the same or different "alkyl substituents" the meaning of which are defined in this section.

The purpose of the present invention is novel substituted hydrogenated thieno-pyrrolo[3,2-c]pyridines exhibiting biological activity.

The purpose in view is achieved by substituted tetrahydro-4H-thieno-pyrrolo[3,2-c]pyridines of the general formula 1, geometrical isomers, mixtures of geometrical isomers, and pharmaceutically acceptable salts thereof, 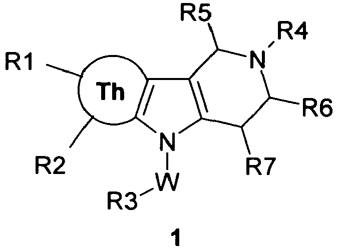 wherein:
**Th** represents annelated thienic cycle;
**W** represents ordinary bond (in this case R3 is bound directly to N-atom of pyrrole cycle), methylene, 1,2-ethylene, 1,2-vinyl, 1,2-ethynylene, 1,3-propanediyl or 1,3-propenylene, optionally substituted with hydroxy group;
**R1** and **R2** represent hydrogen, C₁-C₄alkyl, halogen or CH₂OH;
**R3** represents hydrogen, optionally substituted phenyl or optionally substituted azaheteroaryl;
**R4** represents C₁-C₄alkyl, CO₂C₂H₅ or CO₂C(CH₃)₃;
**R5, R6, R7** independently of each other represent hydrogen or C₁-C₄alkyl, or
**R5** and **R6** form together ethylene bridge, and R7 represents hydrogen, or
**R5** and **R7** form together ethylene bridge, and R6 represents hydrogen.

The preferred tetrahydro-4H-thieno-pyrrolo[3,2-c]pyridines are substituted 5,6,7,8-tetrahydro-4*H*-[2',3':4,5]pyrrolo[3,2-c]pyridines of the general formula **2** and substituted 4,5,6,7-tetrahydro-5*H*-[3',2':4,5]pyrrolo[3,2-c]pyridines of the general formula **3,** 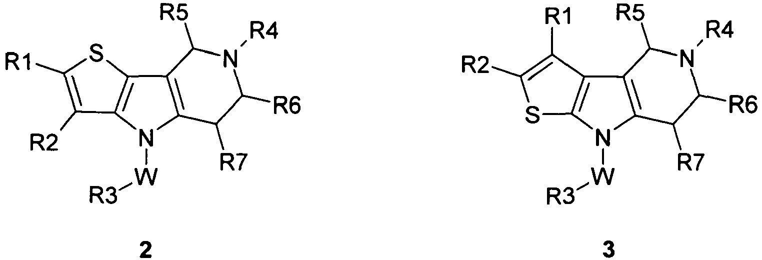 wherein:
**R1, R2, R3, R4, R5, R6, R7** and **W** have the above meanings.

The preferred tetrahydro-4H-thieno-pyrrolo[3,2-c]pyridines are compounds of the general formulas **1.1-1.14, 2.1-2.14, 3.1-3.14** 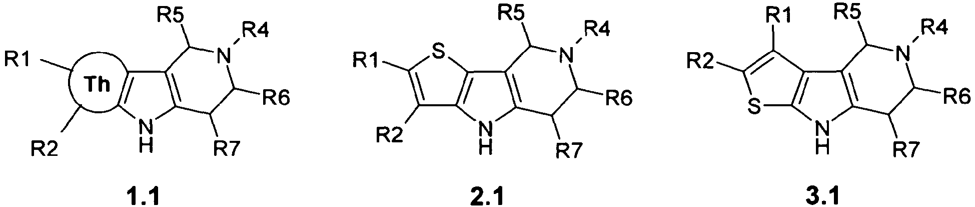 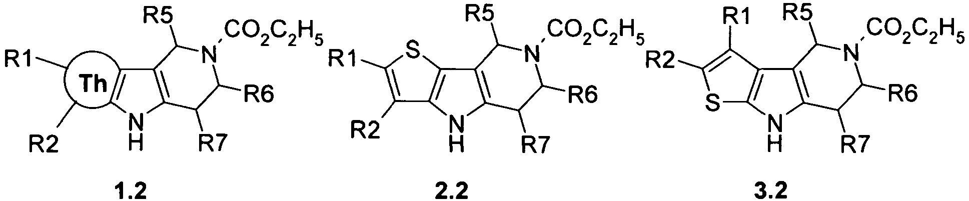 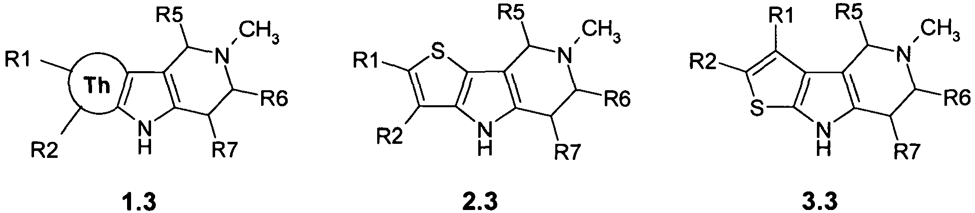 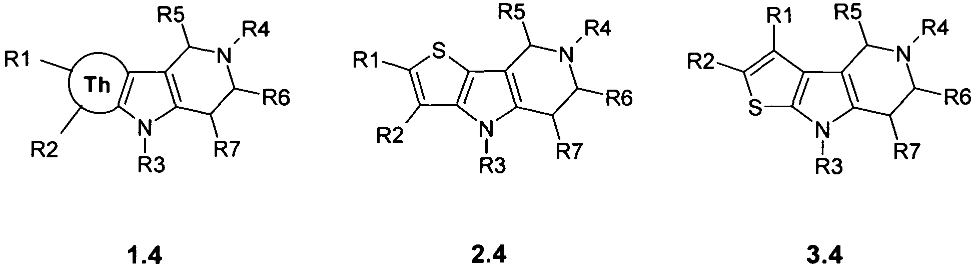 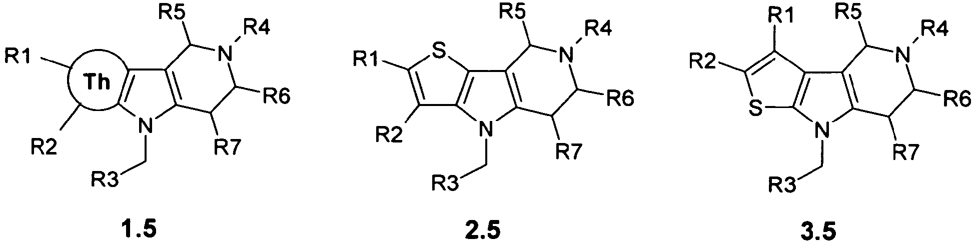 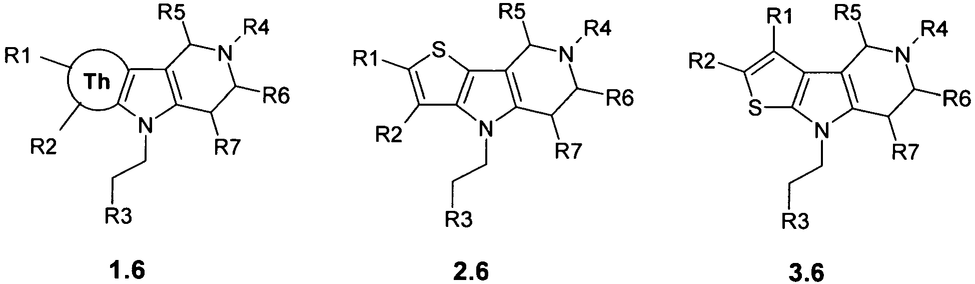 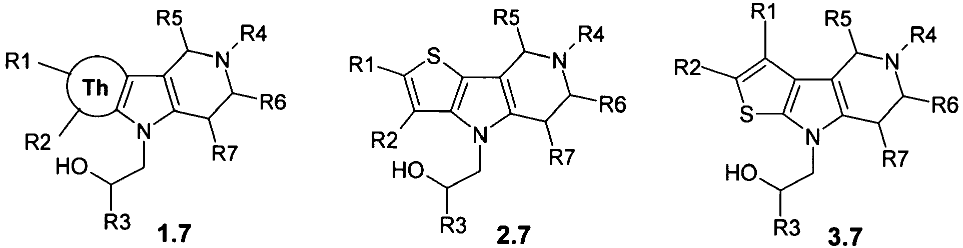 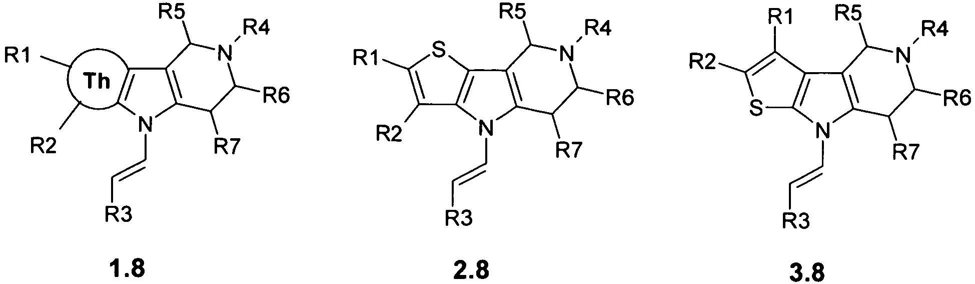 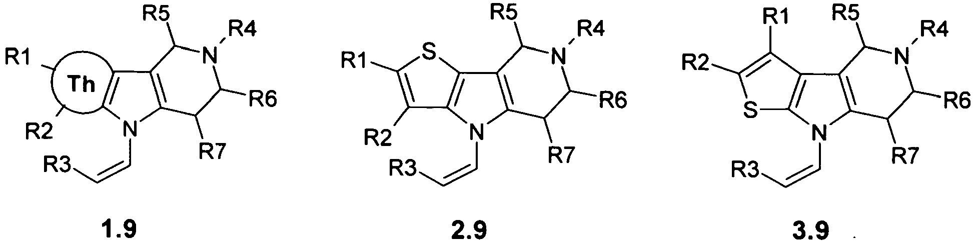 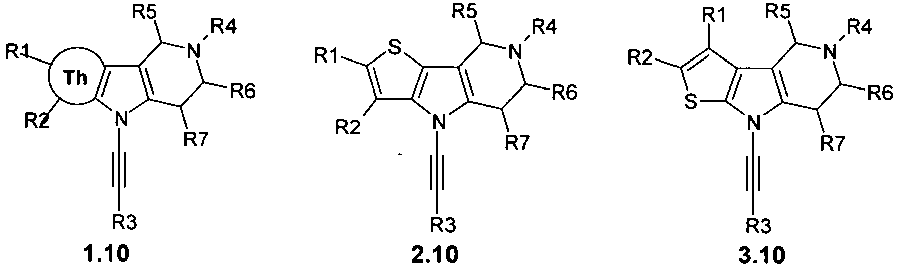 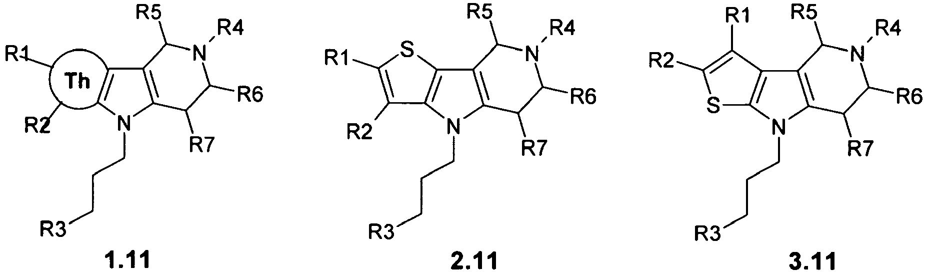 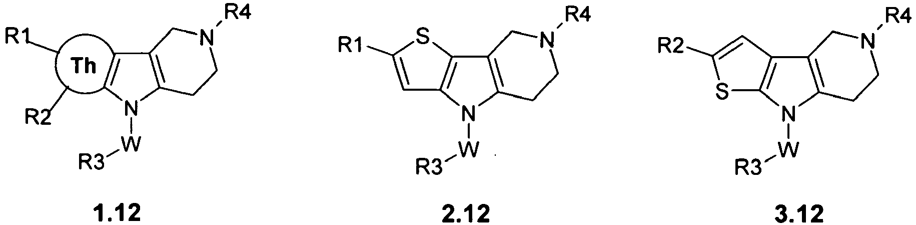 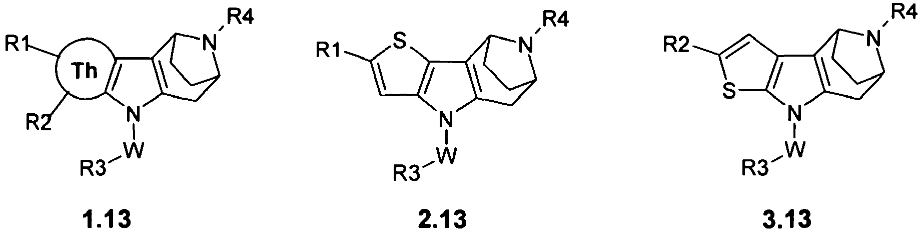 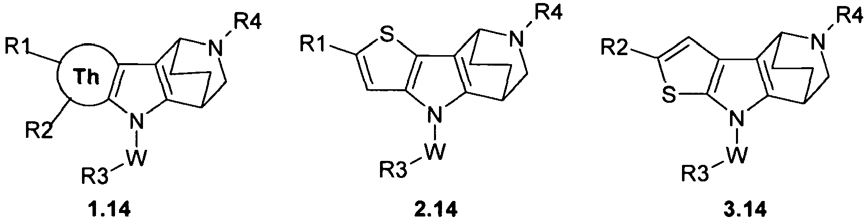 wherein:
**R1, R2, R3, R4, R5, R6, R7** and **W** have the above meanings.

The more preferable 5,6,7,8-tetrahydro-4*H*-thieno[2',3':4,5]pyrrolo[3,2-c]pyridines are the compounds selected from the group consisting of:
2,7-dimethyl-4-(pyridyl-4-yl)-5,6,7,8-tetrahydro-4H-thieno[2',3':4,5]pyrrolo[3,2-c]pyridine **2.4(1**),
4-benzyl-2,7-dimethyl-5,6,7,8-tetrahydro-4H-thieno[2',3':4,5]pyrrolo[3,2-c]pyridine **2.5(1**),
2,7-dimethyl-4-phenethyl-5,6,7,8-tetrahydro-4H-thieno[2',3':4,5]pyrrolo[3,2-c]pyridine **2.6(1),**
(7-methyl-4-phenethyl-5,6,7,8-tetrahydro-4H-thieno[2',3':4,5]pyrrolo[3,2-c]pyridine-2-yl)-methanol **2.6(2),**
2,7-dimethyl-4-(3-fluorophenyl)-5,6,7,8-tetrahydro-4H-thieno[2',3':4,5]pyrrolo[3,2-c]pyridine **2.4(3),**
4-(3-methylbenzyl)-3-methyl-7-benzyl-5,6,7,8-tetrahydro-4H-thieno[2',3':4,5]pyrrolo[3,2-c]pyridine **2.5(3),**
3,5,7-trimethyl-4-[2-(pyridin-3-yl)ethyl]-5,6,7,8-tetrahydro-4H-thieno[2',3':4,5]pyrrolo[3,2-c]pyridine **2.6(4),**
2-(2,7-dimethyl-5,6,7,8-tetrahydro-4H-thieno[2',3':4,5]pyrrolo[3,2-c]pyridin-4-yl)-1-phenyl-ethanol **2.7(1),**
(*E*)-2,7-dimethyl-4-styryl-5,6,7,8-tetrahydro-4H-thieno[2',3':4,5]pyrrolo[3,2-c]pyridine **2.8(1),**
(Z)-2,7-dimethyl-4-styryl-5,6,7,8-tetrahydro-4H-thieno[2',3':4,5]pyrrolo[3,2-c]pyridine **2.9(1),**
2-(2,5,7-trimethyl-5,6,7,8-tetrahydro-4H-thieno[2',3':4,5]pyrrolo[3,2-c]pyridin-4-yl)-1-(6-methylpyridin-3-yl)-ethanol **2.7(3),**
(*E*)-2-methyl-7-ethyl-4-(3-fluorostyryl)-5,6,7,8-tetrahydro-4H-thieno[2',3':4,5]pyrrolo[3,2-c]pyridine **2.8(3),**
(Z)-2-methyl-7-ethyl-4-(3-fluorostyryl)-5,6,7,8-tetrahydro-4H-thieno[2',3':4,5]pyrrolo[3,2-c]pyridine **2.9(3),**
2,7-dimethyl-4-phenylethyl-5,6,7,8-tetrahydro-4H-thieno[2',3':4,5]pyrrolo[3,2-c]pyridine **2.10(1),**
2-methyl-7-(3-fluorobenzyl)-4-cinnamyl-5,6,7,8-tetrahydro-4H-thieno[2',3':4,5]pyrrolo[3,2-c]pyridine **2.11(1)**
(*E*)-3,7-dimethyl-4-[3-(*π*-tolyl)allyl]-5,6,7,8-tetrahydro-4H-thieno[2',3':4,5]pyrrolo[3,2-c]pyridine **2.11(2),**
(*E*)-2,7-dimethyl-4-[3-(3-chlorophenyl)allyl]-5,6,7,8-tetrahydro-4H-thieno[2',3': 4,5]pyrrolo[3,2 -c]pyridine **2.11(3),**
2,7-dimethyl-4-(3-phenylpropyl)-5,6,7,8-tetrahydro-4H-thieno[2',3':4,5]pyrrolo[3,2-c]pyridine **2.12(1**),
3,7-dimethyl-4-[3-(6-methylpyridin-3-yl)propyl)]-5,6,7,8-tetrahydro-4H-thieno[2',3': 4,5]pyrrolo[3,2 -c]pyridine **2.12(3),**
2,6,7,8-tetramethyl-4-[3-(3-chlorophenyl)propyl]-5,6,7,8-tetrahydro-4H-thieno[2',3': 4,5]pyrrolo[3,2-c]pyridine **2.12(4),**
7-methyl-4-(pyridin-4-yl)-5,6,7,8-tetrahydro-4H-thieno[2',3':4,5]pyrrolo[3,2-c]pyridine **2.4(2),**
4-benzyl-7-methyl-2-chloro-5,6,7,8-tetrahydro-4H-thieno[2',3':4,5]pyrrolo[3,2-c]pyridine **2.5(2),**
7-methyl-4-phenethyl-2-chloro-5,6,7,8-tetrahydro-4H-thieno[2',3':4,5]pyrrolo[3,2-c]pyridine **2.6(3),**
2-(7-methyl-2-chloro-5,6,7,8-tetrahydro-4H-thieno[2',3':4,5]pyrrolo[3,2-c]pyridin-4-yl)-1-phenyl-ethanol **2.7(2),**
7-methyl-4-*π*-tolyl-2-chloro-5,6,7,8-tetrahydro-4H-thieno[2',3':4,5]pyrrolo[3,2-c]pyridine **2.4(4),**
4-(6-methylpyridin-3-ylmethyl)-6,7,8-trimethyl-3-chloro-5,6,7,8-tetrahydro-4H-thieno[2',3': 4,5]pyrrolo[3,2-c]pyridine **2.5(4),**
7-methyl-4-[2-(pyridin-4-yl)ethyl]-2-chloro-5,6,7,8-tetrahydro-4H-thieno[2',3': 4,5]pyrrolo[3,2-c]pyridine **2.6(5),**
2-(5,7-dimethyl-2-chloro-5,6,7,8-tetrahydro-4H-thieno[2',3':4,5]pyrrolo[3,2-c]pyridin-4-yl)-1-(3-chlorophenyl)-ethanol **2.7(4),**
(*E*)-7-methy)-4-styryl-2-chloro-5,6,7,8-tetrahydro-4H-thieno[2',3':4,5]pyrrolo[3,2-c]pyridine **2.8(2),**
(*Z*)-7-methyl-4-styryl-2-chloro-5,6,7,8-tetrahydro-4H-thieno[2',3':4,5]pyrrolo[3,2-c]pyridine **2.9(2),**
7-methyl-4-phenylethynyl-2-chloro-5,6,7,8-tetrahydro-4H-thieno[2',3':4,5]pyrrolo[3,2-c]pyridine **2.10(2),**
(*E*)-7-methyl-4-[3-(*m*-tolyl)allyl]-3-chloro-5,6,7,8-tetrahydro-4H-thieno[2',3':4,5]pyrrolo[3,2-c]pyridine **2.11(4),**
(*E*)-7-methyl-4-(3-methylstyryl)-2-chloro-5,6,7,8-tetrahydro-4H-thieno[2',3':4,5]pyrrolo[3,2-c]pyridine **2.8(4),**
(*Z*)-7-methyl-4-(3-methylstyryl)-2-chloro-5,6,7,8-tetrahydro-4H-thieno[2',3':4,5]pyrrolo[3,2-c]pyridine **2.9(4),**
6,7,8-trimethyl-4-[(3-fluorophenyl)ethynyl]-2-chloro-5,6,7,8-tetrahydro-4H-thieno[2',3':4,5]pyrrolo[3,2-c]pyridine **2.10(3),**
(*E*)-7-methyl-4-[3-(*p*-tolyl)allyl]-2-chloro-5,6,7,8-tetrahydro-4H-thieno[2',3':4,5]pyrrolo[3,2-c]pyridine **2.11(5),**
7-methyl-4-(3-phenylpropyl)-2-chloro-5,6,7,8-tetrahydro-4H-thieno[2',3':4,5]pyrrolo[3,2-c] pyridine **2.12(2),**
7-methyl-4-[3-(6-methylpyridin-3-yl)propyl]-3-chloro-5,6,7,8-tetrahydro-4H-thieno[2',3': 4,5]pyrrolo[3,2-c]pyridine **2.12(5),**
7-methyl-4-[3-(3-fluorophenyl)propyl]-2-chloro-5,6,7,8-tetrahydro-4H-thieno[2',3': 4,5]pyrrolo[3,2-c]pyridine **2.12(6),**
2,10-dimethyl-4-(pyridin-3-ylmethyl)-4,5,6,7,8,9-hexahydro-6,9-epiminocyclohepta[b]thieno[2,3-d]pyrrole **2.13(1),**
9-benzyl-4-(3-fluorobenzyl)-2-methyl-5,6,7,8,-tetrahydro-4H-8,5-(epiminomethano)thieno[3,2-b]indole **2.14(1),**
2-chloro-9-methyl-4-(2-phenylethyl)-5,6,7,8-tetrahydro-4H-8,5-(epiminomethano)thieno[3,2-b]indole **2.14(2),**

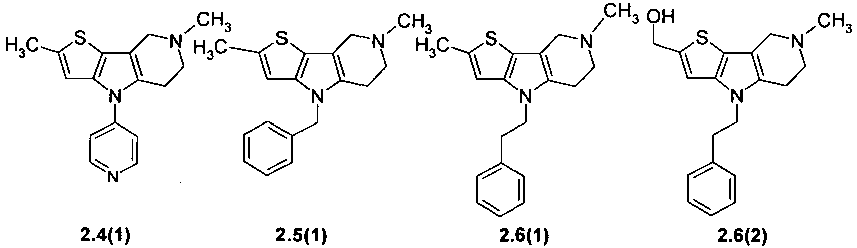

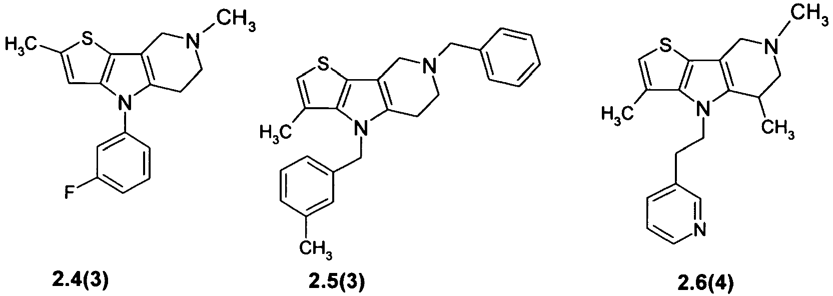

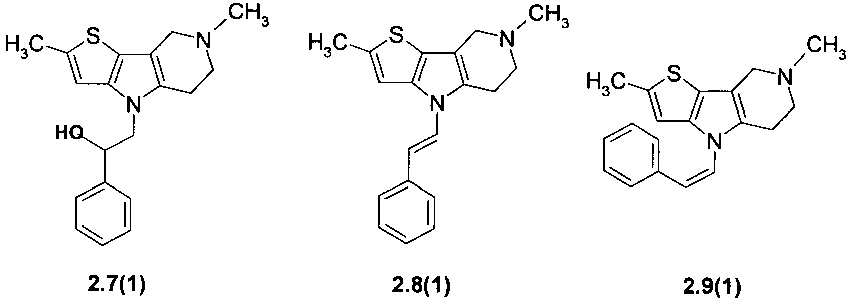

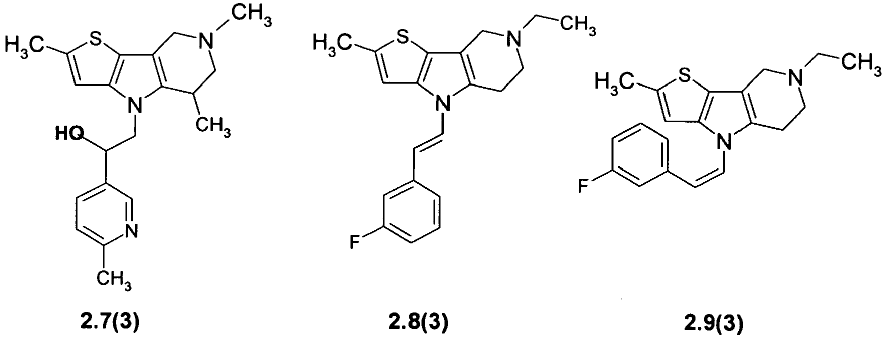

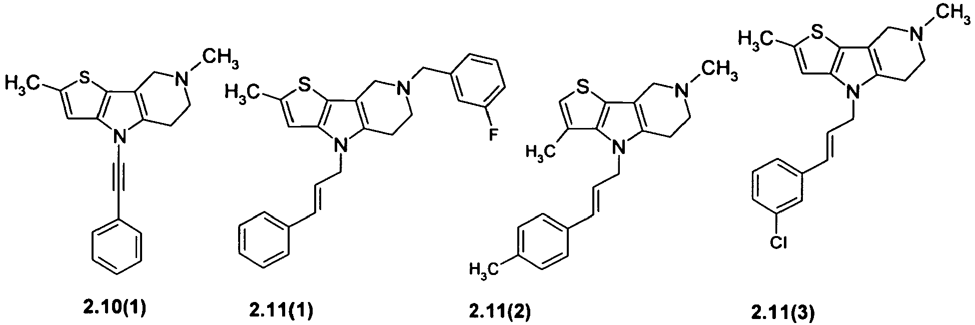

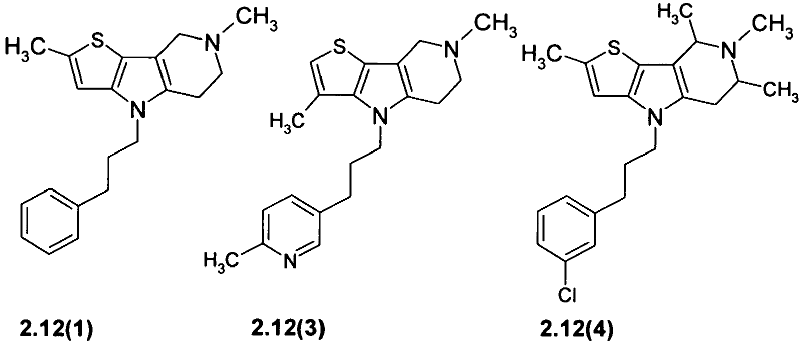

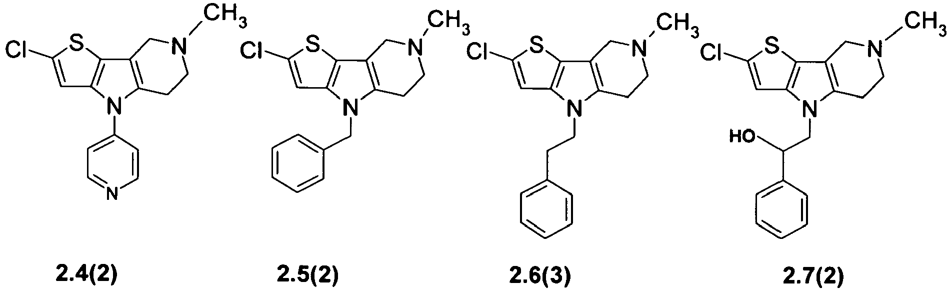

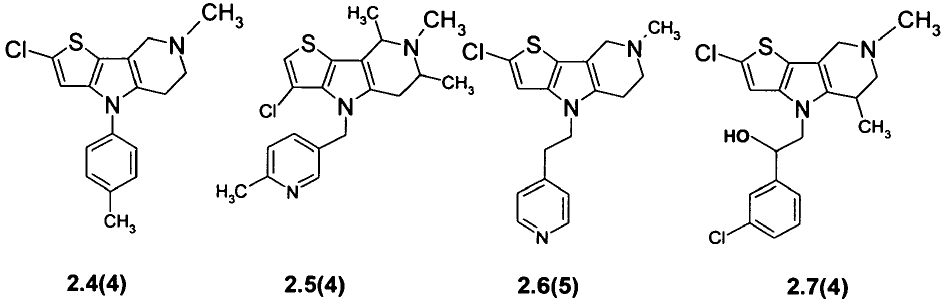

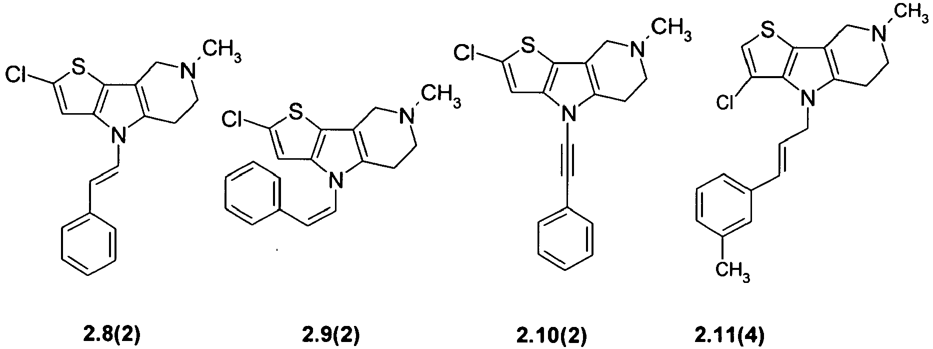

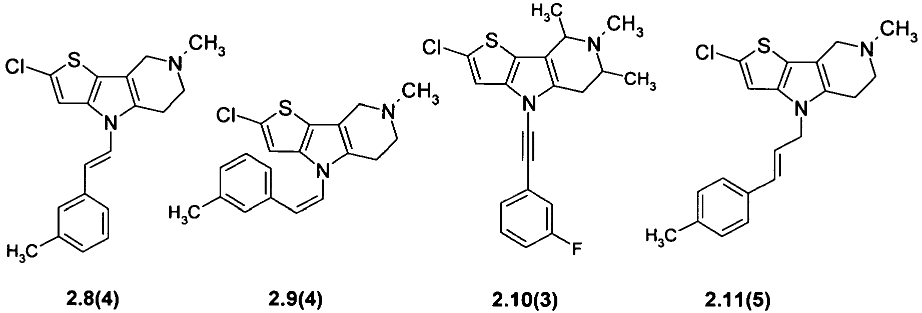

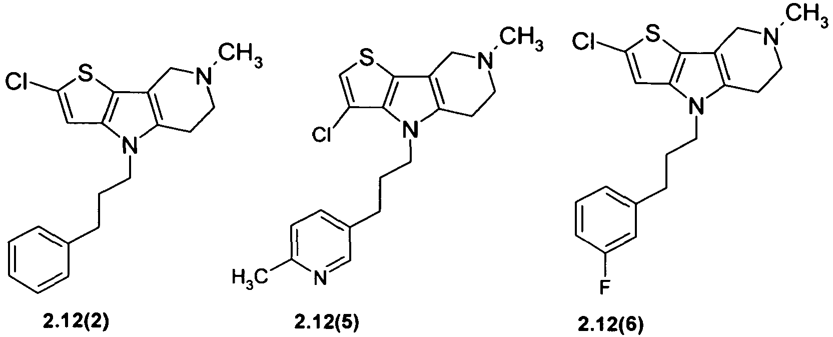

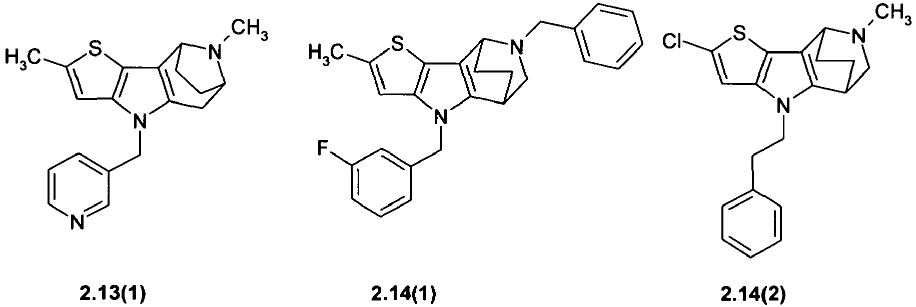

The more preferable 4,5,6,7-tetrahydro-5*H*-thieno[3',2':4,5]pyrrolo[3,2-c]pyridines are the compounds selected from the group consisting of:
2,5-dimethyl-8-(pyridin-4-yl)-4,5,6,7-tetrahydro-5H-thieno[3',2':4,5]pyrrolo[3,2-c] pyridine **3.4(1),**
8-benzyl-2,5-dimethyl-4,5,6,7-tetrahydro-5H-thieno[3',2':4,5]pyrrolo[3,2-c]pyridine **3.5(1**),
2,5-dimethyl-8-phenethyl-4,5,6,7-tetrahydro-5H-thieno[3',2':4,5]pyrrolo[3,2-c]pyridine **3.6(1**),
(5-methyl-8-phenethyl-4,5,6,7-tetrahydro-5H-thieno[3',2':4,5]pyrrolo[3,2-c]pyridin-2-yl)-methanol **3.6(2),**
2,5-dimethyl-8-(3-fluorophenyl)-4,5,6,7-tetrahydro-5H-thieno[3',2':4,5]pyrrolo[3,2-c]pyridine **3.4(3)**,
8-(pyridin-4-ylmethyl)-3-methyl-5-benzyl-4,5,6,7-tetrahydro-5H-thieno[3',2':4,5]pyrrolo [3,2-c]pyridine **3.5(3),**
2,5,7-trimethyl-8-(4-chlorophenethyl)-4,5,6,7-tetrahydro-5H-thieno[3',2':4,5]pyrrolo[3,2-c] pyridine **3.6(4),**
2-(2,5-dimethyl-4,5,6,7-tetrahydro-5H-thieno[3',2':4,5]pyrrolo[3,2-c]pyridin-8-yl)-1-phenyl-ethanol **3.7(1**),
(*E*)-2,5-dimethyl-8-styryl-4,5,6,7-tetrahydro-5H-thieno[3',2':4,5]pyrrolo[3,2-c]pyridine **3.8(1),**
(Z)-2,5-dimethyl-8-styryl-4,5,6,7-tetrahydro-5H-thieno[3',2':4,5]pyrrolo[3,2-c]pyridine **3.9(1),**
2-(2,5,7-trimethyl-4,5,6,7-tetrahydro-5H-thieno[3',2':4,5]pyrrolo[3,2-c]pyridin-8-yl)-1-*p*-tolyl-ethanol **3.7(3),**
(*E*)-2-methyl-5-ethyl-8-(pyridin-3-ylvinyl)-4,5,6,7-tetrahydro-5H-thieno[3',2':4,5]pyrrolo [3,2-c]pyridine **3.8(3),**
(Z)-2-methyl-8-(pyridin-3-ylvinyl)-4,5,6,7-tetrahydro-5H-thieno[3',2':4,5]pyrrolo[3,2-c] pyridine **3.9(3),**
2,5-dimethyl-8-phenylethynyl-4,5,6,7-tetrahydro-5H-thieno[3',2':4,5]pyrrolo[3,2-c]pyridine **3.10(1),**
(2-methyl-5-(3-fluorobenzyl)-8-cinnamyl-4,5,6,7-tetrahydro-5H-thieno[3',2':4,5]pyrrolo[3,2-c]pyridine **3.11(1),**
(2,5-dimethyl-8-[3-(*p*-tolyl)allyl]-4,5,6,7-tetrahydro-5H-thieno[3',2':4,5]pyrrolo[3,2-c] pyridine **3.11(2),**
(2,5-dimethyl-8-[3-(3-chlorophenyl)allyl]-4,5,6,7-tetrahydro-5H-thieno[3',2':4,5]pyrrolo[3,2-c]pyridine **3.11(3),**
(3,5-dimethyl-8-(3-phenylpropyl)-4,5,6,7-tetrahydro-5H-thieno[3',2':4,5]pyrrolo[3,2-c] pyridine **3.12(1),**
(2,5-dimethyl-8-[3-(6-methylpyridin-3-yl)propyl]-4,5,6,7-tetrahydro-5H-thieno[3',2':4,5]pyrrolo[3,2-c]pyridine **3.12(3),**
(3,4,5,6-tetramethyl-8-[3-(3-chlorophenyl)propyl]-4,5,6,7-tetrahydro-5H-thieno[3',2':4,5]pyrrolo[3,2-c]pyridine **3.12(4),**
5-methyl-8-(pyridin-4-yl)-2-chloro-4,5,6,7-tetrahydro-5H-thieno[3',2':4,5]pyrrolo[3,2-c]pyridine **3.4(2),**
8-benzyl-5-methyl-2-chloro-4,5,6,7-tetrahydro-5H-thieno[3',2':4,5]pyrrolo[3,2-c]pyridine **3.5(2),**
5-methyl-8-phenethyl-2-chloro-4,5,6,7-tetrahydro-5H-thieno[3',2':4,5]pyrrolo[3,2-c]pyridine **3.6(3),**
2-(5-methyl-2-chloro-4,5,6,7-tetrahydro-5H-thieno[3',2':4,5]pyrrolo[3,2-c]pyridin-8-yl)-1-phenyl-ethanol **3.7(2),**
5-methyl-8-(6-methylpyridin-3-yl)-2-chloro-4,5,6,7-tetrahydro-5H-thieno[3',2':4,5]pyrrolo [3,2-c]pyridine **3.4(4),**
8-(3-fluorobenzyl)-4,5,6-trimethyl-3-chloro-4,5,6,7-tetrahydro-5H-thieno[3',2':4,5]pyrrolo[3,2-c]pyridine **3.5(4),**
5-methyl-8-[2-(pyridin-4-yl)ethyl]-2-chloro-4,5,6,7-tetrahydro-5H-thieno[3',2':4,5]pyrrolo [3,2-c]pyridine **3.6(5),**
2-(5,7-dimethyl-3-chloro-4,5,6,7-tetrahydro-5H-thieno[3',2':4,5]pyrrolo[3,2-c]pyridin-8-yl)-1-(3-chlorophenyl)-ethanol **3.7(4),**
(*E*)-5-methyl-8-styryl-2-chloro-4,5,6,7-tetrahydro-5H-thieno[3',2':4,5]pyrrolo[3,2-c]pyridine **3.8(2),**
(Z)-5-methyl-8-styryl-2-chloro-4,5,6,7-tetrahydro-5H-thieno[3',2':4,5]pyrrolo[3,2-c]pyridine **3.9(2),**
5-methyl-8-phenylethynyl-2-chloro-4,5,6,7-tetrahydro-5H-thieno[3',2':4,5]pyrrolo[3,2-c]pyridine **3.10(2),**
(*E*)-5-methyl-8-[(3-*m*-tolyl)allyl]-3-chloro-4,5,6,7-tetrahydro-5H-thieno[3',2':4,5]pyrrolo[3,2-c]pyridine **3.11(4),**
(*E*)-5-methyl-8-(3-methylstyryl)-2-chloro-4,5,6,7-tetrahydro-5H-thieno[3',2':4,5]pyrrolo[3,2-c]pyridine **3.8(4),**
(Z)-5-methyl-8-(3-methylstyryl)-2-chloro-4,5,6,7-tetrahydro-5H-thieno[3',2':4,5]pyrrolo[3,2-c]pyridine **3.9(4),**
4,5,6-trimethyl-8-[(3-fluorophenyl)ethynyl]-2-chloro-4,5,6,7-tetrahydro-5H-thieno[3',2':4,5]pyrrolo[3,2-c]pyridine **3.10(3),**
(*E*)-5-methyl-8-[3-(6-methylpyridin-3-yl)allyl]-2-chloro-4,5,6,7-tetrahydro-5H-thieno[3',2':4,5]pyrrolo[3,2-c]pyridine **3.11** (5),
(5-methyl-8-(3-phenylpropyl)-2-chloro-4,5,6,7-tetrahydro-5H-thieno[3',2':4,5]pyrrolo[3,2-c]pyridine **3.12(2),**
5-methyl-8-[(*p*-tolyl)propyl]-3-chloro-4,5,6,7-tetrahydro-5H-thieno[3',2':4,5]pyrrolo[3,2-c]pyridine **3.12(5),**
5-methyl-8-[(3-fluorophenyl)propyl]-2-chloro-4,5,6,7-tetrahydro-5H-thieno[3',2':4,5]pyrrolo[3,2-c]pyridine **3.12(6),**
9-benzyl-2,10-dimethyl-4,5,6,7,8,9-hexahydro-4,7-epiminocyclohepta[b]thieno[3,2-d]pyrrole **3.13(1),**
10-benzyl-8-(3-fluorobenzyl)-2-methyl-5,6,7,8-tetrahydro-4H-4,7-(epiminomethano)thieno[2,3-b]indole **3.14(1),**
2-chloro-10-methyl-8-(2-phenylethyl)-5,6,7,8-tetrahydro-4H-4,7-(epiminomethano)thieno[2,3-b]indole **3.14(2),**

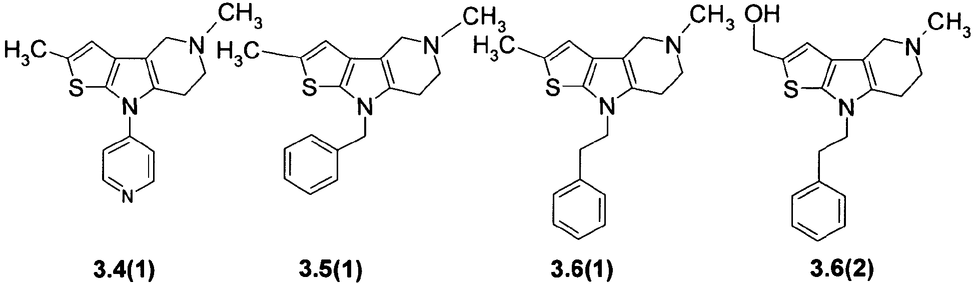

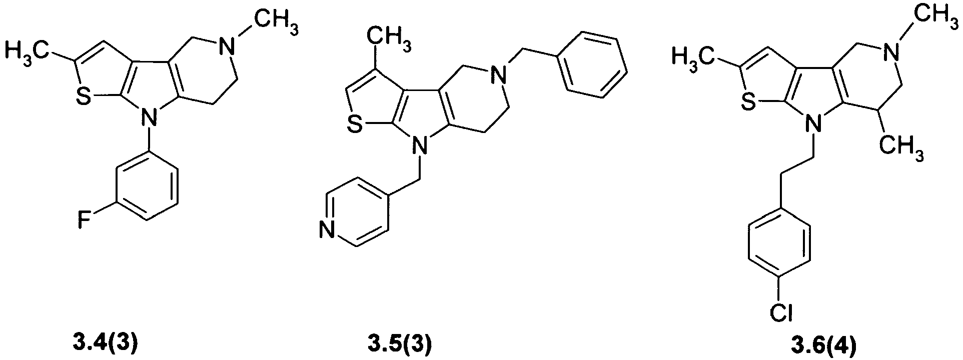

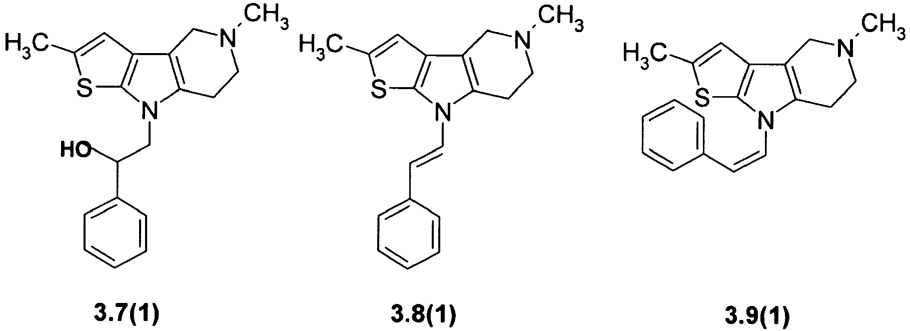

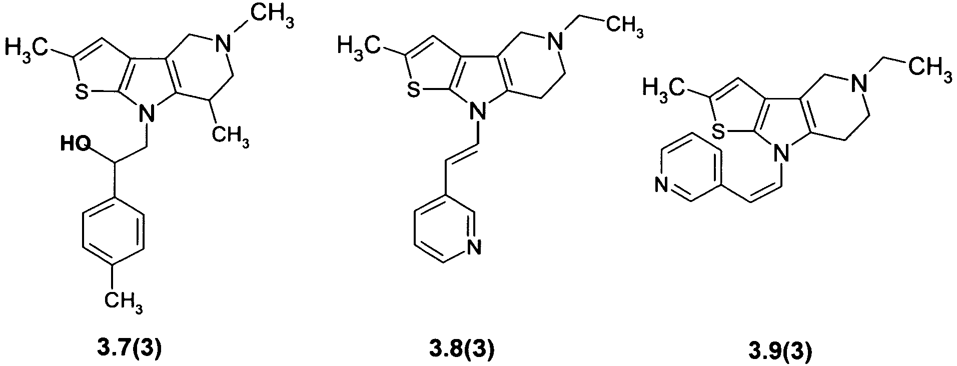

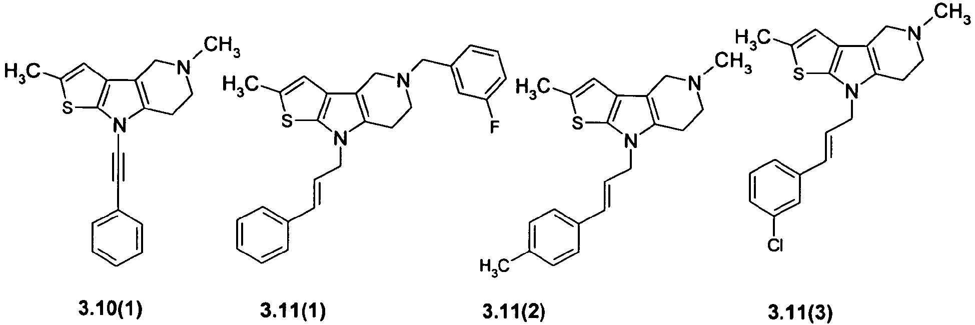

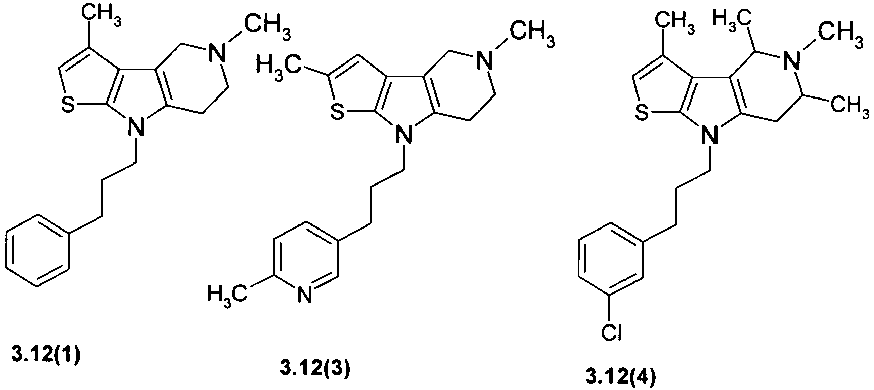

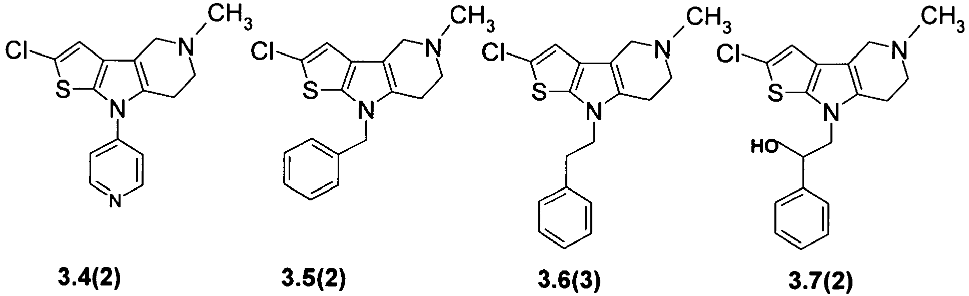

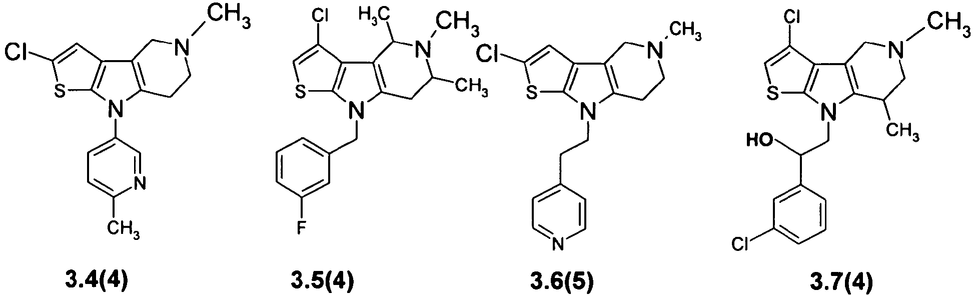

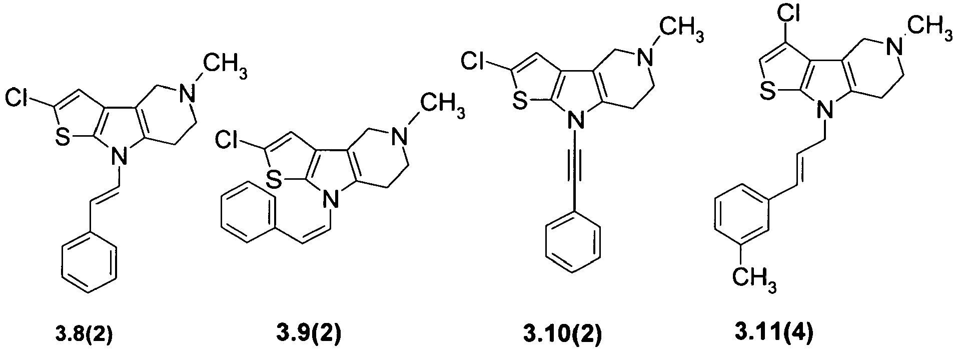

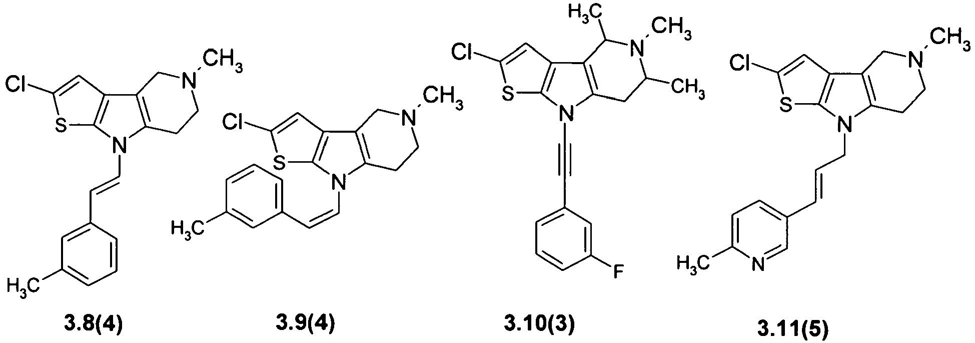

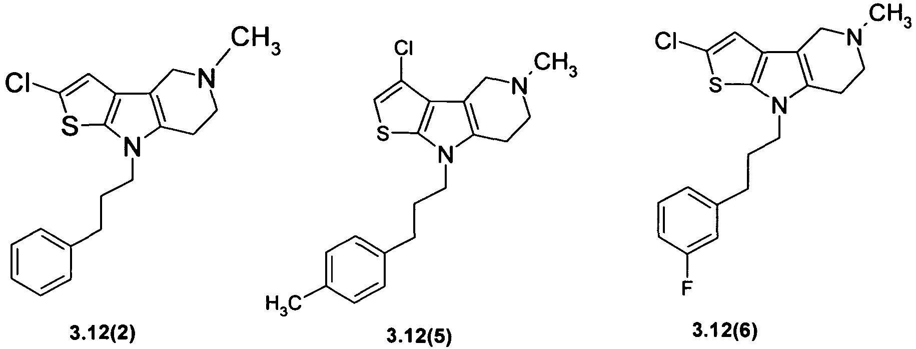

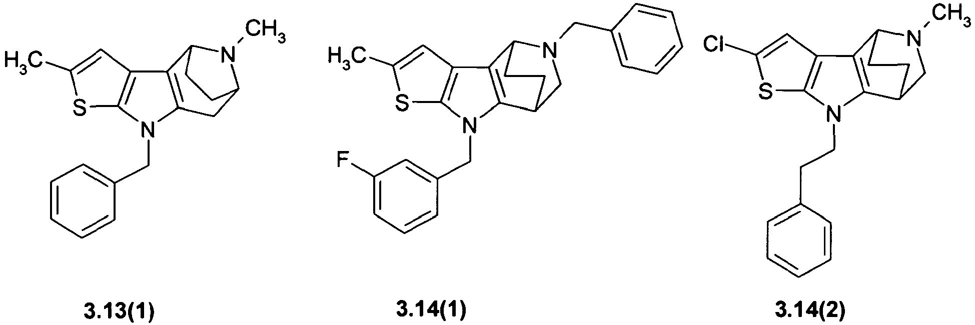

Tetrahydro-thieno-pyrrolo[3,2-c]pyridines could be prepared using reactions commonly used in the chemistry of heterocyclic compounds. Thieno-pyrrolo[3,2-c]pyridines **1.1** unsubstituted at pyrrole nitrogen were prepared by analogy with the synthesis of 2,3,4,5-tetrahydro-1H-pyrido[4,3-b]indoles [Ysp. khim., 79(4), 325-347 (2010)] - by interaction of (1-*t*-BOC-hydrazino)-thiophenes **4** with substituted piperidin-4-ones **5** under Fischer reaction conditions.

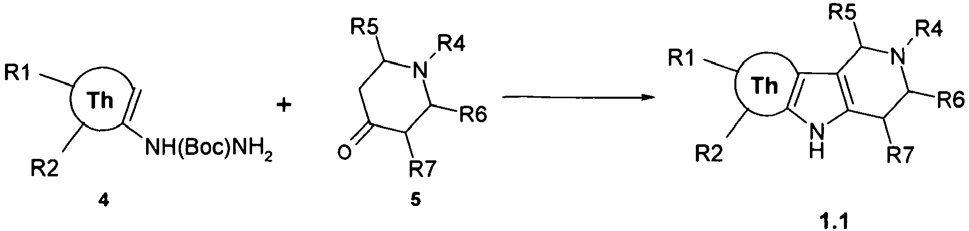

Thus, for example, if R4 in compound **5** represents ethoxycarbonyl, compounds of the general formulas **2.2** and **3.2** were formed, in other words, the corresponding ethoxycarbonyl-tetrahydro-4H-thiopheno-pyrrolo[3,2-c]pyridines **1.2,** which in turn were transformed to methyl derivatives **1.3** by LiAlH₄ reduction, among other things, to 7-methyl-5,6,7,8-tetrahydro-4H-thiopheno[2',3':4,5]pyrrolo[3,2-c]pyridines **2.3** and 5-methyl-4,6,7,8-tetrahydro-5*H*-thieno[3',2':4,5]pyrrolo[3,2-c]pyridines **3.3.**

If in compound **5** R4 represents optionally substituted acyl, for example, acetyl, benzoyl or *m*-fluorobenzoyl, the corresponding compounds of the general formulas **2.1** and **3.1,** were formed; LiAlH₄ reduction of them gave 7-(ethyl-, benzyl-, *m-*fluorobenzyl)-5,6,7,8-tetrahydro-4H-thiopheno[2',3':4,5]pyrrolo[3,2-c]pyridines **2.1** and 5-(ethyl-, benzyl-, *m*-fluorobenzyl)-4,6,7,8-tetrahydro-5*H-*thieno[3',2':4,5]pyrrolo[3,2-c]pyridines **3.1.**

If **5** represents 1,3-dimethylpiperidin-4-one **5a** or 1,2,6-trimethylpiperidin-4-one **5b:** 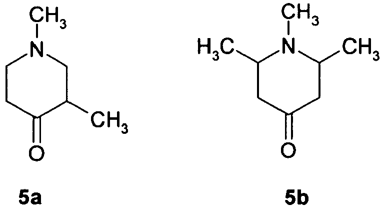 or their 1-alkoxycarbonyl substituted analog, the corresponding compounds of the general formula **1.1** in which R5 and R6 or R7 represent methyl were formed.

If **5** represents 8-methyl-8-azabicyclo[3.2.1]octan-3-one **5c** or 2-methyl-2-azabicyclo[2.2.2]octan-5-one **5d,** 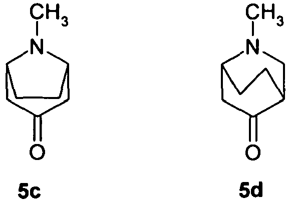 or their 8- or 2-alkoxycarbonyl substituted analog, compounds of the general formulas **1.13 (2.13** and **3.13)** and **1.14 (2.14** and **3.14)** were formed, respectively.

Note that previously unknown 3-(1-*t* BOC-hydrazino)-5-methyl-thiophene **4(1)** was prepared by interaction of 3-bromo-5-methylthiophene [Acta Chem. Scand., 1962, 16, 1127-1132], and *N-t*-BOC-hydrazine according to the method given in [J. Org. Chem., 2009, 74(19), 4542-4546]; and 2-(1-*t*-BOC-hydrazino-5-chloro-thiophene **4(2)** unknown before was prepared according to the method given in [Synthesis, 1977, 7, 487-489] starting from *t*-BOC-(3-bromo-5-chlorothiophen-2-yl)-amine prepared according to [Synthesis, 1977, 4, 255].

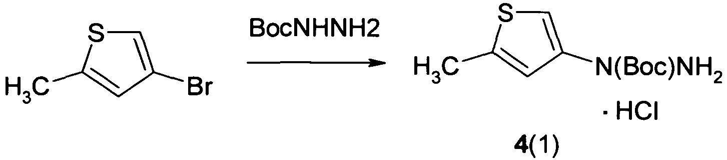

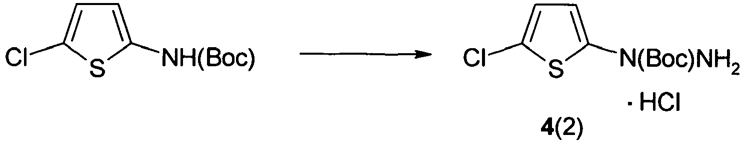

4-Aryl(or heteroaryl) derivatives **1.4,** among them 4-aryl(or heteroaryl)-5,6,7,8-tetrahydro-4*H*-thieno[2',3':4,5]pyrrolo[3,2-*c*]pyridines **2.4** and 8-aryl(or heteroaryl)-4,6,7,8-tetrahydro-4*H*-thieno[3',2':4,5]pyrrolo[3,2-*c*]pyridines **3.4,** were prepared by arylation or heteroarylation of thieno-pyrrolo[3,2-c]pyridines unsubstituted at pyrrole nitrogen **1.1** with aryl iodides or heteroaryl iodides in the presence of cuprous iodide, N,N'-dimethylethylenediamine and K₂CO₃.

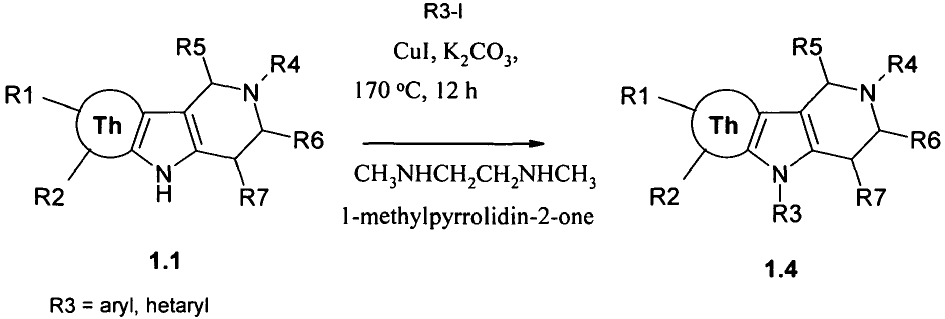

By alkylation of thieno-pyrrolo[3,2-c]pyridines unsubstituted at pyrrole nitrogen **1.2** with benzyl halogenide or their heteroanalogs thieno-pyrrolo[3,2-c]pyridines benzyl substituted at pyrrole nitrogen and their hetero analogues **1.5.1** were prepared, among them **2.5.1** and **3.5.1,** which were transformed into the corresponding thieno-pyrrolo[3,2-c]pyridines **1.5.2** by reduction with LiALH₄ 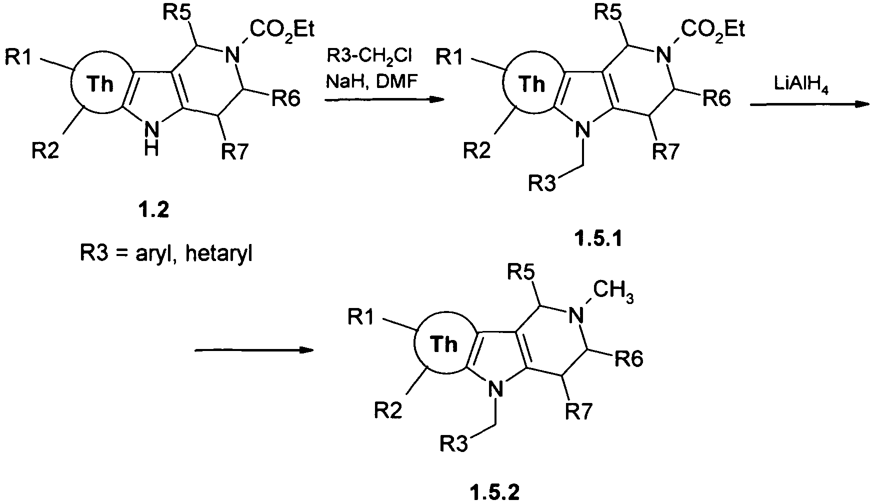 among them **2.5.2** and **3.5.2.**

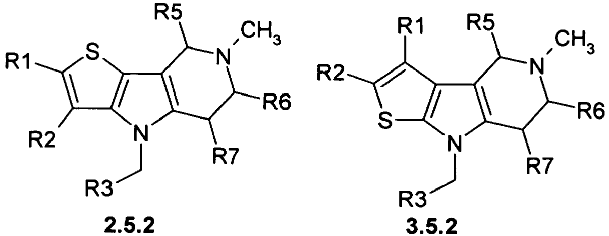

Alkylaion of thieno-pyrrolo[3,2-c]pyridines unsubstituted at pyrrole nitrogen **1.2** with optionally substituted (2-bromoethyl)-benzenes or their hetero analogues gave phenethyl substituted thieno-pyrrolo[3,2-c]pyridines and their hetero analogues **1.6.1,** among them **2.6.1** and **3.6.1,** which were transformed into the corresponding thieno-pyrrolo[3,2-c]pyridines **1.6.2** by reduction with LiAlH₄, 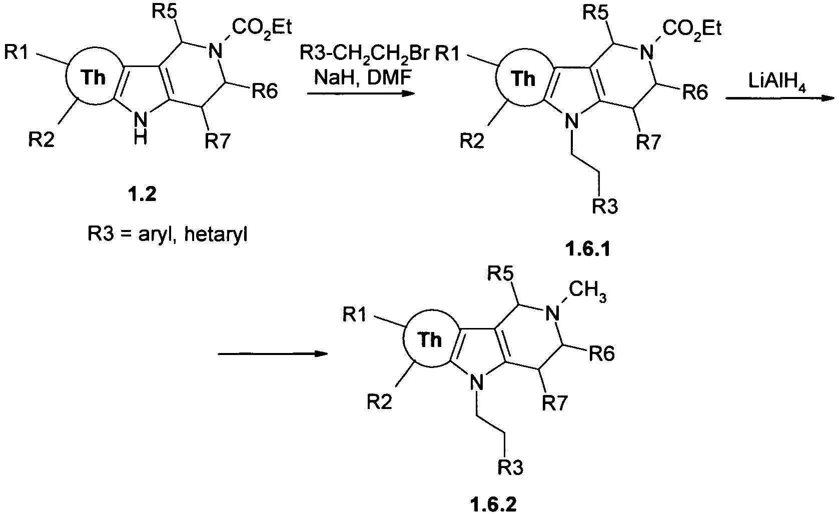 among them **2.6.2** and **3.6.2.**

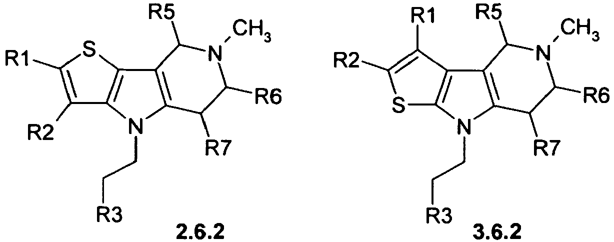

Thieno-pyrrolo[3,2-c]pyridines **1.6** were also prepared by subsequent action of PBr₃ on the corresponding 2-chloro-thieno-pyrrolo[3,2-c]pyridines **1.7** and reduction of the formed bromo derivatives **6** with Zn.

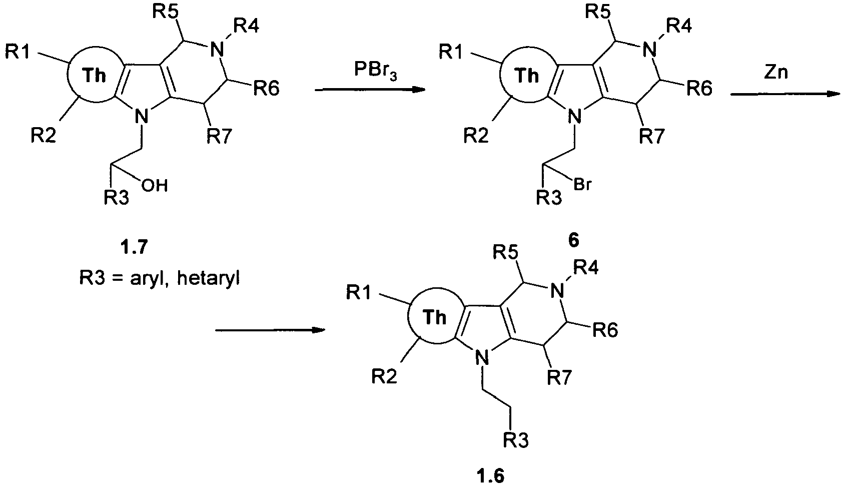

Reaction of thieno-pyrrolo[3,2-c]pyridines unsubstituted at pyrrole nitrogen **1.1** with aryloxiranes or heteroaryloxiranes, their heteroanalogues, in DMF in the presence of K₃PO₄ gave the corresponding thieno-pyrrolo[3,2-c]pyridines **1.7,** among them **2.7** and **3.7.**

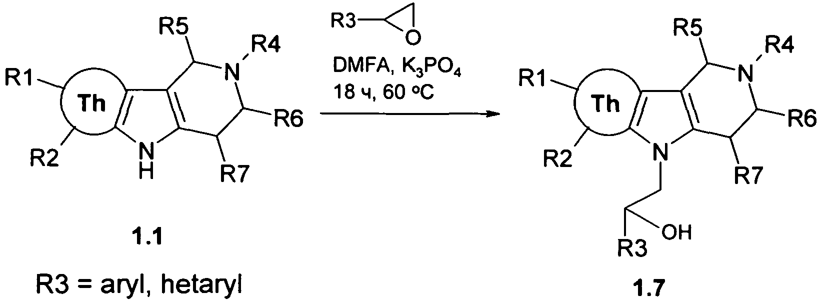

The corresponding thieno-pyrrolo[3,2-c]pyridines **1.8, 1.9,** among them **2.8, 2.9, 3.8,** and **3.9** were prepared by the reaction of thieno-pyrrolo[3,2-c]pyridines unsubstituted at pyrrole nitrogen **1.1** with aryl acetylenes or their hetero analogues in alkaline medium and subsequent separation of isomeric products.

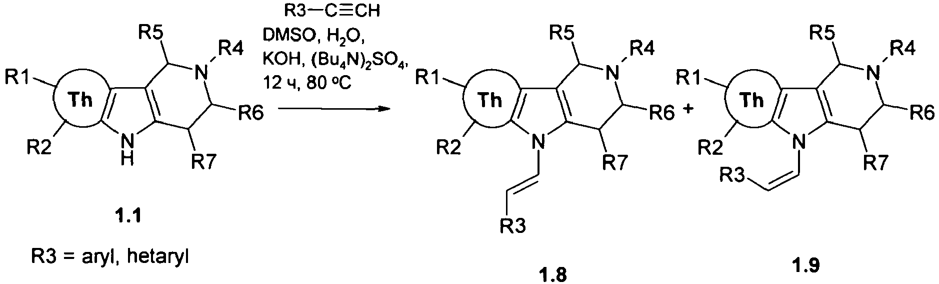

Acetylenes **1.10,** among them **2.10** and **3.10,** were prepared by the action of acetylene halogenides of the general formula **7** on thieno-pyrrolo[3,2-c]pyridines unsubstituted at pyrrole nitrogen **1.1** in toluene in the presence of CuSO₄·5H₂O, 1,10-phenantroline and K₃PO₄.

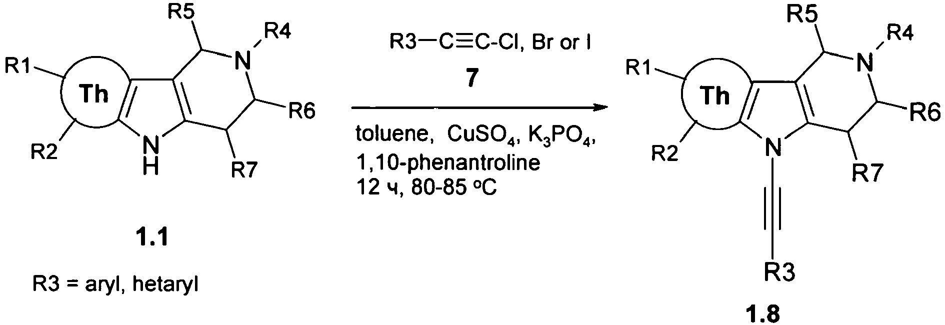

Thieno-pyrrolo[3,2-*c*]pyridines **1.11** were prepared by alkylation of thieno-pyrrolo[3,2-c]pyridines unsubstituted at pyrrole nitrogen **1.2** with cinnamyl chlorides **8** or their hetero analogues, and subsequent reduction gave the corresponding substituted thieno-pyrrolo[3,2-c]pyridines **1.12.** The latter could also be prepared by alkylation of compobnds **1.2** with the corresponding 3-aryl- or 3-hetaryl-propane halogenides:

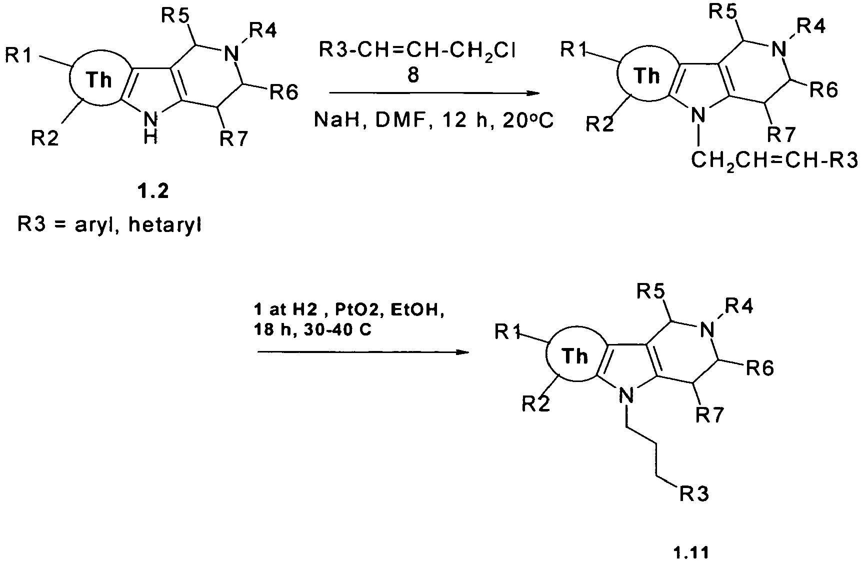

Tetrahydro-4H-thieno-pyrrolo[3,2-*c*]pyridines of the general formula 1 of the present invention could form hydrates or pharmaceutically acceptable salts. For the purpose of salt preparation inorganic and organic acids could be used, such as, for example, hydrochloric acid, hydrobromic acid, hydroiodic acid, sulfuric acid, phosphoric acid, formic acid, acetic acid, propionic acid, trifluoroacetic acid, maleic acid, tartaric acid, methanesulfonic acid, benzenesulfonic acid, *p*-toluenesulfonic acid.

The subject of the present invention is ligands with wide range of receptor activity towards *alpha*-adrenoceptors, dopamine receptors, histamine receptors, and serotonin receptors, representing substituted tetrahydro-thieno-pyrrolo[3,2-c]pyridines of the general formula **1,** geometrical isomers, mixtures of geometrical isomers, pharmaceutically acceptable salts thereof.

The subject of the present invention is an active component for pharmaceutical compositions and medicaments, representing at least one of the ligands.

According to the invention hydrogenated thieno-pyrrolo[3,2-c]pyridines of the general formula **1,** their geometrical isomers, mixtures of geometrical isomers, pharmaceutically acceptable salt and/or hydrates exhibit a wide range of biological activity and could be used as active component for pharmaceutical compositions and medicaments, intended for treatment and prophylaxis of central nervous system diseases (CNS), such as:
depression, including major depression; episodic, chronic and recurrent forms of major depression; dysthymic disorder; cyclothymia; affective disorders; syndrome of seasonal affective disorder; bipolar disorders including bipolar disorder of I and II types; and also other depressive disorders and positions; depressive positions, accompanying Alzheimer's disease, vascular dementia; mood disorder, induced by alcohol and substances; schizoaffective disorders of depressive type; adjustment disorders; beyond that, depression includes depressed mood of oncologic patients; at Parkinson's disease; depression after myocardial infarct; barren women's depression; pediatric depression; post-natal depression; and also other depressive disorders accompanying somatic, neuralgic and other diseases;
disturbance of mental abilities, including attetion, memory, cognition, intellection, training, verbal, brainpower, operating and creative abilities, orientation in time and space, in particular, cognitive disorders associated with Alzheimer's, Parkinson's and Huntington's diseases; senile dementia; age-related mnestic disorders; dysmetabolic encephalopathy; psychogenous memory impairment; amnesia; amnestic disorders; transient global amnesia; dissociative amnesia; nascular dementia; cognitive disorders; syndrome of disorder of attention with superactivity; cognitive disorders, accompanying psychotic diseases, autism, epilepsia, delirium, psychosis, Down syndrome, bipolar disorders and depression; AIDS-related dementia; dementia: at hypothyroidism; alcohol induced; by addictive compounds and neurotoxins; accompanying diseases, for example, cerebellar degeneration and amyotrophic lateral sclerosis; disorders developing at insult, infective and oncological diseases of brain, and also at traumatic brain injury; cognitive impairments, associated with autoimmune and endocrine diseases; and other cognitive disorders;
neurodegenerative diseases, which include but not limited to Alzheimer's and Parkinson's diseases; Hantington's disease (chorea); multiocular sclerosis; cerebellar degeneration; amyotrophic lateral sclerosis; Lewy body dementia; Aran-Duchenne disease; peripheral neuropathy; spongiform encephalopathy (Creutzfeld-Jakob Disease); AIDS-related dementia; multi-infarct dementia; Pick's disease; leucoencephalopathy; chronic neurodegenerative diseases; insult; ischemic, reperfusion and hypoxic brain damage; epilepsy; cerebral ishemia; glaucoma; traumatic brain injury; Down's syndrome; encephalomyelitis; meningitis; encephalitis; neuroblastoma; schizophrenia; depression; besides, pathologic states and disorders, developing at hypoxia, excessive use of addictant, at neurotoxins action, infectious and oncological diseases of brain, and also neuronal damage, associated with autoimmune and endocrine diseases; and other neurodegenerative processes;
psychic disorders including affective disorder (bipolar affective disorders, major depression, hypomania, minor depression, maniacal syndrome, Cotard's syndrome, cyclothymia, schizoaffective disorder and the like); mental-mnestic disorders, manias (hypomania, graphomania, kleptomania, shopping addiction, persecution mania, monomania, pornographomania, erotomania and the like); multiple personality disorder, amentia, delirium, delusion, delirium syndrome, hallucinosis, hallucinnations, homicidomania, delirium, illusion, querulous paranoia, clinical lycanthropy, macropsia, antagonistic delusion, micropsia, narcomania, anorexia nervosa, oneiroid, paranoid, paranoia, paraphrenia, pseudohallucinations, psychosis, schizotypal disorder, schizophrenia, schizo-affective disorder, schizophreniform disorder, Schroeder's syndrome, Daniel Paul's syndrom); phobias (agoraphobia, arachnophobia, autophobia, verminophobia, hydrozophobia, hydrophobia, demophobia, zoophobia, cancerophobia, claustrophobia, climacophobia, xenophobia, mysophobia, radiophobia, photophobia, scoleciphobia, nyctophobia, social phobia, tetraphobia, triskidephobia, erotophobia); alcoholic psychosis, alcoholic palimpsest, allotriophagy, aphasia, graphomania, dissociative fugues, dissociative disorders, disphorias, internet addiction disorders, hypochondria, hysteria, coprophemia, persecution mania, melancholy, misanthropy, obsession, panic attacks, Asperger's disorder, Capgras' syndrome, Munchausen syndrome, Rett's syndrome, Fregoli syndrome, attention deficit and hyperactivity disorder, obsessive-compulsive disorder, syndrome of chronic narcotization backlash, psychic automatism syndrome, infantile autism syndrome, insanity, taphophilia, qualms, Hikikomori syndrome, erotographomania and the like;
psychotic diseases, such as all types of schizophrenia; schizophreniform diseases; schizotypal disorders; schizo-affective disorder, including circular and depressive types; delusional disorders, including reference delusion, persecution, grandeur, jealousy, erotomania, and also hypochondriacal, somatic, mixed and nondifferentiable delirium; short-term psychotic disorders; induced psychotic disorders; psychotic disorders induced by compounds; and also other psychotic disorders;
anxious disorders, such as generalised (inconcrete) anxiety; acute out-of-control anxietypanic disorders; phobias, for example, agoraphobia (pathological fear of crowded places) or social phobia (strong fear of humiliation before other people) or any concrete phobia (strong fear of concrete subjects, animals or situations, such as fear of heights, medical procedures, lifts, open space and the like); compulsion neurosis (obsessive-compulsive disorder); posttraumatic stress disorder and acute stress disorder; anxieties induced by alcohol or compounds; anxiety at adjustment disorder; and also mixed forms of anxious disorders and depression.

The subject of the present invention is pharmaceutical composition for treatment and prophylaxis of various conditions and diseases of central nervous system, pathogenesis of which is associated with receptor activity of alpha-adrenoceptors, dopamine receptors, histamine receptors and serotonin receptors, comprising pharmaceutically effective amount of active component; and also a pharmaceutical composition in the form of tablets, capsules or injections placed in pharmaceutically acceptable packing.

If needed, according to the present invention pharmaceutical compositions could be used in clinical practice in various forms prepared by mixing the said compositions with traditional pharmaceutical carries, for example, peroral forms (such as, tablets, gelatinous capsules, pills, solutions or suspensions); forms for injections (such as, solutions or suspensions for injections, or a dry powder for injections which requires only addition of water for injections before utilization).

According to the present invention the carriers used in pharmaceutical compositions represent carriers which are used in the sphere of pharmaceutics for preparation of commonly used forms. Binding agents, greasing agents, disintegrators, solvents, diluents, stabilizers, suspending agents, colorless agents, taste flavors are used for peroral forms; antiseptic agents, solubilizers, stabilizers are used in forms for injections; base materials, diluents, greasing agents, antiseptic agents are used in local forms.

Pharmaceutical compositions could be administered peroral or parenterally (for exaqmple, intravenous, subcutaneous, intraperitoneally or local). If any drug substance is not stable in stomach, it could be used for preparation of tablets covered with coating soluble in stomach or intestinal tract.

Besides, clinical dose of hydrogenated thieno-pyrrolo[3,2-c]pyridine of the general formula **1** or its geometrical isomer, or its pharmaceutically acceptable salt at patients may be corrected depending on: therapeutic efficiency and bio-accessibility of active ingredients in patients' organism, rate of their exchange and removal from organism, and age, gender, and severity of patient's symptoms. Thus, the daily intake for adults normally being 10∼500 mg, preferably 50∼300 mg. While preparing pharmaceutical composition as a dose unit the above effective dose is to be taken into consideration, at this each dose unit of composition contains 10∼500 mg of a compound of the general formula **1,** preferably - 50 - 300 mg. Following the instructions of physician or pharmacist, the medicaments may be taken several times over specified periods of time (preferably, from one to six times).

The subject of the present invention is a therapeutic kit for prophylaxis and treatment of various diseases of central nervous system at humans and animals comprising an active component or pharmaceutical composition.

Therapeutic kits for prophylaxis and treatment of neuralgic disorders, neurodegenerative and cognitive diseases at humans and animals, including prophylaxis and treatment of Alzheimer's disease, Parkinson's disease, Huntington's disease, mental disorders and schizophrenia; hypoxia-ischemia, hypoglycemia, convulsive states, cerebral traumas, lathyrism, amyotrophic lateral sclerosis, obesity and insult; in addition to the drug substance disclosed in the invention, may include other active ingredients such as: nonsteroidal antiinflammatory drugs (Orthophene, Indomethacin, Ibuprophen and others); acetyl cholinesterase inhibitors (Tacrine, Amiridine, Fizostigmine, Aricept, Phenserine and others); estrogens (for example, Estradiol); NMDA-receptor antagonists (for example, Memantine, Neramexane); nootropic drugs (for example, Pyracetam, Fenibut and others); AMPA receptor modulators (for example, Ampalex); cannabinoid CB-1 receptor antagonists (for example, Rimonabant); monoaminooxidase inhibitors MAO-B and/or MAO-A (for example, Rasagiline); antiamyloidogenic drugs (for example, Tramiprosate); lowering β-amyloidal neurotoxicity compounds (for example, Indole-3-propionic acid); γ- and/or β-secretase inhibitors; muscarinic receptor agonists (for example, Cevimeline); metal helates (for example, Clioquinol); GABA(A) receptor antagonists (for example, CGP-36742); monoclonal antibodies (for example, Bapineuzumab); antioxidants; neurotrophic agents (for example, Cerebrolisine); antidepressants (for example, Imipramine, Sertraline and others) and others.

The subject of the present invention is a method for prophylaxis and treatment of various diseases of central nervous system, pathogenesis of which is associated with receptor activity of alpha-adrenoceptors, dopamine receptors, histamine receptors and serotonin receptors consisting in administration to the patient of an active component, or pharmaceutical composition, or therapeutic kit.

The subject of the present invention is substituted tetrahydro-4*H*-thieno-pyrrolo[3,2-c]pyridines of the general formula **1,** their racemates, optical isomers, geometrical isomers, mixtures of optical or geometrical isomers, pharmaceutically acceptable salts for investigation of peculiarities of physiologically active compounds, exhibiting a wide range of biological activity towards *alpha-*adrenoceptors, dopamine receptors, histamine receptors and serotonin receptors ("molecular tools").

Below the invention is described by means of specific examples, which illustrate but not limit the scope of invention.

### Example 1

General method for preparation of ethyl tetrahydro-thieno-pyrrolo[3,2-c]pyridine-carboxylates **1.2,** among them ethyl 4,5,6,8-tetrahydro-4*H-*thieno[2',3':4,5]pyrrolo[3,2-*c*]pyridine-7-carboxylates **2.2** and ethyl 4,6,7,8-tetrahydro-5*H*-thieno[3',2':4,5]pyrrolo[3,2-*c*]pyridine-5-carboxylates **3.2.** The starting hydrazine hydrochloride of the general formula **4** (3.5 mmol) and ethyl 4-oxopiperidine-1-carboxylate of the general formula **5** (3.5 mmol) were added to ethanol HCl solution (50 ml) (concentration is 65 mg/ml). The prepared solution was stirred at room temperature for 2 h (prolongation of the reaction is undesirable because of strong resin formation). After the solvent was evaporated product of the general formula **1.2,** among them. **2.2** and **3.2,** was separated by column chromatography (eluent EtOAc:hexane = 1:4). Ethyl 2-methyl-4,5,6,8-tetrahydro-4H-thieno[2',3':4,5]pyrrolo[3,2-c]pyridine-7-carboxylate **2.2(1).** Yield is 27%. LCMS (ESI): *m*/*z* 265 (M+H)⁺. ¹H NMR (DMSO-*d*₆, 400 MHz), δ, ppm (*J*, Hz): 10.76 (s, 1 H), 6.68 (q, *J =* 0.8, 1H), 4.38 (br. s, 2H), 4.07 (q, *J =* 7.0, 2H), 3.68 (t, *J* = 5.2, 2H), 2.69 (t, *J* = 5.2, 2H), 2.45 (br. s, 3H), 1.20 (t, *J* = 7.0, 3H). ¹³C NMR (DMSO-*d*₆, 75 MHz), δ, ppm.: 155.12, 136.79, 134.82, 128.42, 116.97, 110.40, 106.09, 60.87, 41.65, 41.18, 23.40, 16.27, 14.68. Ethyl 2-chloro-4,5,6,8-tetrahydro-4H-thieno[2',3':4,5]pyrrolo[3,2-c]pyridine-7-carboxylate **2.2(2).** Yield is 42%. LCMS (ESI): *m*/*z* 285 (M+H)⁺. ¹H NMR (DMSO-*d*₆, 400 MHz), δ, ppm. (J, Hz): 11.14 (s, 1 H), 7.10 (s, 1H), 4.40 (br. s, 2H), 4.07 (q, *J =* 7.2, 2H), 3.68 (t, *J* = 5.6, 2H), 2.71 (t, *J* = 5.6, 2H), 1.20 (t, *J* = 7.2, 3H).

Ethyl 2-methyl-4,6,7,8-tetrahydro-5H-thieno[3',2':4,5]pyrrolo[3,2-c]pyridine-5-carboxylate **3.2(1**). Yield is 24%. LCMS (ESI): *m*/*z* 265 (M+H)⁺. ¹H NMR (DMSO-*d*₆, 400 MHz), δ, ppm. (J, Hz): 10.89 (s, 1 H), 6.61 (d, *J =* 0.8, 1 H), 4.43 (br. s, 2H), 4.07 (q, *J* = 7.2, 2H), 3.66 (t, *J* = 5.6, 2H), 2.68 (t, *J* = 5.6, 2H), 2.41 (d, *J* = 0.8, 3H), 1.20 (t, *J* = 7.2, 3H). ¹³C NMR (CDCl₃, 75 MHz), δ, ppm: 155.63, 131.74, 130.20, 129.46, 126.73, 113.26, 106.53, 61.02, 41.59, 40.96, 23.19, 15.65, 14.24. Ethyl 2-chloro-4,6,7,8-tetrahydro-5H-thieno[3',2':4,5]pyrrolo[3,2-c]pyridine-5-carboxylate **3.2(2).** Yield is 36%. LCMS (ESI): *m*/*z* 285 (M+H)⁺. ¹H NMR (DMSO-*d*₆, 400 MHz), δ, ppm (*J,* Hz): 11.09 (s, 1 H), 7.08 (s, 1 H), 4.45 (br. s, 2H), 4.09 (q, *J* = 7.2, 2H), 3.67 (t, *J* = 5.6, 2H), 2.70 (t, *J* = 5.6, 2H), 1.20 (t, *J* = 7.2, 3H). ¹³C NMR (CDCl₃, 75 MHz), δ, ppm: 155.66, 129.95, 128.37, 124.72, 121.24, 115.46, 107.35, 61.23, 41.43, 40.93, 23.28, 14.34.

The interaction of hydrazine hydrochloride of the general formula **4** with 1-acylpiperidin-4-ones gave the corresponding compounds of the general formulas **2.1** and **3.1,** in which R4 represents acetyl, benzoyl or m-fluorobenzoyl. 2-Methyl-7-benzoyl-5,6,7,8-tetrahydro-4H-thieno[2',3':4,5]pyrrolo[3,2-c]pyridine **2.1(1).** Yield is 34%. LCMS (ESI): *m*/*z* 297 (M+H)⁺. ¹H NMR (DMSO-*d*₆, 400 MHz), δ, ppm. (*J,* Hz): 2.64 (t, *J₁* = 2.88, *J₂* = 0.90, 3H), 3.69 (m, 2H), 3.78 (m, 2H), 4.06 (q, *J* = 7.75, 2H), 4.70 (q, *J* = 16.33, 2H), 6.73 (s, 1 H), 7.38 (t, *J* = 7.32, 1 H), 7.52 (t, *J₁* = 7.32, *J₂* = 1.25, 2H), 7.80 (t, *J₁* = 7.59, *J₂* = 1.46, 2H), 9.15 (s, 1 H);
2-Methyl-7-acetyl-5,6,7,8-tetrahydro-4H-thieno[2',3':4,5]pyrrolo[3,2-c]pyridine **2.1(2)** Yield is 42%. LCMS (ESI): *m*/*z* 235 (M+H)⁺. ¹H NMR (DMSO-*d*₆, 400 MHz), δ, ppm. (*J,* Hz): 2.10 (t, *J* = 5.44, 3H), 2.64 (t, *J₁* = 2.88, *J₂* = 0.90, 3H), 3.21 (m, 2H), 3.80 (t, *J₁* = 7.75, *J₂* = 1.42, 2H),4.44 (m, 2H), 6.61 (s, 1 H), 9.15 (s, 1 H);

2-methyl-5-(3-fluorobenzoyl)-4,5,6,7-tetrahydro-5H-thieno[3',2':4,5]pyrrolo[3,2-c]pyridine **3.1(1).** Yield is 28%. LCMS (ESI): *m*/*z* 315 (M+H)⁺. ¹H NMR (DMSO-*d*₆, 400 MHz), δ, ppm. (*J,* Hz): 2.64 (t, *J₁* = 2.88, *J₂* = 0.90, 3H), 3.69 (m, 2H), 4.09 (q, *J* = 7.75, 2H), 4.69 (q, *J* = 16.33, 2H), 6.73 (s, 1 H), 7.25 (d, *J* = 7.80, 1 H), 7.40 (m, 1 H), 7.52 (d, *J* = 2.40, 1 H), 7.60 (t, *J₁* = 7.80, *J₂* = 1.20, 1 H), 9.15 (s, 1 H).

The interaction of hydrazine hydrochloride of the general formula **4** with 1,3-dimethylpiperidin-4-one **5a** or 1,2,6-trimethylpiperidin-4-one **5b** or their 1-alkoxycarbonyl substituted analogues gave compounds of the general formula **1.1,** in which R7 or R5 and R6 represent methyl.

2,5,7-trimethyl-5,6,7,8-tetrahydro-4H-thieno[2',3':4,5]pyrrolo[3,2-c]pyridine **2.1(3).** Yield is 37%. LCMS (ESI): *m*/*z* 221 (M+H)⁺. ¹H NMR (DMSO-*d*₆, 400 MHz), δ, ppm (*J,* Hz): 1.15 (t, *J₁* = 6.60, *J₂* = 0.93, 3H), 2.01 (t, *J₁* = 4.20, *J₂* = 1.51, 1H), 2.35 (t, *J₁* = 13.35, *J₂* = 0.99, 3H), 2.64 (t, *J₁* = 2.88, *J₂* = 0.90, 3H), 2.82 (t, *J₁* = 13.00, *J₂* = 4.20, 1 H), 3.10 (m, 1 H), 3.43 (m, 1 H), 3.61 (m, 1 H), 6.68 (s, 1 H), 9.15 (s, 1 H);

2,4,5,6-tetramethyl-4,5,6,7-tetrahydro-5H-thieno[3',2':4,5]pyrrolo[3,2-c]pyridine **3.1(2).** Yield is 29%. LCMS (ESI): *m*/*z* 235 (M+H)⁺. ¹H NMR (DMSO-*d*₆, 400 MHz), δ, ppm (*J,* Hz): 1.08 (t, *J₁* = 6.51, *J₂* = 1.13, 3H), 1.49 (t, *J₁* = 6.68, *J₂* = 1.13, 3H), 2.05 (t, *J₁* = 13.35, *J₂* = 0.99, 3H), 2.64 (d, *J* = 2.88, 3H), 2.87 (m, 2H), 3.13 (m, 1 H), 4.18 (m, 1 H), 5.85 (s, 1 H), 10.07 (s, 1 H).

When 8-methyl-8-azabicyclo[3.2.1]octan-3-one **5c** or 2-methyl-2-azabicyclo[2.2.2]octan-5-one **5d,** or their 8- and 2-alkoxycarbonyl substituted analogues respectively, were used, compounds of the general formulas **1.13 (2.13** and **3.13)** and **1.14 (2.14** and **3.14)** were prepared, in which W+R3 = H.

2,10-dimethyl-4,5,6,7,8,9-hexahydro-6,9-epiminocyclohepta[b]thieno[2,3-d]pyrrole **2.1(4).** Yield is 33%. LCMS (ESI): *m*/*z* 233 (M+H)⁺. ¹H NMR (DMSO-*d*₆, 400 MHz), δ, ppm (*J,* Hz): 1.69 (m, 1 H), 2.0 (t, *J₁* = 6.80, *J₂* = 0.80, 2H), 2.10 (t, *J₁* = 13.35, *J₂* = 0.99, 3H), 2.17 (m, 1H),2.64 (t, *J* = 2.88, 3H), 2.90 (m, 1H),3.02 (m, 1 H), 3.49 (m, 1 H), 4.17 (m, 1 H), 5.87 (s, 1 H), 10.07 (s, 1 H);

2,10-dimethyl-5,6,7,8-tetrahydro-4H-4,7-(epiminomethano)thieno[2,3-b]indole **3.1(3)** Yield is 19%. LCMS (ESI): *m*/*z* 233 (M+H)⁺. ¹H NMR (DMSO-*d*₆, 400 MHz), δ, ppm (*J,* Hz): 1.58 (m, 1 H), 1.81 (m, 1 H), 2.23 (m, 1 H), 2.38 (t, *J₁* = 13.35, *J₂* = 0.99, 3H), 2.52 (m, 2H), 2.64 (t, *J* = 2.88, 3H), 3.09 (m, 1 H), 3.26 (m, 1 H), 3.78 (m, 1H), 5.89 (s, 1 H), 10.07 (s, 1 H).

### Example 2

The general method for preparation of tetrahydro-thieno-pyrrolo[3,2-c]pyridines **1.3,** among them **2.3** and **3.3.** LiAlH₄ (100 mg, 2.66 mmol) in small portions was added to a solution of ethyl carboxylate **1.2** (1.33 mmol), among them **2.2** and **3.3** in Et₂O (50 ml). The reaction mixture was stirred at room temperature until the reaction was completed (about 2 h, LCMS control). After finishing the reaction water (0.5 ml) was added and stirring was continued at 20°C for 15 min. The precipitated solid was filtered off and washed with ether. The filtrate was washed with water, dried over Na₂SO₄, solven was evaporated. The residue was washed with hexane and dried. It gave final products **1.3,** among them **2.3** and **3.3.** 2,7-Dimethyl-5,6,7,8-tetrahydro-4H-thieno[2',3':4,5]pyrrolo[3,2-c]pyridine **2.3(1).** Yield is 70%. LCMS (ESI): *m*/*z* 207 (M+H)⁺. ¹H NMR (DMSO-*d*₆, 400 MHz), δ, ppm (*J,* Hz): 10.61 (s, 1H), 6.65 (q, *J =* 0.6, 1 H), 3.33 (br. s, 2H), 2.70 (t, *J =* 5.2, 2H), 2.63 (t, *J* = 5.2, 2H), 2.44 (d, *J* = 0.6, 3H), 2.36 (s, 3H). ¹³C NMR (DMSO-*d*₆, 75 MHz), δ, ppm: 136.40, 133.91, 128.46, 117.25, 110.38, 107.66, 52.35, 52.23, 45.50, 23.90, 16.29. 2,5-Dimethyl-4,6,7,8-tetrahydro-4H-thieno[3',2':4,5]pyrrolo[3,2-c]pyridine **3.3(1**). Yield is 79 %. LCMS (ESI): *m*/*z* 207 (M+H)⁺. ¹H NMR (DMSO-*d*₆, 400 MHz), δ, ppm (*J,* Hz): 10.71 (s, 1 H), 6.52 (s, 1 H), 3.36 (br. s, 2H), 2.67 (t, *J* = 5.2, 2H), 2.61 (t, *J* = 5.2, 2H), 2.40 (s, 3H), 2.35 (s, 3H). 5-Methyl-2-chloro-4,6,7,8--tetrahydro-4H-thieno[3',2':4,5]pyrrolo[3,2-c]pyridine **3.3(2).** Yield is 77 %. LCMS (ESI): *m*/*z* 227 (M+H)⁺. ¹H NMR (DMSO-*d*₆, 400 MHz), δ, ppm (*J,* Hz): 10.96 (s, 1 H), 6.97 (s, 1 H), 3.38 (br. s, 2H), 2.69 (t, *J* = 5.2, 2H), 2.62 (t, *J* = 5.2, 2H), 2.35 (s, 3H).

According to the method given above and using compounds of the general formula **1.1** in which **R4** represents acyl as starting materials tetrahydro-thieno-pyrrolo[3,2-c]pyridines were prepared in which **R4** represents optionally substituted lower alkyl.

2-Methyl-7-benzyl-5,6,7,8-tetrahydro-4H-thieno[2',3':4,5]pyrrolo[3,2-c]pyridine **2.1(5).** Yield is 68%. LCMS (ESI): *m*/*z* 283 (M+H)⁺. ¹H NMR (DMSO-*d*₆, 400 MHz), δ, ppm. (J, Hz): 2.64 (t, *J₁* = 2.88, *J₂* = 0.90, 3H), 2.86 (m, 1 H), 2.97 (m, 1 H), 3.69 (m, 1 H), 3.75 (m, 1 H), 3.95 (m, 1 H), 6.69 (s, 1 H), 7.20 (t, *J₁* = 7.13, *J₂* = 0.50, 1 H), 7.31 (t, *J₁* = 7.13, *J₂* = 1.39, 2H), 7.40 (t, *J₁* = 7.23, *J₂* = 0.70, 1 H), 9.15 (s, 1 H);

2-methyl-7-ethyl-5,6,7,8-tetrahydro-4H-thieno[2',3':4,5]pyrrolo[3,2-c]pyridine **2.1(6)** Yield is 79%. LCMS (ESI): *m*/*z* 221 (M+H)⁺. ¹H NMR (DMSO-*d*₆, 400 MHz), δ, ppm (*J,* Hz): 1.30 (t, *J₁* = 7.21, *J₂* = 2.88, 3H), 2.55 (t, *J₁* = 12.34, *J₂* = 0.99, 2H), 2.55 (m, 1H), 2.64 (t, *J₁* = 2.88, *J₂* = 0.90, 3H), 2.76 (m, 1 H), 2.85 (m, 1H), 3.55 (m, 1 H), 3.83 (m, 1 H), 6.80 (s, 1 H), 9.15 (s, 1 H);

2-methyl-5-(3-fluorobenzyl)-4,5,6,7-tetrahydro-5H-thieno[3',2':4,5]pyrrolo[3,2-c]pyridine **3.1(4).** Yield is 67%. LCMS (ESI): *m*/*z* 301 (M+H)⁺. ¹H NMR (DMSO-*d*₆, 400 MHz), δ, ppm (*J,* Hz): 2.64 (t, *J₁* = 2.88, *J₂* = 0.99, 3H), 2.74 (m, 1 H), 2.81 (m, 1H), 2.95 (m, 2H), 3.59 (m, 2H), 3.98 (m, 1 H), 4.26 (m, 1 H), 6.31 (s, 1 H), 6.84 (m, 2H), 7.07 (m, 2H), 10.07 (s, 1 H).

### Example 3.

General method for preparation of tetrahydro-thieno-pyrrolo[3,2-*c*]pyridines **1.4,** among them **2.4** and **3.4.** Compound **1.1** (2.28 mmol), aryl iodide or its hetero analogue (2.74 mmol), K₂CO₃ (2.51 mmol) and CuJ (0.684 mmol) were mixed carefully together with *N*-methylpyrrolidinone (5.6 ml) in a hermetically closing vial. The vial was filled with argon, closed and heated at 170°C for 7.5 h. After cooling the contents of the vial was poured into water, extracted with CH₂Cl₂, organic layer was washed with 15% K₂CO₃ solution, dried over unhydrous Na₂SO₄ and evaporated. The final product was isolated by column chromatography, eluent-benzene:ethyl acetate:triethylamine 5:1:0.1. Yield is 9-17%. It gave 2,7-dimethyl-4-(pyridin-4-yl)-5,6,7,8-tetrahydro-4H-thieno[2',3':4,5]pyrrolo[3,2-c]pyridine **2.4(1),** LCMS (ESI): *m*/*z* 284 2.45 (M+H)⁺. ¹H NMR (DMSO-*d*₆, 400 MHz), δ, ppm (*J*, Hz): 2.45 (m, 1 H), 2.55 (t, *J₁* = 13.35, *J₂* = 0.99, 3H), 2.56 (m, 1 H), 2.64 (t, *J₁* = 2.88, *J₂* = 0.90, 3H), 2.89 (m, 2H), 2.49 (q, 1 H), 3.67 (q, 1 H), 6.51 (s, 1 H), 6.64 (m, 2H), 8.62 (m, 2H).

Using analogous procedure and suitable starting materials, compounds **2.4(2), 2.4(3), 2.4(4), 3.4(1), 3.4(3), 3.4(2), 3.4(4)** were prepared.

2,7-dimethyl-4-(3-fluorophenyl)-5,6,7,8-tetrahydro-4H-thieno[2',3':4,5]pyrrolo[3,2-c]pyridine **2.4(3),** LCMS (ESI): *m*/*z* 301 (M+H)⁺. ¹H NMR (DMSO-*d*₆, 400 MHz), δ, ppm (*J*, Hz): 2.46 (m, 1 H), 2.50 (t, *J₁* = 13.35, *J₂* = 0.99, 3H), 2.54 (m, 1 H), 2.64 (t, *J₁* = 2.88, *J₂* = 0.90, 3H), 2.88 (m, 2H), 3.52 (q, 1 H), 3.72(q, 1 H), 6.43 (s, 1 H), 6.80(m, 2H), 6.92 (t, *J₁* = 8.20, *J₂* = 0.80, 1), 7.40 (d, *J* = 8.20);

7-methyl-4-(pyridin-4-yl)-2-chloro-5,6,7,8-tetrahydro-4H-thieno[2',3':4,5]pyrrolo[3,2-c]pyridine **2.4(2),** LCMS (ESI): *m*/*z* 304 (M+H)⁺. ¹H NMR (DMSO-*d*₆, 400 MHz), δ, ppm (*J,* Hz): 2.48 (m, 1H), 2.50 (t, *J₁* = 13.35, *J₂* = 0.99, 3H), 2.52 (m, 1 H), 2.84 (q, 1 H), 2.95 (q, 1 H), 3.48 (m, 1 H), 3.71 (m, 1H), 6.60 (m, 2H), 6.68 (s, 1 H), 8.62 (t, *J₁* = 5.62, *J₂* = 0.95, 2H);

7-methyl-4-*p*-tolyl-2-chloro-5,6,7,8-tetrahydro-4H-thieno[2',3':4,5]pyrrolo[3,2-c]pyridine **2.4(4),** LCMS (ESI): *m*/*z* 317 (M+H)⁺. ¹H NMR (DMSO-*d*₆, 400 MHz), δ, ppm (*J*, Hz): 2.40 (t, *J₁* = 2.88, *J₂* = 0.47, 3H), 2.46 (m, 1 H), 2.50 (t, *J₁* = 13.35, *J₂* = 0.99, 3H), 2.57 (m, 1 H), 2.86 (m, 1 H), 2.95 (m, 1 H), 3.49 (m, 1 H), 3.66 (m, 1 H), 6.99 (m, 2H), 7.23 (m, 2H);

2,5-dimethyl-8-(pyridin-4-yl)-4,5,6,7-tetrahydro-5H-thieno[3',2':4,5]pyrrolo[3,2-c]pyridine **3.4(1),** LCMS (ESI): *m*/*z* 284 (M+H)⁺. ¹H NMR (DMSO-*d*₆, 400 MHz), δ, ppm (*J*, Hz): 2.46 (m, 1 H), 2.54 (m, 1 H), 2.64 (t, *J₁* = 2.88, *J₂* = 0.47, 3H), 2.85 (m, 1 H), 2.92 (t, *J₁* = 13.35, *J₂* = 0.99, 3H), 2.99 (m, 1 H), 3.71 (m, 1H), 3.82(m, 1H), 6.33 (s, 1H), 6.66 (m, 2H), 8.38 (d, *J* = 5.62, 2H);

2,5-dimethyl-8-(3-fluorophenyl)-4,5,6,7-tetrahydro-5H-thieno[3',2':4,5]pyrrolo[3,2-c]pyridine **3.4(3)**, LCMS (ESI): *m*/*z* 301 (M+H)⁺. ¹H NMR (DMSO-*d*₆, 400 MHz), δ, ppm (*J ,* Hz): 2.44 (m, 1 H), 2.52 (m, 1 H), 2.64 (t, *J₁* = 2.88, *J₂* = 0.47, 3H), 2.87 (m, 1H), 2.92 (t, *J*₁ = 13.35, *J₂* = 0.99, 3H), 2.99 (m, 1 H), 3.69 (m, 1 H), 3.80(m, 1 H), 6.33 (s, 1 H), 6.90 (m, 2H), 7.13 (t, *J₁* = 8.20, *J₂* = 0.80, 1 H), 7.15 (d, *J* = 8.20, 1 H);

5-methyl-8-(pyridin-4-yl)-2-chloro-4,5,6,7-tetrahydro-5H-thieno[3',2':4,5]pyrrolo[3,2-c]pyridine **3.4(2),** LCMS (ESI): *m*/*z* 304 (M+H)⁺. ¹H NMR (DMSO-*d*₆, 400 MHz), δ, ppm (*J,* Hz): 2.44 (m, 1 H), 2.53 (m, 1H), 2.84 (m, 1 H), 2.91 (t, *J₁* = 13.35, *J₂* = 0.99, 3H), 3.00 (m, 1 H), 3.71 (m, 1 H), 3.76 (m, 1 H), 6.62 (t, *J₁* = 5.62, *J₂* = 1.77, 2H), 6.89 (s, 1 H), 8.38 (t, *J₁* = 5.62, *J₂* = 0.95, 2H);

5-methyl-8-(6-methylpyridin-3-yl)-2-chloro-4,5,6,7-tetrahydro-5H-thieno[3',2':4,5]pyrrolo[3,2-c]pyridine **3.4(4),** LCMS (ESI): *m*/*z* 318 (M+H)⁺. ¹H NMR (DMSO-*d*₆, 400 MHz), δ, ppm. (*J,* Hz): 2.48 (m, 1 H), 2.56 (m, 1 H), 2.71 (t, *J*₁ = 2.88, *J₂* = 0.47, 3H), 2.87 (m, 1H), 2.95 (t, *J₁* = 13.35, *J₂* = 0.99, 3H), 3.00 (m, 1 H), 3.74 (m, 2H), 6.73 (t, *J₁* = 8.14, *J₂* = 0.49, 1H), 6.96 (s, 1 H), 7.23 (d, *J* = 2.47, 1H), 8.51 (m, 1 H).

**Example 4** General method for preparation of tetrahydro-thieno-pyrrolo[3,2-c]pyridines **1.5.1,** among them **2.5.1** and **3.5.1.** A solution of the corresponding thieno-pyrrolo[3,2-c]pyridine unsubstituted at pyrrole nitrogen **1.2** (1.0 mmol) in anhydrous DMF (5 ml) was added to NaH (60mg, 1.5 mmol) (60% in oil, previously washed with hexane) using external cooling with ice. In 30 min, when the effervescence of hydrogen ceased, benzyl chloride (152 mg, 1.2 mmol) or its heteroanalog was added, the flask was blown through with argon, and the resultant mixture was stirred at room temperature for 12 h. The reaction mixture was diluted with benzene (30 ml), washed twice with water, then with saline, dried over Na₂SO₄, solvent was evaporated in vacuo. Purification was carried out by column chromatography on SiO₂ (eluent hexane : EtOAc = 8:1). It gave the final thieno-pyrrolo[3,2-c]pyridin or its heteroanalogues **1.5.1,** among them **2.5.1** and **3.5.1.**

Ethyl 4-benzyl-2-methyl-4,5,6,8-tetrahydro-4H-thieno[2',3':4,5]pyrrolo[3,2-c]pyridine-7-carboxylate **2.5.1(1).** Yield is 88 %. LCMS (ESI): *m*/*z* 355 (M+H)⁺. ¹H NMR (DMSO-*d*₆, 400 MHz), δ, ppm (*J,* Hz): 7.32 (m, 2H), 7.25 (m, 1H), 7.10 (m, 2H), 6.80 (d, *J* = 0.6, 1 H), 5.18 (s, 2H), 4.41 (br. s, 2H), 4.06 (q, *J* = 7.0, 2H), 3.69 (t, *J =* 5.4, 2H), 2.67 (br. m, 2H), 2.44 (t, *J =* 0.6, 3H), 1.19 (t, *J =* 7.0, 3H). Ethyl 4-benzyl-2-chloro-4,5,6,8-tetrahydro-4H-thieno[2',3':4,5]pyrrolo[3,2-c]pyrdine-7-carboxylate **2.5.1(2).** Yield is 66 %. LCMS (ESI): *m*/*z* 375 (M+H)⁺. ¹H NMR (CDCl₃, 400 MHZ), δ, ppm. (*J*, Hz): 7.31 (m, 3H), 7.04 (m, 2H), 6.72 (s, 1 H), 5.10 (s, 2H), 4.56 (s, 2H), 4.19 (q, *J* = 6.8, 2H), 3.81 (br. m, 2H), 2.69 (br. m, 2H), 1.30 (t, *J* = 6.8, 3H). Ethyl 8-benzyl-2-methyl-4,6,7,8-tetrahydro-5H-thieno[3',2':4,5]pyrrolo[3,2-c]pyridine-5-carboxylate **3.5.1(1).** Yield is 64 %. LCMS (ESI): *m*/*z* 355.2 (M+H)⁺. Ethyl 8-benzyl-2-chloro-4,6,7,8-tetrahydro-5H-thieno[3',2':4,5]pyrrolo[3,2-c]pyridine-5-carboxylate **3.5.1(2).** Yield is 79 %. LCMS (ESI): *m*/*z* 375 (M+H)⁺. ¹H NMR (DMSO-*d*₆, 400 MHz δ, ppm (*J,* Hz): 7.33 (m, 3H), 7.21 (m, 2H), 7.12 (s, 1 H), 5.11 (s, 2H), 4.48 (br. s, 2H), 4.08 (q, *J* = 7.0, 2H), 3.71 (q, *J* = 5.6, 2H), 2.76 (t, *J* = 5.6, 2H), 1.20 (t, *J* = 7.0, 3H).

### Example 5.

General method for preparation of tetrahydro-thieno-pyrrolo[3,2-c]pyridines **1.5.2,** among them **2.5.2** and **3.5.2.** LiAlH₄ (45 mg, 1.2 mmol) (or 243 mg of NaAlH₂(OC₂H₄OCH₃)₂) was added in small portions to a solution of the starting ethyl carboxylate **1.5.1,** among them **2.5.1** and **3.5.1** (0.6 mmol) in Et₂O (40 ml). The reaction mixture was stirred at room temperature for 2 h. After the reaction was completed, H₂O (0.1 ml) was added, and stirring was continued at 20°C for 15 min. Solid was filtered off and washed with ether. The filtrate was washed with 10 % K₂CO₃ solution, dried over Na₂SO₄, and solvent was evaporated. The residue was washed with mixture ether : hexane = 20 : 1 and dried in *vacuo.* It gave thieno-pyrrolo[3,2-*c*]pyridine **1.5.2** or its hetero analogue, among them **2.5.2** and **3.5.2.** Hydrochlorides were prepared by adition of 10 % excess of 3N HCl solution in dioxane to a solution of the base in acetone. 4-Benzyl-2,7-dimethyl-5,6,7,8-tetrahydro-4H-thieno[2',3':4,5]pyrrolo[3,2-c]pyridine **2.5(1).** Yield is 71 %. LCMS (ESI): *m*/*z* 297 (M+H)⁺. 4-Benzyl-2,7-dimethyl-5,6,7,8-tetrahydro-4H-thieno[2',3':4,5]pyrrolo[3,2-c]pyridine hydrochloride **2.5(1)·HCl.** ¹H NMR (DMSO-*d*₆, 400 MHz), δ, ppm: 11.00 (br. s, 1 H), 7.33 (m, 2H), 7.26 (m, 1 H), 7.14 (m, 2H), 6.85 (d, *J* = 0.8, 1 H), 5.23 (s, 2H), 4.37 (br. m, 1H), 4.16 (br. m, 1 H), 3.63 (br. m, 1H), 3.40 (br. m, 1 H), 3.06 (br. m, 1 H), 2.99 (br. m, 1 H), 2.88 (s, 3H), 2.45 (d, *J* = 0.8, 3H). 4-Benzyl-7-methyl-2-chloro-5,6,7,8-tetrahydro-4H-thieno[2',3':4,5]pyrrolo[3,2-c]pyridine 2.5(2). Yield is 67 %. LCMS (ESI): *m*/*z* 317 (M+H)⁺. ¹H NMR (CDCl₃, 400 MHz), δ, ppm *(J,* Hz): 7.29 (m, 3H), 7.04 (m, 2H), 6.69 (s, 1 H), 5.09 (s, 2H), 3.54 (s, 2H), 2.78 (t, *J* = 4.8, 2H), 2.73 (t, *J* = 4.8, 2H), 2.52 (s, 3H). ¹³C NMR (CDCl₃, 75 MHz), δ, ppm: 136.92, 135.18, 129.83, 128.44, 127.20, 126.07, 124.50, 116.72, 110.26, 108.67, 52.10, 51.95, 48.29, 45.26, 22.85.

4-Benzyl-7-methyl-2-chloro-5,6,7,8-tetrahydro-4H-thieno[2',3':4,5]pyrrolo[3,2-c]pyridine hydrochloride **2.5(2)·HCl.** ¹H NMR (DMSO-*d*₆, 400 MHz), δ, ppm (J, Hz): 10.83 (br. s, 1 H), 7.34 (m, 3H), 7.28 (m, 1 H), 7.15 (m, 2H), 5.28 (s, 2H), 4.41 (br. m, 1 H), 4.16 (br. m, 1 H), 3.64 (br. m, 1 H), 3.40 (br. m, 1 H), 3.02 (br. m, 2H), 2.88 (s, 3H). 8-Benzyl-2,5-dimethyl-5,6,7,8-tetrahydro-4H-thieno[3',2':4,5]pyrrolo[3,2-c]pyridine **3.5(1).** Yield is 70 %. LCMS (ESI): *m*/*z* 297 (M+H)⁺. ¹H NMR (CDCl₃, 400 MHz), δ, ppm (*J,* Hz): 7.30 (m, 3H), 7.14 (m, 2H), 6.56 (q, *J* = 0.8, 1 H), 5.03 (s, 2H), 3.68 (br. s, 2H), 2.86 (t, J = 5.2, 2H), 2.80 (t, *J* = 5.2, 2H), 2.56 (s, 3H), 2.46 (d, *J* = 0.8, 3H). ¹³C NMR (CDCl₃, 75 MHz), δ, ppm: 135.80, 132.52, 131.62, 128.60, 128.28, 127.30, 126.68, 125.74, 113.54, 106.09, 51.86, 51.48, 49.28, 43.81, 21.60, 15.71. 8-Benzyl-2,5-dimethyl-5,6,7,8-tetrahydro-4H-thieno[3',2':4,5]pyrrolo[3,2-c]pyridine hydrochloride **3.5(1)·HCl.** ¹H NMR (DMSO-*d*₆, 400 MHz), δ, ppm. (J, Hz): 10.68 (br. s, 1 H), 7.33 (m, 3H), 7.19 (m, 2H), 6.64 (s, 1 H), 5.12 (s, 2H), 4.40 (br. m, 1 H), 4.16 (br. m, 1 H), 3.65 (br. m, 1 H), 3.40 (br. m, 1 H), 3.06 (br. m, 2H), 2.89 (s, 3H), 2.39 (s, 3H). 8-Benzyl-5-methyl-2-chloro-5,6,7,8-tetrahydro-4H-thieno[3',2':4,5]pyrrolo[3,2-c]pyridine **3.5(2).** Yield is 92 %. LCMS (ESI): *m*/*z* 317 (M+H)⁺. ¹H NMR (CDCl₃, 400 MHz), δ, ppm (*J,* Hz): 7.34 (m, 3H), 7.15 (m, 2H), 6.80 (s, 1H), 5.01 (s, 2H), 3.58 (s, 2H), 2.79 (br. s, 4H), 2.53 (s, 3H). ¹³C NMR (CDCl₃, 75 MHz), δ, ppm: 135.36, 130.42, 129.99, 128.48, 127.62, 127.03, 123.79, 120.56, 115.80, 109.00, 52.16, 52.06, 49.35, 45.25, 22.88. 8-Benzyl-5-methyl-2-chloro-5,6,7,8-tetrahydro-4H-thieno[3',2': 4,5]pyrrolo[3,2-c]pyridine hydrochloride **3.5(2)·HCl.** ¹H NMR (DMSO-*d*₆, 400 MHz), δ, ppm (*J,* Hz): 10.82 (br. s, 1H), 7.37 (m, 3H), 7.24 (m, 2H), 7.10 (s, 1H), 5.19 (s, 2H), 4.42 (br. m, 1H), 4.18 (br. m, 1 H), 3.68 (br. m, 1 H), 3.42 (br. m, 1 H), 3.11 (br. m, 2H), 2.91 (s, 3H).

Using the procedures described in examples 4 and 5 compounds **2.5(3), 2.5(4), 2.13(1**), **2.14(1**), **3.5(3), 3.5(4), 3.13(1**), **3.14(1**) were prepared from the corresponding starting materials

4-(3-methylbenzyl)-3-methyl-7-benzyl-5,6,7,8-tetrahydro-4H-thieno[2',3':4,5]pyrrolo[3,2-c]pyridine **2.5(3),** LCMS (ESI): *m*/*z* 387 (M+H)⁺. ¹H NMR (CDCl₃, 400 MHz), δ, ppm (*J,* Hz): 2.25 (d, *J* = 2.88, 3H), 2.32 (d, *J* = 2.88, 3H), 2.78 (m, 1 H), 2.85 (m, 1 H), 3.08 (m, 2H), 3.72 (m, 2H), 3.95 (m, 2H), 4.88 (d, *J* = 7.60, 2H), 6.63 (s, 1 H), 6.90 (m, 2H), 7.06 (d, *J* = 1.51, 1 H), 7.16 (t, *J*₁ = 7.49, *J*₂ = 0.68, 1H), 7.22 (t, *J*₁ = 7.13, *J*₂ = 0.50, 1 H), 7.30 (t, *J*₁ = 7.13, *J*₂ = 0.62, 2H), 7.40 (t, *J*₁ = 7.23, *J*₂ = 0.70, 2H);

4-(6-methylpyridin-3-ylmethyl)-6,7,8-trimethyl-2-chloro-5,6,7,8-tetrahydro-4H-thieno[2',3':4,5]pyrrolo[3,2-c]pyridine **2.5(4),** LCMS (ESI): *m*/*z* 360 (M+H)⁺. ¹H NMR (CDCl₃, 400 MHz), δ, ppm (*J,* Hz): 1.08 (t, *J*₁ = 6.51, *J*₂ = 0.51, 3H), 1.58 (t, *J*₁ = 6.68, *J*₂ = 1.13, 3H), 2.06 (t, *J*₁ = 13.35, *J*₂ = 0.99, 3H), 2.46 (t, *J*₁ = 2.88, *J*₂ = 0.50, 3H), 2.99 (m, 1 H), 3.02 (m, 1 H), 3.16 (t, *J*₁ = 7.93, *J*₂ = 1.46, 1H), 3.74 (t, *J*₁ = 6.68, *J*₂ = 0.99, 1 H), 5.08 (d, *J* = 13.17, 2H), 6.50 (s, 1 H), 7.13 (t, *J*₁ = 7.75, *J*₂ = 0.56, 2H), 7.93 (t, *J*₁ = 2.07, *J*₂ = 0.24, 1 H); 2,10-dimethyl-4-(pyridin-3-ylmethyl)-4,5,6,7,8,9-hexahydro-6,9-epiminocyclohepta[b]thieno[2,3-d]pyrrole **2.13(1)** LCMS (ESI): *m*/*z* 324 (M+H)⁺. ¹H NMR (CDCl₃, 400 MHz), δ, ppm (*J,* Hz): 1.99 (m, 2H), 2.10 (t, *J*₁ = 13.35, *J*₂ = 0.99, 3H), 2.18 (m, 1 H), 2.30 (m, 1 H), 2.64 (t, *J*₁ = 2.88, *J*₂ = 0.90, 3H), 3.05 (d, *J* = 4.60, 1H), 3.11 (m, 1H), 3.30 (m, 1H), 3.60 (m, 1 H), 5.13 (d, *J* = 13.17, 2H), 6.99 (s, 1H), 7.39 (t, *J*₁ = 7.90, *J*₂ = 0.81, 1 H), 7.58 (d, *J* = 2.05, 1 H), 8.37 (s, 1 H), 8.65 (d, *J* = 0.43, 1 H);
9-benzyl-4-(3-fluorobenzyl)-2-methyl-5,6,7,8,-tetrahydro-4H-8,5-(epiminomethano) thieno[3,2-b]indole **2.14(1):** LCMS (ESI): *m*/*z* 417 (M+H)⁺. ¹H NMR (CDCl₃, 400 MHz), δ, ppm (*J,* Hz): 1.61 (m, 1 H), 1.86 (m, 1 H), 2.45 (m, 2H), 2.64 (t, *J*₁ = 2.88, *J*₂ = 0.90, 3H), 2.73(m, 1 H), 3.25 (m, 1 H), 3.46 (s, 1 H), 3.56 (m, 1 H), 3.76 (s, 1 H), 3.92 (m, 1 H), 5.20 (t, *J*₁ = 7.60, *J*₂ = 0.30, 2H), 6.62 (d, *J* = 0.70, 2H), 6.89 (s, 1 H), 6.95 (m, 2H), 7.24 (m, 3H), 7.46 (t, *J*₁ = 2.23, *J*₂ = 2.12, 2H);
8-(pyridin-4-ylmethyl)-3-methyl-5-benzyl-4,5,6,7-tetrahydro-5H-thieno[3',2':4,5]pyrrolo[3,2-c]pyridine **3.5(3),** LCMS (ESI): *m*/*z* 374 (M+H)⁺. ¹H NMR (CDCl₃, 400 MHz), δ, ppm (*J,* Hz): 2.52 (s, 3H), 2.82 (m, 2H), 3.10 (m, 2H), 3.76 (t, *J*₁ = 7.60, *J*₂ = 0.50, 2H), 4.04 (m, 1 H), 4.30 (m, 1 H), 5.08 (s, 2H), 6.53 (s, 2H), 6.64 (s, 1H), 7.22 (t, *J*₁ = 7.13, *J*₂ = 0.50, 1 H), 7.30 (t, *J*₁ = 7.13, *J*₂ = 1.39, 2H), 7.38 (t, *J*₁ = 7.23, *J*₂ = 0.62, 2H), 8.73 (s, 2H);
8-(3-fluorobenzyl)-4,5,6-trimethyl-2-chloro-4,5,6,7-tetrahydro-5H-thieno[3',2':4,5]pyrrolo[3,2-c]pyridine **3.5(4)** LCMS (ESI): *m*/*z* 363 (M+H)⁺. ¹H NMR (CDCl₃, 400 MHz), δ, ppm (*J,* Hz): 1.08 (t, *J*₁ = 6.51, *J*₂ = 0.50, 3H), 1.49 (t, *J*₁ = 6.68, *J*₂ = 1.13, 3H), 2.06 (t, *J*₁ = 13.35, *J*₂ = 0.99, 3H), 2.97 (m, 1 H), 3.04 (m, 1 H), 3.19 (d, *J* = 7.93, 1H), 4.19 (t, *J*₁ = 6.68, *J*₂ = 0.99, 1 H), 5.32 (t, *J*₁ = 13.17, *J*₂ = 0.30, 2H), 6.48 (s, 1 H), 6.56 (d, *J* = 0.70, 1 H), 6.86 (d, *J* = 1.71, 1 H), 6.95 (t, *J*₁ = 1.59, *J*₂ = 0.50, 1 H), 7.40 (t, *J*₁ = 8.12, *J*₂ = 0.40, 1 H);
9-benzyl-2,10-dimethyl-4,5,6,7,8,9-hexahydro-4,7-epiminocyclohepta[b]thieno[3,2-d]pyrrole **3.13(1)** LCMS (ESI): *m*/*z* 323 (M+H)⁺. ¹H NMR (CDCl₃, 400 MHz), δ, ppm (J, Hz): 1.70 (m, 1 H), 2.01 (m, 2H), 2.10 (d, *J* = 13.35, 3H), 2.17 (m, 1 H), 2.64 (t, *J*₁ = 7.60, *J*₂ = 2.88, 3H), 3.05 (m, 1 H), 3.11 (m, 1 H), 3.61 (m, 1 H), 4.22 (m, 1 H), 5.28 (d, *J* = 7.60, 2H), 5.85 (s, 1 H), 6.88 (m, 2H), 7.24 (t, *J*₁ = 7.13, *J*₂ = 1.80, 1 H), 7.50 (t, *J*₁ = 7.23, *J*₂ = 0.62, 2H);
10-benzyl-8-(3-fluorobenzyl)-2-methyl-5,6,7,8-tetrahydro-4H-4,7-(epiminomethano)thieno[2,3-b]indole **3.14(1)** LCMS (ESI): m/z 417 (M+H)⁺. ¹H NMR (CDCl₃, 400 MHz), δ, ppm (*J,* Hz): 1.61 (m, 1 H), 1.85 (m, 1 H), 2.33 (m, 2H), 2.64 (d, J = 2.88, 3H), 2.73 (m, 1H), 3.24 (m, 1 H), 3.46 (m, 1 H), 3.56 (m, 1H), 3.90 (m, 1H), 4.00 (m, 1H), 5.31 (m, 2H), 5.96 (s, 1 H), 6.40 (d, *J* = 1.71, 1 H), 6.71 (d, *J* = 0.70, 1 H), 6.97 (t, *J*₁ = 8.12, *J*₂ = 0.95, 1 H); 7.18 (d, *J* = 7.49, 1 H), 7.26 (t, *J*₁ = 7.13, *J*₂ = 0.50, 3H); 7.49 (t, *J*₁ = 2.12, *J*₂ = 0.70, 2H).

### Example 6.

General method for preparation of ethyl tetrahydro-thieno-pyrrolo[3,2-*c*]pyridine-carboxylates **1.6.1,** among them **2.6.1** and **3.6.1.** A solution of the corresponding thieno-pyrrolo[3,2-*c*]pyridine unsubstituted at pyrrole nitrogen **1.2** (1.0 mmol) in anhydrous DMF (5 ml) was added to NaH (60mg, 1.5 mmol) (60% in oil, previously washed with hexane) using external cooling with ice. In 30 min, when the effervescence of hydrogen ceased, phenethyl bromide (1.3 mmol) was added, the flask was blown through with argon, and the resultant mixture was stirred at 20°C for 30 min. Then, at 0°C the reaction mixture was added to a second portion of NaH (1.5 mmol), stirred for 30 min, added substituted 2-bromoethane (1.3 mmol), the fkask was blown with argon, the mixture was stirred at 20°C for 30 min. The operation was repeated 1-3 times until the starting material **1.2** was completely disappeared (LCMS control). The reaction mixture was diluted with benzene (30 ml), washed twice with water, then with saline, dried over Na₂SO₄, solvent was evaporated in *vacuo.* Purification was carried out by column chromatography on SiO₂ (eluent hexane : EtOAc = 10:1). It gave compounds **1.6.1,** among them **2.6.1** and **3.6.1.** Ethyl 2-methyl-4-(2-phenylethyl)-4,5,6,8-tetrahydro-4H-thieno[2',3':4,5]pyrrolo[3,2-c]pyridine-7-carboxylate **2.6.1(1).** Yield is 39 %. LCMS (ESI): *m*/*z* 369 (M+H)⁺. ¹H NMR (DMSO-*d*₆, 400 MHz), δ, ppm (*J,* Hz): 7.22 (m, 3H), 7.09 (br. m, 2H), 6.86 (d, *J* = 1.2, 1 H), 4.35 (s, 2H), 4.19 (t, *J* = 7.0, 2H), 4.06 (q, *J* = 7.2, 2H), 3.56 (t, *J* = 5.4, 2H), 2.90 (t, *J* = 7.0, 2H), 2.47 (s, 3H), 2.41 (br. m, 2H), 1.19 (t, *J* = 7.2, 3H). Ethyl 4-(2-phenylethyl)-2-chloro-4,5,6,8-tetrahydro-4H-thieno[2',3':4,5]pyrrolo[3,2-c]pyridine-7-carboxylate 2.6.1(2). Yield is 62 %. LCMS (ESI): *m*/*z* 389 (M+H)⁺. ¹H NMR (CDCl₃, 400 MHz), δ, ppm (*J,* Hz): 7.24 (m, 3H), 6.96 (br. m, 2H), 6.77 (s, 1 H), 4.49 (s, 2H), 4.18 (q, *J* = 7.2, 2H), 4.09 (t, *J* = 7.0, 2H), 3.64 (br. m, 2H), 2.97 (t, *J =* 7.0, 2H), 2.36 (br. s, 1 H), 2.28 (br. s, 1 H), 1.30 (t, *J* = 7.2, 3H). Ethyl 2-methyl-8-(2-phenylethyl)-4,6,7,8-tetrahydro-5H-thieno[3',2':4,5]pyrrolo[3,2-c]pyridine-5-carboxylate **3.6.1(1).** Yield is 74 %. LCMS (ESI): *m*/*z* 369 (M+H)⁺. ¹H NMR (CDCl₃, 400 MHz), δ, ppm (*J,* Hz): 7.26 (m, 3H), 7.02 (br. m, 2H), 6.63 (d, *J* = 1.2, 1 H), 4.55 (s, 2H), 4.17 (q, *J* = 7.2, 2H), 4.04 (t, *J* = 7.2, 2H), 3.63 (br. m, 2H), 3.04 (t, *J* = 7.2, 2H), 2.54 (s, 3H), 2.37 (br. s, 1 H), 2.28 (br. s, 1 H), 1.29 (t, *J* = 7.2, 3H).

### Example 7.

General method for preparation of tetrahydro-thieno-pyrrolo[3,2-c]pyridines **1.6.2,** among them **2.6.2** and **3.6.2.** LiAlH₄ (45 mg, 1.2 mmol) (or 243 mg of NaAlH₂(OC₂H₄OCH₃)₂) was added in small portions to a solution of the starting ethyl carboxylate **1.6.1,** (0.6 mmol) in Et₂O (40 ml). The reaction mixture was stirred at 20°C for 2 h. After the reaction was completed, H₂O (0.1 ml) was added, and stirring was continued at 20°C for 15 min. Solid was filtered off and washed with ether. The filtrate was washed with 10 % K₂CO₃ solution, dried over Na₂SO₄, and solvent was evaporated. The residue was washed with mixture ether: hexane = 20 : 1 and dried in *vacuo.* It gave compounds **1.6.2,** among them **2.6.2** and **3.6.2.** Hydrochlorides were prepared by adition of 10 % excess of 3N HCl solution in dioxane to a solution of the base in acetone. 2,7-Dimethyl-4-(2-phenylethyl)-5,6,7,8-tetrahydro-4H-thieno[2',3':4,5]pyrrolo[3,2-c]pyridine **2.6(1).** Yield is 68 %. LCMS (ESI): *m*/*z* 311 (M+H)⁺. 2,7-Dimethyl-4-(2-phenylethyl)-5,6,7,8-tetrahydro-4H-thieno[2',3':4,5]pyrrolo[3,2-c]pyridine hydrochloride **2.6(1)·HCl.** ¹H NMR (DMSO-*d*₆, 400 MHz), δ, ppm (*J,* Hz): 10.83 (br. s, 1 H), 7.27 (m, 2H), 7.21 (m, 1 H), 7.14 (m, 2H), 6.88 (d, *J =* 1.2, 1 H), 4.34 (br. m, 1 H), 4.14 (m, 3H), 3.56 (br. m, 1 H), 3.26 (br. m, 1 H), 2.97 (br. m, 1 H), 2.93 (t, *J* = 7.2, 2H), 2.83 (s, 3H), 2.66 (br. m, 1 H), 2.47 (s, 3H). 7-Methyl-4-(2-phenylethyl)-2-chloro-5,6,7,8-tetrahydro-4H-thieno[2',3':4,5]pyrrolo[3,2-c]pyridine **2.6(3).** Yield is 62 %. LCMS (ESI): *m*/*z* 331 (M+H)⁺. ¹H NMR (CDCl₃, 400 MHz), δ, ppm (J, Hz): 7.26 (m, 3H), 7.03 (m, 2H), 6.71 (s, 1 H), 4.08 (t, *J =* 7.2, 2H), 3.49 (s, 2H), 2.97 (t, *J =* 7.2, 2H), 2.69 (t, *J =* 5.6, 2H), 2.53 (t, *J* = 5.6, 2H), 2.49 (s, 3H). 7-Methyl-4-(2-phenylethyl)-2-chloro-5,6,7,8-tetrahydro-4H-thieno[2',3':4,5]pyrrolo[3,2-c]pyridine hydrochloride **2.6(3)·HCl.** ¹H NMR (DMSO-*d*₆, 400 MHz), δ, ppm. (*J,* Hz): 10.82 (br. s, 1 H), 7.29 (s, 1 H), 7.26 (m, 2H), 7.20 (m, 1H), 7.14 (m, 2H), 4.36 (br. m, 1 H), 4.21 (br. m, 2H), 4.13 (br. m, 1H), 3.57 (br. m, 1H), 3.33 (br. m, 1 H), 2.96 (br. m, 1 H), 2.93 (t, *J =* 7.0, 2H), 2.84 (s, 3H), 2.75 (br. m, 1 H). 2,5-Dimethyl-8-(2-phenylethyl)-5,6,7,8-tetrahydro-4H-thieno[3',2':4,5]pyrrolo[3,2-c]pyridine **3.6(1).** Yield is 97 %. LCMS (ESI): *m*/*z* 311 (M+H)⁺. ¹H NMR (CDCl₃, 400 MHz), δ, ppm. (*J,* Hz): 7.28 (m, 3H), 7.11 (m, 2H), 6.61 (q, *J* = 1.2, 1 H), 4.05 (t, *J* = 7.4, 2H), 3.57 (s, 2H), 3.06 (t, *J* = 7.4, 2H), 2.69 (t, *J* = 5.6, 2H), 2.54 (d, *J* = 1.2, 3H), 2.53 (t, *J* = 5.6, 2H), 2.50 (s, 3H). ¹³C NMR (CDCl₃, 75 MHz), δ, ppm: 138.08, 131.15, 130.90, 129.72, 128.44, 128.18, 126.22, 126.02, 114.10, 107.77, 52.42, 52.13, 47.24, 45.13, 35.53, 22.51, 15.91. 2,5-Dimethyl-8-(2-phenylethyl)-5,6,7,8-tetrahydro-4H-thieno[3',2':4,5]pyrrolo[3,2-c]pyridine hydrochloride **3.6(1)·HCl.** ¹H NMR (DMSO-*d*₆, 400 MHz), δ, ppm (J, Hz): 10.81 (br. s, 1 H), 7.26 (m, 2H), 7.20 (m, 1 H), 7.10 (m, 2H), 6.65 (s, 1 H), 4.35 (br. m, 1 H), 4.10 (m, 3H), 3.52 (br. m, 1 H), 3.23 (br. m, 1 H), 2.98 (t, *J =* 6.8, 2H), 2.89 (br. m, 1 H), 2.81 (s, 3H), 2.60 (br. m, 1H), 2.44 (s, 3H).

Using the procedures described in examples 6 and 7 compounds **2.6(2), 2.6(4), 2.6(5), 2.14(2), 3.6(4)** were prepared from the corresponding starting materials.

(7-methyl-4-phenethyl-5,6,7,8-tetrahydro-4H-thieno[2',3':4,5]pyrrolo[3,2-c]pyridin-2-yl)-methanol **2.6(2),** LCMS (ESI): *m*/*z* 327 (M+H)⁺. ¹H NMR (CDCl₃, 400 MHz), δ, ppm (*J,* Hz): 2.43 (m, 1 H), 2.50 (m, 1 H), 2.87 (d, *J* = 6.91, 2H), 2.91 (t, *J*₁ = 13.35, *J₂*= 0.99, 3H), 2.97 (d, *J* = 17.12, 2H), 3.63 (s, 1H), 3.70 (m, 2H), 4.20 (d, *J* = 14.00, 2H), 6.58 (m, 1H), 6.65 (s, 1H), 7.21 (t, *J*₁ = 7.13, *J₂*= 1.38, 1 H), 7.26 (d, *J* = 1.11, 2H), 7.30 (t, *J*₁ = 7.07, *J₂*= 1.39, 2H);
3,5,7-trimethyl-4-(2-(pyridin-3-yl)ethyl)-5,6,7,8-tetrahydro-4H-thieno[2',3':4,5]pyrrolo[3,2-c]pyridine **2.6(4),** LCMS (ESI): *m*/*z* 326 (M+H)⁺. ¹H NMR (CDCl₃, 400 MHz), δ, ppm (*J,* Hz): 1.10 (m, 3H), 2.08 (m, 1 H), 2.31 (d, *J* = 2.88, 3H), 2.37 (t, *J*₁ = 13.35, *J₂*= 0.99, 3H), 2.86 (m, 1H), 3.10 (d, *J* = 6.91, 2H), 3.19 (t, *J*₁ = 6.60, *J₂*= 4.20, 1H), 3.42 (m, 1 H), 3.62 (m, 1 H), 3.92 (d, *J* = 14.00, 2H), 6.79 (s, 1 H), 7.35 (t, *J*₁ = 7.90, *J₂*= 0.77, 1 H), 7.98 (d, *J =* 1.64, 1 H), 8.42 (d, *J* = 1.60, 1H), 8.83 (s, 1 H);
7-methyl-4-(2-(pyridin-4-yl)ethyl)-2-chloro-5,6,7,8-tetrahydro-4H-thieno[2',3':4,5]pyrrolo[3,2-c]pyridine **2.6(5),** LCMS (ESI): *m*/*z* 332 (M+H)⁺. ¹H NMR (CDCl₃, 400 MHz), δ, ppm. (*J,* Hz): 2.41 (m, 2H), 2.50 (t, *J*₁ = 13.35, *J₂*= 0.99, 3H), 2.90 (m, 2H), 3.02 (m, 2H), 3.44 (m, 1 H), 3.65 (m, 1H), 4.01 (d, *J* = 14.00, 2H), 6.93 (s, 1 H), 7.19 (m, 2H), 8.51 (m, 2H);
2-chloro-9-methyl-4-(2-phenylethyl)-5,6,7,8-tetrahydro-4H-8,5-(epiminomethano)thieno[3,2-b]indole **2.14(2):** LCMS (ESI): *m*/*z* 357 (M+H)⁺. ¹H NMR (CDCl₃, 400 MHz), δ, ppm. (*J,* Hz): 1.60 (m, 1 H), 1.86 (m, 1 H), 2.38 (t, *J*₁ = 13.35, *J₂*= 0.99, 3H), 2.35 (m, 1 H), 2.42 (m, 1 H), 2.55 (m, 1 H), 2.83 (d, *J =* 7.00, 2H), 3.07 (m, 1 H), 3.32 (m, 1 H), 3.55 (m, 1 H), 4.18 (d, *J* = 14.00, 2H), 6.85 (d, *J* = 2.30, 2H), 6.97 (s, 1 H), 7.23 (t, *J*₁ = 7.13, *J₂*= 1.38, 1 H), 7.29 (t, *J*₁ = 7.07, *J₂*= 0.77, 2H);
2,5,7-trimethyl-8-(4-chlorophenethyl)-4,5,6,7-tetrahydro-5H-thieno[3',2':4,5]pyrrolo[3,2-c]pyridine **3.6(4),** LCMS (ESI): *m*/*z* 359 (M+H)⁺. ¹H NMR (CDCl₃, 400 MHz), δ, ppm. (*J,* Hz): 1.11(t, *J*₁ = 6.60, *J₂*= 0.93, 3H); 2.07 (m, 1 H), 2.36 (t, *J*₁ = 13.35, *J₂*= 0.99, 3H), 2.64 (d, *J* = 2.88, 3H), 2.86 (m, 2H), 3.19 (m, 1 H), 3.63 (m, 1 H), 3.72 (m, 2H), 4.22 (d, *J* = 14.00, 2H), 6.12 (s, 1 H), 7.15 (t, *J*₁ = 2.20, *J₂*= 0.50, 2H); 7.26 (t, *J*₁ = 8.20, *J₂*= 0.50, 2H).

### Example 8.

General method for preparation of chloro substituted tetrahydro-4*H*-thieno-pyrrolo[3,2-c]pyridines **1.6,** among them **2.6** and **3.6.** A solution of PBr₃ (121 mkl, 1.29 mmol) and pyridine (53.5 mkl) in toluene (0.5 ml) was added dropwise to a suspension of compound **1.7** (1.07 mmol) (synthesis of which is described in the following example) in DMF (2 ml) at external cooling with ice, and the resultant mixture was stirred for 12 h at 20°C. Then, the mixture was evaporated in *vacuo,* crushed ice was added to the residue, the solid obtained was filtered off, washed with water and dried in the air. According to ¹H NMR and LCMS data the product represents a mixture of two compounds: compound **6** and a product of HBr elimination with a double bond in ratio 2:3. The obtained mixture was used in the next stage without separation, for what it was dissolved in AcOH (20 ml), then Zn (495 mg, 7.61 mmol) was added and stirring was continued for 6 h at 90 °C. The reaction mixture was evaporated in *vacuo,* the residue was treated with water (100 ml), and extracted with CH₂Cl₂ after addition of concentrated water NH₃ (10 ml). Organic extract was washed with water several times to pH 7, dried over Na₂SO₄ and evaporated in *vacuo.* Compounds **1.6,** among them **2.6** and **3.6,** were isolated by HPLC. Hydrochloride was prepared by addition of 10 % excess of 3N HCl solution in dioxane to a solution of the base **1.6,** among them **2.6** and **3.6** in acetone. 5-Methyl-8-(2-phenylethyl)-2-chloro-5,6,7,8-tetrahydro-4H-thieno[3',2':4,5]pyrrolo[3,2-c]pyridine hydrochloride **3.6(3)·HCl.** ¹H NMR (DMSO-*d*₆, 400 MHz), δ, ppm (*J,* Hz): 10.58 (br. s, 1 H), 7.23 (m, 3H), 7.10 (m, 2H), 7.08 (s, 1 H), 4.37 (br. m, 1 H), 4.17 (m, 2H), 4.12 (br. m, 1 H), 3.56 (br. m, 1 H), 3.37 (br. m, 1 H), 2.98 (t, *J* = 6.8, 2H), 2.91 (br. m, 1 H), 2.84 (s, 3H), 2.70 (br. m, 1 H).

Analogous procedure was applied to the synthesis of compounds **3.6(5), 3.14(2).**

5-Methyl-8-(2-(pyridin-4-yl)ethyl)-2-chloro-4,5,6,7-tetrahydro-5H-thieno[3',2':4,5]pyrrolo[3,2-c]pyridine **3.6(5)** LCMS (ESI): *m*/*z* 332 (M+H)⁺. ¹H NMR (CDCl₃, 400 MHz), δ, ppm. (*J,* Hz): 2.44 (m, 1 H), 2.50 (m, 1 H), 2.83 (m, 1 H), 2.91 (t, *J*₁ = 13.35, *J₂*= 0.99, 3H), 3.00 (d, *J* = 6.91, 2H), 3.67(m, 2H), 3.74 (m, 1 H), 4.21 (d, *J* = 2.33, 2H), 6.67 (s, 1 H), 7.38 (t, *J*₁ = 5.68, *J₂*= 0.70, 2H), 8.56 (t, *J*₁ = 5.68, *J₂*= 0.30, 2H);
2-chloro-10-methyl-8-(2-phenylethyl)-5,6,7,8-tetrahydro-4H-4,7-(epiminomethano)thieno[2,3-b]indole **3.14(2)** LCMS (ESI): *m*/*z* 357 (M+H)⁺. ¹H NMR (CDCl₃, 400 MHz), δ, ppm. *(J,* Hz): 1.60 (m, 1H), 1.86 (m, 1H), 2.22 (m, 2H), 2.38 (t, *J*₁ = 13.35, *J₂*= 0.99, 3H), 2.55 (m, 1 H), 2.67 (d, *J =* 6.91, 2H), 3.10 (t, *J*₁ = 13.20, *J₂*= 2.57, 1 H), 3.32 (m, 1H), 3.83 (m, 1 H), 4.37 (d, *J* = 14.00, 2H), 6.54 (s, 1 H), 7.06 (d*, J =* 1.11, 2H), 7.25 (t, *J*₁ = 7.13, *J₂*= 1.38, 1 H), 7.32 (t, *J*₁ = 7.07, *J₂*= 0.50, 21 H).

### Example 9.

General method for preparation of tetrahydro-thieno-pyrrolo[3,2-c]pyridines **1.7,** among them **2.7** and **3.7.** Aryl- (1.1 mmol) or heteroaryl oxirane and anhydrous K₃PO₄ (0.414 g, 2 mmol) were added to a solution of thieno-pyrrolo[3,2-*c*]pyridine unsubstituted at pyrrole nitrogen (1 mmol) **1.1** in DMF (5 ml). The reaction mixture was stirred under argon at 60°C for 18 h (LCMS control). After that the reaction mixture was poured into 5% NaHCO₃ solutuon, precipitated solid was filtered off, washed with water and dried in *vacuo.* The reaction product was isolated by column chromatogtaphy on SiO₂ (eluent hexane : EtOAc : Et₃N = 2:4:0.1). It gave corresponding thieno-pyrrolo[3,2-*c*]pyridines **1.7,** among them **2.7** and **3.7.** Hydrochlorides were prepared by addition of 10 % excess of 3N HCl solution in dioxane to a solution of the base in acetone. 2-(2,5-Dimethyl-4,5,6,7-tetrahydro-5H-thieno[3',2':4,5]pyrrolo[3,2-c]pyridin-8-yl)-1-phenyl-ethanol **3.7(1).** Yield is 61 %. LCMS (ESI): *m*/*z* 327 (M+H)⁺. ¹H NMR (CDCl₃, 400 MHz), δ, ppm (*J,* Hz): 7.31 (m, 5H), 6.49 (q, *J* = 0.8, 1 H), 4.83 (br. s, 1 H), 4.73 (dd, *J*₁ = 8.4, *J*₂ = 3.6, 1 H), 3.89 (dd, *J*₁ = 14.4, *J*₂ = 8.4, 1 H), 3.82 (dd, *J*₁ = 14.4, *J*₂ = 3.6, 1 H), 3.30 (m, 2H), 2.64 (m, 3H), 2.53 (m, 1 H), 2.52 (d, *J =* 0.8, 3H), 2.32 (s, 3H). ¹³C NMR (CDCl₃, 75 MHz), δ, ppm.: 141.94, 132.20, 130.87, 130.08, 127.99, 127.32, 125.83, 125.47, 113.87, 107.53, 72.76, 53.81, 52.18, 52.10, 44.87, 22.63, 15.87. 2-(2,5-Dimethyl-4,5,6,7-tetrahydro-5H-thieno[3',2':4,5]pyrrolo[3,2-c]pyridin-8-yl)-1-phenylethanol hydrochloride **3.7(1)·HCl.** ¹H NMR (DMSO-*d*₆, 400 MHz), δ, ppm (J, Hz): 10.67 (br. s, 1 H), 7.30 (m, 5H), 6.63 (s, 1 H), 5.78 (br. s, 1 H), 4.88 (m, 1 H), 4.37 (br. m, 1 H), 4.12 (br. m, 1H), 3.98 (br. m, 2H), 3.54 (br. m, 1 H), 3.25 (br. m, 1 H), 2.94 (br. m, 1H), 2.84 (s, 3H), 2.64 (br. m, 1 H), 2.44 (s, 3H). 1-(5-Methyl-2-chloro-4,5,6,7-tetrahydro-5H-thieno[3',2':4,5]pyrrolo[3,2-c]pyridin-8-yl)-1-phenylethanol **3.7(2).** Yield is 57 %. LCMS (ESI): *m*/*z* 347 (M+H)⁺. 2-(5-Methyl-2-chloro-4,5,6,7-tetrahydro-5H-thieno[3',2':4,5]pyrrolo[3,2-c]pyridin-8-yl)-1-phenylethanol hydrochloride **3.7(2)·HCl.** ¹H NMR (DMSO-*d*₆, 400 MHz), δ, ppm (J, Hz): 10.81 (br. s, 1 H), 7.30 (m, 5H), 7.05 (s, 1 H), 5.88 (d, *J =* 3.2, 1 H), 4.90 (br. m, 1 H), 4.37 (br. m, 1 H), 4.12 (br. m, 1 H), 4.03 (d, *J* = 6.4, 2H), 3.55 (br. m, 1H), 3.30 (br. m, 1 H), 2.95 (br. m, 1 H), 2.84 (s, 3H), 2.70 (br. m, 1 H).

In analogous manner, using the corresponding starting materials the following compounds: **2.7(1), 2.7(3), 2.7(2), 2.7(4), 3.7(3), 3.7(4)** were prepared.

2-(2,7-Dimethyl-5,6,7,8-tetrahydro-4H-thieno[2',3':4,5]pyrrolo[3,2-c]pyridin-4-yl)-1-phenylethanol **2.7(1)**, LCMS (ESI): m/z 327 (M+H)⁺. ¹H NMR (CDCl₃, 400 Mhz), δ, ppm (*J,* Hz): 2.43 (m, 1H), 2.50 (m, 1 H), 2.60 (t, *J*₁ = 13.35, *J₂*= 0.99, 3H), 2.64 (d, *J =* 2.88, 3H), 2.95 (m, 2H), 3.47 (m, 1 H), 3.64 (m, 2H), 3.68 (m, 1 H), 4.00 (m, 1 H), 4.75 (t, *J*₁ = 7.95, *J*₂= 4.75, 1 H), 6.91 (s, 1H), 7.22 (t, *J*₁ = 7.13, *J₂*= 1.57, 1 H), 7.32 (t, *J*₁ = 7.14, *J₂*= 0.69, 2H), 7.40 (d, *J=* 0.67, 2H);
2-(2,5,7-trimethyl-5,6,7,8-tetrahydro-4H-thieno[2',3':4,5]pyrrolo[3,2-c]pyridin-4-yl)-1-(6-methylpyridin-3-yl)-ethanol 2.7(3), LCMS (ESI): m/z 356 (M+H)⁺. ¹H NMR (CDCl₃, 400 MHz), δ, ppm (J, Hz): 1.11 (t, *J*₁ = 6.60, *J₂*= 0.93, 3H), 2.10 (t, *J*₁ = 4.20, *J₂*= 1.51, 1 H), 2.35 (t, *J*₁ = 13.35, *J₂*= 0.99, 3H), 2.48 (t, *J*₁ = 2.88, *J₂*= 0.70, 3H), 2.64 (d, *J=* 2.88, 3H), 2.89 (m, 1 H), 3.19 (m, 1 H), 3.48 (m, 2H), 3.61 (m, 2H), 3.94 (m, 1 H), 4.99 (t, *J*₁ = 7.95, *J₂*= 4.75, 1 H), 6.89 (d, *J =* 2.33, 1 H), 7.03 (t, *J*₁ = 7.60, *J₂*= 0.70, 1 H), 7.44 (t, *J*₁ = 2.00, *J₂*= 0.67, 1 H), 8.37 (s, 1 H);
2-(7-methyl-2-chloro-5,6,7,8-tetrahydro-4H-thieno[2',3':4,5]pyrrolo[3,2-c]pyridin-4-yl)-1-phenyl-ethanol **2.7(2),** LCMS (ESI): *m*/*z* 347 (M+H)⁺. ¹H NMR (CDCl₃, 400 MHz), δ, ppm (*J,* Hz): 2.43 (m, 1 H), 2.50 (t, *J*₁ = 13.35, *J₂*= 0.99, 4H), 2.97 (m, 2H), 3.47 (m, 1 H), 3.68 (m, 3H), 3.97 (m, 1 H), 4.74 (t, *J*₁ = 7.95, *J₂*= 2.33, 1 H), 7.10 (s, 1H), 7.22 (t, *J*₁ = 7.13, *J₂*= 1.57, 1H), 7.33 (t, *J*₁ = 7.14, *J₂*= 0.69, 2H), 7.42 (t, *J*₁ = 2.20, *J₂*= 0.67, 2H);
2-(5,7-dimethyl-2-chloro-5,6,7,8-tetrahydro-4H-thieno[2',3':4,5]pyrrolo[3,2-c]pyridin-4-yl)-1-(3-chlorophenyl)-ethanol **2.7(4),** LCMS (ESI): *m*/*z* 395 (M+H)⁺. ¹H NMR (CDCl₃, 400 MHz), δ, ppm (*J,* Hz): 1.11 (d, *J =* 0.93, 3H), 2.08 (m, 1 H), 2.35 (t, *J*₁ = 13.35, *J₂*= 0.99, 3H), 2.89 (m, 1 H), 3.19 (m, 1 H), 3.46 (m, 1 H), 3.63 (m, 2H), 3.70 (m, 1 H), 3.97 (d, *J =* 13.83, 1 H), 4.68 (t, *J*₁ = 7.95, *J₂*= 2.33, 1 H), 7.05 (t, *J*₁ = 7.58, *J₂*= 0.69, 1 H), 7.08 (s, 1 H), 7.22 (t, *J*₁ = 7.58, *J₂*= 1.15, 2H), 7.36 (t, *J*₁ = 2.03, *J₂*= 0.43, 1 H);
2-(2,5,7-trimethyl-4,5,6,7-tetrahydro-5H-thieno[3',2':4,5]pyrrolo[3,2-c]pyridin-8-yl)-1-*p*-tolyl-ethanol **3.7(3),** LCMS (ESI): *m*/*z* 355 (M+H)⁺. ¹H NMR (CDCl₃, 400 MHz), δ, ppm (*J*, Hz): 1.11 (t, *J*₁ = 6.60, *J₂*= 0.93, 3H), 2.07 (m, 1 H), 2.28 (d, *J*= 2.88, 3H), 2.35 (t, *J*₁ = 13.35, *J₂*= 0.99, 3H), 2.64 (d, *J=* 2.88, 3H), 2.88 (m, 1 H), 3.19 (m, 1 H), 3.70 (m, 3H), 3.84 (m, 1 H), 4.17 (m, 1H), 4.64 (t, *J*₁ = 7.95, *J₂*= 4.75, 1 H), 6.16 (s, 1 H), 7.22 (t, *J*₁ = 7.75, *J₂*= 0.63, 2H), 7.55 (t, *J*₁ = 2.20, *J₂*= 0.67, 2H);
2-(5,7-dimethyl-3-chloro-4,5,6,7-tetrahydro-5H-thieno[3',2':4,5]pyrrolo[3,2-c]pyridin-8-yl)-1-(3-chlorophenyl)-ethanol **3.7(4)** LCMS (ESI): *m*/*z* 395 (M+H)⁺. ¹H NMR (CDCl₃, 400 MHz), δ, ppm (*J,* Hz): 1.10 (t, *J*₁ = 6.60, *J₂*= 0.93, 3H), 2.05 (m, 1 H), 2.35 (t, *J*₁ = 13.35, *J₂*= 0.99, 3H), 2.88 (m, 1 H), 3.19 (m, 1 H), 3.70 (m, 3H), 3.84 (m, 1H), 4.18 (m, 1 H), 4.58 (t, *J*₁ = 7.95, *J₂*= 4.75, 1H), 6.70 (s, 1 H), 7.05 (t, *J*₁ = 7.58, *J₂*= 0.69, 1 H), 7.22 (t, *J*₁ = 7.86, *J₂*= 1.15, 1 H), 7.43 (d, *J=* 0.67, 1 H), 7.56 (s, 1 H).

### Example 10.

General method for preparation of tetrahydro-thieno-pyrrolo[3,2-c]pyridines **1.8, 1.9,** among them **2.8, 2.9** and **3.8, 3.9.** 50% Solution of Bu₄NHSO₄ (0.08 ml), aryl acetylene (3.16 mmol) or its hetero analogue and 60% KOH solution (3.2 ml) were added to a solution of thieno-pyrrolo[3,2-*c*]pyridine unsubstituted at pyrrole nitrogen **1.1** (79 mmol) in DMSO (0.9 ml). The reaction was conducted in a pressuretight vial under argon atmosphere at 80°C for 18 h. Completeness of the reaction was controlled by LCMS. The reaction mixture was diluted with CH₂Cl₂, washed with water, drid over Na₂SO₄ and solvent was distilled off on rotary evaporator. Reaction products were isolated by column chromatography on SiO₂ (eluent hexane : EtOAc : Et₃N = 3:1:0.1). It gave the corresponding thieno-pyrrolo[3,2-c]pyridines **1.8, 1.9,** among them **2.8, 2.9** and **3.8, 3.9.**

2,5-Dimethyl-8-[(E)-2-phenylvinyl]-5,6,7,8-tetrahydro-4H-thieno[3',2':4,5]pyrrolo[3,2-c]pyridine **3.8(1).** Yield is 36 mg (15 %). LCMS (ESI): *m*/*z* 309 (M+H)⁺. ¹H NMR (CDCl₃, 400 MHz), δ, ppm (*J,* Hz): 7.42 (m, 2H), 7.37 (m, 2H), 7.33 (d, *J* = 14.4, 1 H), 7.26 (m, 1 H), 6.88 (s, 1 H), 6.44 (d, *J* = 14.4, 1 H), 3.58 (s, 2H), 2.93 (t, *J* = 5.2, 2H), 2.85 (t, *J* = 5.2, 2H), 2.55 (s, 3H). ¹³C NMR (CDCl₃, 75 MHz), δ, ppm: 135.39, 130.51, 128.43, 126.62, 126.15, 126.07, 125.31, 122.68, 122.08, 115.47, 113.68, 111.32, 51.76, 51.75, 45.11, 22.76.

2,5-Dimethyl-8-[(Z)-2-phenylvinyl]-5,6,7,8-tetrahydro-4H-thieno[3',2':4,5]pyrrolo[3,2-c]pyridine **3.9(1).** Yield is 157 mg (65 %). LCMS (ESI): m/z 309 (M+H)⁺. ¹H NMR (CDCl₃, 400 MHz), δ, ppm (*J*, Hz): 7.24 (m, 3H), 7.11 (m, 2H), 6.76 (s, 1H), 6.68 (d, *J =* 8.8, 1 H), 6.34 (d, *J =* 8.8, 1 H), 3.57 (s, 2H), 2.76 (m, 4H), 2.52 (s, 3H).

In analogous manner, on the bases of the corresponding starting materials the following compounds: **2.8(1), 2.9(1), 2.8(3), 2.9(3), 2.8(2), 2.9(2), 2.8(4), 2.9(4), 3.8(3), 3.9(3), 3.8(2), 3.9(2), 3.8(4), 3.9(4)** were prepared.

(E)-2,7-Dimethyl-4-(styryl)-5,6,7,8-tetrahydro-4H-thieno[2',3':4,5]pyrrolo[3,2-c]pyridine **2.8(1),** LCMS (ESI): *m*/*z* 309 (M+H)⁺. ¹H NMR (CDCl₃, 400 MHz), δ, ppm (*J,* Hz): 2.36 (m, 1 H), 2.46 (m, 1 H), 2.50 (t, *J*₁ = 13.35, *J*₂ = 0.99, 3H), 2.64 (t, *J*₁ = 2.88, *J*₂ = 0.90, 3H), 3.11 (m, 1 H), 3.19 (m, 1 H), 3.39 (m, 1 H), 3.56 (m, 1 H), 6.15 (t, *J*₁ = 14.11, *J*₂ = 0.70, 1 H), 6.59 (s, 1 H), 7.03 (m, 2H), 7.30 (m, 2H), 7.37 (d, *J =* 14.11, 1H), 7.51 (t, *J*₁ = 7.32, *J*₂ = 1.69, 1 H);
(Z)-2,7-dimethyl-4-(styryl)-5,6,7,8-tetrahydro-4H-thieno[2',3':4,5]pyrrolo[3,2-c]pyridine **2.9(1),** LCMS (ESI): *m*/*z* 309 (M+H)⁺. ¹H NMR (CDCl₃, 400 MHz), δ, ppm (*J,* Hz): 2.36 (m, 1H), 2.44 (m, 1 H), 2.50 (t, *J*₁ = 13.35, *J*₂ = 0.99, 3H), 2.64 (t, *J*₁ = 2.88, *J*₂ = 0.90, 3H), 3.08 (m, 1H), 3.18 (m, 1 H), 3.39 (m, 1 H), 3.56 (m, 1 H), 6.59 (d, *J* = 0.90, 1 H), 6.66 (t, *J*₁ = 9.54, *J*₂ = 0.33, 1H), 7.11 (d, *J* = 9.54, 1 H), 7.28 (m, 5H);
(E)-2-methyl-7-ethyl-4-(3-fluorostyryl)-5,6,7,8-tetrahydro-4H-thieno[2',3':4,5]pyrrolo[3,2-c]pyridine **2.8(3),** LCMS (ESI): *m*/*z* 341 (M+H)⁺. ¹H NMR (CDCl₃, 400 MHz), δ, ppm (*J,* Hz): 1.32 (t, *J*₁ = 7.21, *J*₂ = 2.88, 3H), 2.55 (m, 3H), 2.64 (t, *J*₁ = 2.88, *J*₂ = 0.90, 3H), 2.81 (m, 1 H), 3.03 (m, 1 H), 3.12 (m, 1 H), 3.55 (m, 1 H), 3.79 (m, 1 H), 6.35 (t, *J*₁ = 14.11, *J*₂ = 0.70, 1 H), 6.70 (s, 1 H), 6.91 (d, *J* = 2.40, 1H); 7.01 (t, *J*₁ = 7.80, *J*₂ = 0.63, 3H), 7.37 (d, *J* = 14.11, 1 H);
(*Z*)-2-methyl-7-ethyl-4-(3-fluorostyryl)-5,6,7,8-tetrahydro-4H-thieno[2',3':4,5]pyrrolo[3,2-c]pyridine **2.9(3),** LCMS (ESI): *m*/*z* 341 (M+H)⁺. ¹H NMR (CDCl₃, 400 MHz), δ, ppm (*J,* Hz): 1.32 (t, *J*₁ = 7.21, *J*₂ = 2.88, 3H), 2.55 (m, 3H), 2.64 (t, *J*₁ = 2.88, *J*₂ = 0.90, 3H), 2.81 (m, 1H), 3.03 (m, 1 H), 3.12 (m, 1 H), 3.55 (m, 1 H), 3.79 (m, 1 H), 5.68 (s, 1 H), 6.87 (d, *J* = 9.54, 1 H), 6.93 (m, 1 H), 7.01 (m, 3H), 7.11 (d, *J* = 9.54, 1 H);
(*E*)-7-methyl-4-(styryl)-2-chloro-5,6,7,8-tetrahydro-4H-thieno[2',3':4,5]pyrrolo[3,2-c]pyridine **2.8(2),** LCMS (ESI): *m*/*z* 329 (M+H)⁺. ¹H NMR (CDCl₃, 400 MHz), δ, ppm (*J,* Hz): 2.35 (m, 1 H), 2.42 (m, 1 H), 2.54 (t, *J*₁ = 13.35, *J*₂ = 0.99, 3H), 3.08 (m, 1 H), 3.20 (m, 1 H), 3.41 (m, 1 H), 3.56 (m, 1 H), 6.15 (t, *J*₁ = 14.11, *J*₂ = 0.70, 1 H), 6.79 (s, 1 H), 7.05 (d, *J =* 7.43, 2H), 7.28 (m, 2H), 7.35 (d, *J =* 14.11, 1 H), 7.51 (t, *J*₁ = 7.32, *J*₂ = 1.69, 1 H);
(Z)-7-methyl-4-(styryl)-2-chloro-5,6,7,8-tetrahydro-4H-thieno[2',3':4,5]pyrrolo[3,2-c]pyridine **2.9(2),** LCMS (ESI): *m*/*z* 329 (M+H)⁺. ¹H NMR (CDCl₃, 400 MHz), δ, ppm (*J,* Hz): 2.36 (m, 1 H), 2.45 (m, 1 H), 2.50 (t, *J*₁ = 13.35, *J*₂ = 0.99, 3H), 3.11 (m, 1 H), 3.19 (m, 1 H), 3.39 (m, 1 H), 3.60 (m, 1 H), 6.60 (s, 1 H), 6.68 (d, *J* = 9.54, 1 H), 7.11 (d, *J =* 9.54, 1 H), 7.26 (m, 5H);
*(E)*-7-methyl-4-(3-methylstyryl)-2-chloro-5,6,7,8-tetrahydro-4H-thieno[2',3':4,5]pyrrolo[3,2-c]pyridine **2.8(4),** LCMS (ESI): *m*/*z* 343 (M+H)⁺. ¹H NMR (CDCl₃, 400 MHz), δ, ppm (*J,* Hz): 2.28 (m, 1 H), 2.38 (t, *J*₁ = 2.88, *J*₂ = 0.35, 3H), 2.44 (m, 1 H), 2.50 (t, *J*₁ = 13.35, *J*₂ = 0.99, 3H), 3.11 (m, 1 H), 3.22 (m, 1 H), 3.40 (m, 1 H), 3.60 (m, 1 H), 6.16 (t, *J*₁ = 14.11, *J*₂ = 0.21, 1H), 6.60 (s, 1 H), 7.01 (m, 3H), 7.17 (d, *J* = 1.97, 1 H), 7.33 (d, *J* = 14.11, 1 H);
*(Z)*-7-methyl-4-(3-methylstyryl)-2-chloro-5,6,7,8-tetrahydro-4H-thieno[2',3':4,5]pyrrolo[3,2-c]pyridine **2.9(4),** LCMS (ESI): *m*/*z* 343 (M+H)⁺. ¹H NMR (CDCl₃, 400 MHz), δ, ppm (*J,* Hz): 2.28 (m, 1H), 2.38 (t, *J*₁ = 2.88, *J*₂ = 0.35, 3H), 2.42 (m, 1 H), 2.50 (t, *J*₁ = 13.35, *J*₂ = 0.99, 3H), 3.09 (m, 1 H), 3.20 (m, 1 H), 3.41 (m, 1 H), 3.60 (m, 1 H), 6.67 (t, *J*₁ = 9.54, *J*₂ = 0.33, 1 H), 6.79 (s, 1 H), 6.95 (m, 1H),7.00 (d, *J* = 7.30, 1 H), 7.02 (m, 1 H), 7.11 (d, *J* = 9.54, 1 H), 7.17 (d, *J* = 7.60, 1 H);
*(E)*-2-methyl-5-ethyl-8-(pyridin-3-ylvinyl)-4,5,6,7-tetrahydro-5H-thieno[3',2':4,5]pyrrolo[3,2-c]pyridine **3.8(3),** LCMS (ESI): *m*/*z* 341 (M+H)⁺. ¹H NMR (CDCl₃, 400 MHz), δ, ppm (*J,* Hz): 1.30 (t, *J*₁ = 7.21, *J*₂ = 2.88, 3H), 2.55 (t, *J*₁ = 12.34, *J*₂ = 0.99, 3H), 2.64 (d, *J* = 2.88, 3H), 2.80 (m, 1 H), 3.03 (m, 1 H), 3.12 (m, 1H), 3.80 (m, 1 H), 4.06 (m, 1 H), 6.04 (t, *J*₁ = 14.11, *J*₂ = 0.70, 1 H), 6.25 (s, 1 H), 7.19 (d, *J* = 14.11, 1H), 7.33 (t, *J*₁ = 7.92, *J*₂ = 0.80, 1 H), 8.07 (d, *J* = 1.90, 1 H), 8.74 (d, *J* = 1.33, 1 H), 8.99 (s, 1 H);
*(Z)*-2-methyl-5-ethyl-8-(pyridin-3-ylvinyl)-4,5,6,7-tetrahydro-5H-thieno[3',2':4,5]pyrrolo[3,2-c]pyridine **3.9(3),** LCMS (ESI): *m*/*z* 341 (M+H)⁺. ¹H NMR (CDCl₃, 400 MHz), δ, ppm (*J,* Hz): 1.30 (t, *J*₁ = 7.21, *J*₂ = 2.88, 3H), 2.52 (m, 1H), 2.55 (t, *J*₁ = 12.34, *J*₂ = 0.99, 2H), 2.80 (m, 1 H), 3.04 (m, 1 H), 3.12 (m, 1 H), 3.82 (m, 1 H), 4.09 (m, 1H), 6.50 (t, *J*₁ = 9.54, *J*₂ = 0.43, 1 H), 6.77 (s, 1 H), 6.98 (d, *J* = 9.54, 1H), 7.14 (t, *J*₁ = 5.80, *J*₂ = 0.80, 1 H), 8.27 (d, *J* = 7.92, 1 H), 8.56 (d, *J* = 5.08, 1H), 8.94 (s, 1 H);
*(E)*-5-methyl-8-styryl-2-chloro-4,5,6,7-tetrahydro-5H-thieno[3',2':4,5]pyrrolo[3,2-c]pyridine 3.8(2), LCMS (ESI): *m*/*z* 329 (M+H)⁺. ¹H NMR (CDCl₃, 400 MHz), δ, ppm (*J,* Hz): 2.35 (m, 1H), 2.43 (m, 1 H), 2.92 (t, *J*₁ = 13.35, *J*₂ = 0.99, 3H), 3.11 (m, 1 H), 3.20 (m, 1H), 3.60 (m, 1H), 3.70 (m, 1H), 6.14 (d, *J* = 14.11, 1H), 6.77 (s, 1 H), 7.03 (t, *J*₁ = 7.43, *J*₂ = 0.57, 2H), 7.14 (d, *J =* 14.11, 1H), 7.52 (t, *J*₁ = 7.32, *J*₂ = 1.69, 3H);
(Z)-5-methyl-8-(styryl)-2-chloro-4,5,6,7-tetrahydro-5H-thieno[3',2':4,5]pyrrolo[3,2-c]pyridine 3.9(2), LCMS (ESI): *m*/*z* 329 (M+H)⁺. ¹H NMR (CDCl₃, 400 MHz), δ, ppm (*J,* Hz): 2.36 (m, 1 H), 2.43 (m, 1 H), 2.92 (t, *J*₁ = 13.35, *J*₂ = 0.99, 3H), 3.11 (m, 1 H), 3.20 (m, 1 H), 3.60 (m, 1 H), 3.70 (m, 1 H), 6.55 (t, *J*₁ = 9.54, *J*₂ = 0.33, 1H), 6.77 (s, 1 H), 6.84 (d, *J =* 9.54, 1 H), 7.24 (m, 3H), 7.47 (m, 2H);
*(E)*-5-methyl-8-(3-methylstyryl)-2-chloro-4,5,6,7-tetrahydro-5H-thieno[3',2':4,5]pyrrolo[3,2-c]pyridine 3.8(4) LCMS (ESI): *m*/*z* 343 (M+H)⁺. ¹H NMR (CDCl₃, 400 MHz), δ, ppm (*J,* Hz): 2.32 (m, 1 H), 2.37 (t, *J*₁ =2.88, *J*₂ = 0.35, 3H), 2.43 (m, 1 H), 2.92 (t, *J*₁ = 13.35, *J*₂ = 0.99, 3H), 3.12 (m, 1 H), 3.22 (m, 1 H), 3.61 (m, 1 H), 3.70 (m, 1 H), 6.14 (t, *J*₁ = 14.11, *J*₂ = 0.70, 1 H), 6.77 (s, 1 H), 6.99 (m, 2H), 7.16 (d, *J* = 14.11, 1 H), 7.23 (s, 1 H), 7.37 (t, *J*₁ = 7.60, *J*₂ = 0.70, 1 H);
*(Z)*-5-methyl-8-(3-methylstyryl)-2-chloro-4,5,6,7-tetrahydro-5H-thieno[3',2':4,5]pyrrolo[3,2-c]pyridine **3.9(4)** LCMS (ESI): *m*/*z* 343 (M+H)⁺. ¹H NMR (CDCl₃, 400 MHz), δ, ppm (*J,* Hz): 2.30 (m, 1 H), 2.37 (t, *J*₁ =2.88, *J*₂ = 0.35, 3H), 2.46 (m, 1H), 2.91 (t, *J*₁ = 13.35, *J*₂ = 0.99, 3H), 3.11 (m, 1 H), 3.32 (m, 1 H), 3.60 (m, 1 H), 3.70 (m, 1 H), 6.54 (t, *J*₁ = 9.54, *J*₂ = 0.33, 1 H), 6.76 (s, 1 H), 6.81 (d, *J* = 9.54, 1 H), 6.95 (d, *J* = 7.60, 1H), 7.30 (d, *J* = 7.60, 1H), 7.22 (s, 1H), 7.37 (t, *J* = 7.60, 1 H).

### Example 11.

General method for preparation of tetrahydro-thieno-pyrrolo[3,2-*c*]pyridines **1.10,** among them **2.10** and **3.10.** Thieno-pyrrolo[3,2-c]pyridine unsubstituted at pyrrole nitrogen **1.1** (2 mmol), toluene (15 ml), CuSO₄ (50 mg, 0,2 mmol), and 1,10-phenantroline (0.4 mmol) were placed into a conical flask hermetically closed with a rubber cork, the flask was blown with argon, and a solution of 3-(bromoethynyl)pyridine (2,2 mmol) 7 in toluene (5,0 ml) was introduced using syringe under argon. The reaction mixture was srirred vigorously at 80-85°C until total disappearance of the starting thieno-pyrrolo[3,2-c]pyridine (LCMS control, 12-24 h). After the reaction was completed the reaction mixture was treated with 10% K₂CO₃ solution, filtered, extracted with benzene and dried over Na₂SO₄. Solvent was evaporated on rotary evaporator, the residue was subjected to chromatography on SiO₂ impregnated with triethylamine (eluent - hexane - CHCl₃ - triethylamine 5 : 4 : 1). It gave, for example, compound **2.10(1),** yield is 68%; 2,7-dimethyl-4-phenylethynyl-5,6,7,8-tetrahydro-4H-thieno[2',3':4,5]pyrrolo[3,2-c]pyridine **2.10(1),** LCMS (ESI): *m*/*z* 307 (M+H)⁺. ¹H NMR (CDCl₃, 400 MHz), δ, ppm (*J,* Hz): 2.43 (m, 1 H), 2.50 (t, *J*₁ = 13.35, *J*₂ = 0.99, 4H), 2.92 (m, 1 H), 3.01 (m, 1 H), 3.50 (m, 1 H), 3.64 (m, 1 H), 6.76 (s, 1 H), 7.24 (t, *J*₁ = 7.13, *J*₂ = 1.36, 1 H), 7.37 (t, *J*₁ = 7.73, *J*₂ = 0.63, 2H), 7.48 (t, *J*₁ = 1.76, *J*₂ = 0.63, 2H).

Using analogous procedure the folowing compounds **2.10(2), 2.10(3), 3.10(1), 3.10(2), 3.10(3)** were prepared.

7-Methyl-4-phenylethynyl-2-chloro-5,6,7,8-tetrahydro-4H-thieno[2',3':4,5]pyrrolo[3,2-c]pyridine **2.10(2),** LCMS (ESI): *m*/*z* 327 (M+H)⁺. ¹H NMR (CDCl₃, 400 MHz), δ, ppm (*J,* Hz): 2.43 (m, 1 H), 2.50 (t, *J*₁ = 13.35, *J*₂ = 0.99, 4H), 2.92 (m, 1 H), 3.01 (m, 1H), 3.50 (m, 1 H), 3.64 (m, 1 H), 6.85 (s, 1 H), 7.24 (t, *J*₁ = 7.13, *J*₂ = 1.36, 1 H), 7.37 (t, *J*₁ = 7.73, *J*₂ = 0.63, 2H), 7.48 (t, *J*₁ = 1.76, *J*₂ = 0.63, 2H);
6,7,8-trimethyl-4-(3-fluorophenyl)ethynyl-2-chloro-5,6,7,8-tetrahydro-4H-thieno[2',3':4,5]pyrrolo[3,2-c]pyridine **2.10(3),** LCMS (ESI): *m*/*z* 373 (M+H)⁺. ¹H NMR (CDCl₃, 400 MHz), δ, ppm (*J,* Hz): 1.08 (t, *J*₁ = 6.51, *J*₂ = 1.13, 3H), 1.59 (t, *J*₁ = 6.68, *J*₂ = 1.13, 3H), 2.05 (t, *J*₁ = 13.35, *J*₂ = 0.99, 3H), 2.92 (m, 1 H), 2.97 (m, 1 H), 3.16 (t, *J*₁ = 7.93, *J*₂ = 1.46, 1 H), 3.75 (t, *J*₁ = 6.68, *J*₂ = 0.99, 1 H), 6.85 (d, *J* = 1.59, 1 H), 6.87 (s, 1 H), 7.30 (m, 3H); 2,5-dimethyl-8-phenylethynyl-4,5,6,7-tetrahydro-5H-thieno[3',2':4,5]pyrrolo[3,2-c]pyridine **3.10(1),** LCMS (ESI): *m*/*z* 307 (M+H)⁺. ¹H NMR (CDCl3, 400 MHz), δ, ppm (*J,* Hz): 2.42 (m, 1H), 2.50 (m, 1 H), 2.64 (d, *J =* 2.88, 3H), 2.92 (t, *J*₁ = 13.35, *J*₂ = 0.99, 3H), 3.30 (m, 2H), 3.65 (m, 1 H), 3.77 (m, 1H), 6.14 (s, 1 H), 7.26 (t, *J*₁ = 7.13, *J*₂ = 1.36, 1 H), 7.39 (d, *J* = 7.73, 2H), 7.68 (d, *J* = 1.36, 3H);
5-methyl-8-phenylethynyl-2-chloro-4,5,6,7-tetrahydro-5H-thieno[3',2':4,5]pyrrolo[3,2-c]pyridine **3.10(2),** LCMS (ESI): *m*/*z* 327 (M+H)⁺. ¹H NMR (CDCl₃, 400 MHz), δ, ppm (*J,* Hz): 2.43 (m, 1 H), 2.50 (m, 1 H), 2.89 (m, 1 H), 2.92 (t, *J*₁ = 13.35, *J*₂ = 0.99, 3H), 3.03 (m, 2H), 3.68 (m, 1 H), 3.77 (m, 1 H), 6.69 (s, 1 H), 7.26 (t, *J*₁ = 7.13, *J*₂ = 1.36, 1 H), 7.38 (d, *J* = 7.73, 2H), 7.67 (d, *J* = 1.36, 3H);
4,5,6-trimethyl-8-[(3-fluorophenyl)ethynyl]-2-chloro-4,5,6,7-tetrahydro-5H-thieno[3',2':4,5]pyrrolo[3,2-c]pyridine **3.10(3)** LCMS (ESI): *m*/*z* 373 (M+H)⁺. ¹H NMR (CDCl₃, 400 MHz), δ, ppm (*J*, Hz): 1.08 (t, *J*₁ = 1.13, *J*₂ = 0.50, 3H), 1.49 (t, *J*₁ = 6.68, *J*₂ = 1.13, 3H), 2.06 (t, *J*₁ = 13.35, *J*₂ = 0.99, 3H), 2.92 (m, 1 H), 3.00 (m, 1 H), 3.16 (d, *J* = 7.93, 1 H); 4.21 (t, *J*₁ = 6.68, *J*₂ = 0.99, 1 H), 6.51 (s, 1 H), 6.85 (d, *J* = 1.59, 1 H); 7.32 (m, 1 H), 7.46 (t, *J*₁ = 10.70, *J*₂ = 1.59, 2H).

### Example 12.

General method for preparation of tetrahydro-thieno-pyrrolo[3,2-*c*]pyridine **1.11,** among them **2.11** and **3.11.** Tetrahydro-thieno-pyrrolo[3,2-*c*]pyridines **1.11,** among them **2.11** and **3.11** were synthesized according to the method described in example **3** by the action of the corresponding cinnamyl chlorides or bromides or their hetero analogues on thieno-pyrrolo[3,2-*c*]pyridines unsubstituted at pyrrole nitrogen **1.1.** 2-Methyl-7-(3-fluorobenzyl)-4-cinnamyl-5,6,7,8-tetrahydro-4H-thieno[2',3':4,5]pyrrolo[3,2-c]pyridine **2.11(1)** was prepared with yield 92%. LCMS (ESI): *m*/*z* 417 (M+H)⁺. ¹H NMR (CDCl₃, 400 MHz), δ, ppm (*J,* Hz): 2.64 (t, *J*₁ = 2.88, *J*₂ = 0.90, 3H), 2.75 (m, 1 H), 2.85 (m, 1 H), 2.97 (m, 1 H), 3.05 (m, 1 H), 3.58 (m, 1 H), 3.65 (m, 1 H), 3.74 (m, 1 H), 3.97 (m, 1 H), 4.49 (s, 2H), 5.97 (t, *J*₁ = 15.91, *J*₂ = 0.70, 1 H), 6.26 (d, *J* = 4.00, 1 H); 6.86 (m, 2H), 6.95 (s, 1 H), 7.10 (d, *J* = 0.70, 2H), 7.28 (d, *J =* 1.88, 2H); 7.39 (t, *J*₁ = 7.43, *J*₂ = 0.57, 3H).

Compounds **2.11(2), 2.11 (3), 2.11(4), 2.11(5), 3.11(1), 3.11(2), 3.11(3), 3.11(4), 3.11(5)** were prepared in analogous manner.

(E)-3,7-Dimethyl-4-[3-(*p*-tolyl)allyl]-5,6,7,8-tetrahydro-4H-thieno[2',3':4,5]pyrrolo[3,2-c]pyridine **2.11(2):** LCMS (ESI): *m*/*z* 337 (M+H)⁺. ¹H NMR (CDCl₃, 400 MHz), δ, ppm (*J,* Hz): 2.32 (d, *J* = 2.88, 3H), 2.43 (m, 1 H), 2.50 (t, *J*₁ = 13.35, *J*₂ = 0.99, 4H), 2.90 (m, 1 H), 2.98 (m, 1 H), 3.46 (m, 1 H), 3.67 (m, 1 H), 4.42 (s, 2H), 5.97 (t, *J*₁ = 15.91, *J*₂ = 1.27, 1 H), 6.26 (d, *J* = 4.00, 1H), 6.75 (s, 1 H), 7.07 (t, *J*₁ = 8.26, *J*₂ = 0.77, 2H), 7.36 (t, *J*₁ = 2.00, *J*₂ = 0.77, 2H);
(E)-2,7-dimethyl-4-[3-(3-chlorophenyl)allyl]-5,6,7,8-tetrahydro-4H-thieno[2',3':4,5]pyrrolo[3,2-c]pyridine **2.11(3):** LCMS (ESI): *m*/*z* 357 (M+H)⁺. ¹H NMR (CDCl₃, 400 MHz), δ, ppm (*J,* Hz): 2.43 (m, 1 H), 2.50 (t, *J*₁ = 13.35, *J*₂ = 0.99, 4H), 2.92 (m, 1 H), 2.97 (m, 1 H), 3.46 (m, 1H), 3.64 (m, 1 H), 4.49 (s, 2H), 6.25 (d, *J* = 4.00, 1 H), 6.59 (t, *J*₁ = 15.91, *J*₂ = 0.70, 1 H), 6.93 (t, *J*₁ = 8.00, *J*₂ = 0.63, 1 H), 6.99 (s, 1 H), 7.39 (t, *J*₁ = 2.20, *J*₂ = 1.20, 1H), 7.69 (d, *J* = 2.36, 1H), 7.98 (s, 1 H);
(E)-7-methyl-4-[3-(*m*-tolyl)allyl]-3-chloro-5,6,7,8-tetrahydro-4H-thieno[2',3':4,5]pyrrolo[3,2-c]pyridine **2.11(4):** LCMS (ESI): *m*/*z* 357 (M+H)⁺. ¹H NMR (CDCl₃, 400 MHz), δ, ppm (*J,* Hz): 2.31 (d, *J* = 2.88, 3H), 2.43 (m, 1 H), 2.50 (t, *J*₁ = 13.35, *J*₂ = 0.99, 4H), 2.92 (m, 1 H), 2.99 (m, 1 H), 3.47 (m, 1 H), 3.67 (m, 1 H), 4.45 (s, 2H), 6.24 (d, *J* = 4.00, 1 H), 6.54 (t, *J*₁ = 15.91, *J*₂ = 0.33, 1 H), 6.63 (s, 1 H), 6.97 (d, *J* = 1.92, 1H), 7.11 (d, *J* = 1.97, 2H), 7.22 (s, 1 H);
(E)-7-methyl-4-[3-(*p*-tolyl)allyl]-2-chloro-5,6,7,8-tetrahydro-4H-thieno[2',3':4,5]pyrrolo[3,2-c]pyridine **2.11(5):** LCMS (ESI): *m*/*z* 357 (M+H)⁺. ¹H NMR (CDCl₃, 400 MHz), δ, ppm (*J*, Hz): 2.33 (d, *J* = 2.88, 3H), 2.43 (m, 1 H), 2.50 (t, *J*₁ = 13.35, *J*₂ = 0.99, 4H), 2.92 (m, 1 H), 2.99 (m, 1 H), 3.46 (m, 1 H), 3.67 (m, 1 H), 4.49 (s, 2H), 5.98 (t, *J*₁ = 15.91, *J*₂ = 0.35, 1 H), 6.25 (d, *J* = 4.00, 1 H), 7.07 (t, *J*₁ = 2.23, *J*₂ = 0.77, 2H), 7.14 (s, 1 H), 7.38 (d, *J =* 2.00, 2H);
2-methyl-5-(3-fluorobenzyl)-8-cinnamyl-4,5,6,7-tetrahydro-5H-thieno[3',2':4,5]pyrrolo[3,2-c]pyridine **3.11(1):** LCMS (ESI): *m*/*z* 417 (M+H)⁺. ¹H NMR (CDCl₃, 400 MHz), δ, ppm (*J,* Hz): 2.64 (d, *J* = 2.88, 3H), 2.76(m, 1 H), 2.85 (m, 1 H), 2.96 (m, 1 H), 3.06 (m, 1 H), 3.60 (m, 2H), 4.00 (m, 1 H), 4.26 (m, 1 H), 4.56 (s, 2H), 6.12 (d, *J* = 4.00, 1H), 6.23 (s, 1 H), 6.30 (t, *J*₁ = 15.91, *J*₂ = 0.70, 1H), 6.88 (t, *J*₁ = 8.12, *J*₂ = 0.50, 1 H), 7.10 (d, *J* = 7.49, 1 H), 7.29 (t, *J*₁ = 7.43, *J*₂ = 1.88, 2H), 7.37 (m, 3H);
2,5-dimethyl-8-[3-(*p*-tolyl)allyl]-4,5,6,7-tetrahydro-5H-thieno[3',2':4,5]pyrrolo[3,2-c]pyridine **3.11(2)** LCMS (ESI): *m*/*z* 337 (M+H)⁺. ¹H NMR (CDCl₃, 400 MHz), δ, ppm (*J,* Hz): 2.33 (d, *J* = 2.88, 3H), 2.43 (m, 1 H), 2.50 (m, 1H), 2.64 (d, *J* = 2.88, 3H), 2.89(m, 1 H), 2.91 (d, *J* = 13.35, 3H), 2.99 (m, 1 H), 3.68 (m, 1 H), 3.77 (m, 1 H), 4.56 (s, 2H), 6.12 (d, *J =* 1.20, 2H), 6.30 (t, *J*₁ = 15.91, *J*₂ = 0.70, 1 H), 7.07 (t, *J*₁ = 8.26, *J*₂ = 0.77, 2H), 7.36 (t, *J*₁ = 8.26, *J*₂ = 0.32, 2H);
(2,5-dimethyl-8-[3-(3-chlorophenyl)allyl]-4,5,6,7-tetrahydro-5H-thieno[3',2':4,5]pyrrolo[3,2-c]pyridine 3.11(3): LCMS (ESI): m/z 357 (M+H)⁺. ¹H NMR (CDCl₃, 400 MHz), δ, ppm (*J,* Hz): 2.42 (m, 1 H), 2.49 (m, 1 H), 2.65 (d, *J* = 2.88, 3H), 2.87 (m, 1 H), 2.93 (t, *J*₁ = 13.35, *J*₂ = 0.99, 3H), 2.97 (m, 1 H), 3.64 (m, 1H), 3.73 (m, 1 H), 4.56 (t, *J*₁ = 4.00, *J*₂ = 1.27, 2H), 6.13 (d, *J* = 1.20, 1 H), 6.15 (d, *J* = 4.11, 1 H), 6.57 (t, *J*₁ = 15.91, *J*₂ = 0.70, 1 H), 6.95 (t, *J*₁ = 8.00, *J*₂ = 0.63, 1 H), 7.38 (t, *J*₁ = 2.20, *J*₂ = 1.20, 1 H), 7.69 (d, *J* = 2.36, 1 H), 7.98 (s, 1 H);
(E)-5-methyl-8-[(3-*m*-tolyl)allyl]-3-chloro-4,5,6,7-tetrahydro-5H-thieno[3',2':4,5]pyrrolo[3,2-c]pyridine **3.11(4):** LCMS (ESI): *m*/*z* 357 (M+H)⁺. ¹H NMR (CDCl₃, 400 MHz), δ, ppm (*J,* Hz): 2.31 (d, *J* = 2.88, 3H), 2.42 (m, 1 H), 2.50 (m, 1 H), 2.95 (t, *J*₁ = 13.35, *J*₂ = 0.99, 5H), 3.63 (m, 1 H), 3.74 (m, 1H), 4.56 (s, 2H), 6.10 (d, *J* = 4.00, 1 H), 6.55 (t, *J*₁ = 15.91, *J*₂ = 0.70, 1 H), 6.63 (s, 1 H), 6.97 (d, *J* = 1.92, 1H), 7.10 (m, 2H), 7.24 (s, 1 H);
(E)-5-methyl-8-[3-(6-methylpyridin-3-yl)allyl]-2-chloro-4,5,6,7-tetrahydro-5H-thieno[3',2':4,5]pyrrolo[3,2-c]pyridine **3.11(5):** LCMS (ESI): *m*/*z* 358 (M+H)⁺. ¹H NMR (CDCl₃, 400 MHz), δ, ppm (*J,* Hz): 2.42 (m, 1 H), 2.47 (m, 1 H), 2.51 (d, *J* = 2.88, 3H), 2.92 (t, *J*₁ = 13.35, *J*₂ = 0.99, 4H), 3.00 (m, 1 H), 3.65 (m, 1 H), 3.74 (m, 1H), 4.56 (s, 2H), 6.18 (s, 2H), 6.67 (s, 1 H), 6.77 (t, *J*₁ = 15.91, *J*₂ = 0.70, 1 H), 7.12 (t, *J*₁ = 8.10, *J*₂ = 0.50, 1 H), 7.86 (d, *J* = 2.00, 1H), 8.78 (s, 1 H).

### Example 13.

General method for preparation of tetrahydro-thieno-pyrrolo[3,2-*c*]pyridines **1.12,** among them **2.12** and **3.12.** NaH (4.55 mmol) was added at stirring to a solution of compound **1.1** (3.03 mmol) in anhydrous DMF (8 ml); in 30 min the corresponding aryl or hetaryl propyl halogenide (3.5 mmol) was added and the reaction mixture was stirred at 20°C (LCMS control). When needed, the addition of NaH and alkylating agent was repeated until total disappearance of compound **1.1.** After the reaction was completed the mixture was poured into water, extracted with ethyl acetate, the extract was washed with water, dried over Na₂SO₄. The solvent was distilled, the reaction product was isolated by column chromatography. Yield is 35-48%. It gave 2,7-dimethyl-4-(3-phenylpropyl)-5,6,7,8-tetrahydro-4H-thieno[2',3':4,5]pyrrolo[3,2-c]pyridine **2.12(1):** LCMS (ESI): *m*/*z* 325 (M+H)⁺. ¹H NMR (CDCl₃, 400 MHz), δ, ppm (*J,* Hz): 2.08 (d, *J* = 6.50, 2H), 2.42 (m, 1 H), 2.50 (t, *J*₁ = 13.35, *J*₂ = 0.99, 4H), 2.64 (t, *J*₁ = 13.50, *J*₂ = 7.07, 5H), 2.89 (m, 1 H), 2.96 (m, 1 H), 3.46 (m, 1 H), 3.67 (m, 1 H), 3.72 (d, *J =* 14.00, 2H), 6.85 (s, 1 H), 7.14 (m, 5H);
Compounds **2.12(3), 2.12(4), 2.12(2), 2.12(5), 2.12(6), 3.12(1), 3.12(3), 3.12(4), 3.12(2), 3.12(5), 3.12(6)** were prepared in analogous manner.

3,7-Dimethyl-4-[3-(6-methylpyridin-3-yl)propyl)]-5,6,7,8-tetrahydro-4H-thieno[2',3':4,5]pyrrolo[3,2-c]pyridine **2.12(3)** LCMS (ESI): *m*/*z* 340 (M+H)⁺. ¹H NMR (CDCl₃, 400 MHz), δ, ppm (*J,* Hz): 2.06 (d, *J* = 6.50, 2H), 2.32 (d, *J* = 2.88, 3H), 2.44 (m, 1 H), 2.50 (m, 7H), 2.66 (t, *J*₁ = 13.47, *J*₂ = 7.07, 2H), 2.89 (m, 1 H), 2.97 (m, 1 H), 3.47 (m, 1 H), 3.65 (m, 1 H), 3.66 (d, *J* = 14.00, 2H), 6.70 (s, 1 H), 7.08 (t, *J*₁ = 8.10, *J*₂ = 0.77, 1 H), 7.18 (d, *J* = 2.03, 1H), 8.22 (s, 1 H);
2,6,7,8-tetramethyl-4-[3-(3-chlorophenyl)propyl]-5,6,7,8-tetrahydro-4H-thieno[2',3':4,5]pyrrolo[3,2-c]pyridine **2.12(4):** LCMS (ESI): *m*/*z* 387 (M+H)⁺. ¹H NMR (CDCl₃, 400 MHz), δ, ppm (*J,* Hz): 1.09 (s, 1 H), 1.59 (t, *J*₁ = 6.68, *J*₂ = 1.13, 3H), 2.07 (m, 5H), 2.62 (m, 5H), 2.90 (m, 1 H), 2.94 (m, 1 H), 3.16 (t, *J*₁ = 7.93, *J*₂ = 1.51, 1 H), 3.74 (m, 1H), 3.82 (d, *J =* 14.00, 2H), 6.88 (s, 1H), 7.04 (t, *J*₁ = 7.86, *J*₂ = 0.43, 2H), 7.11 (d, *J* = 1.96, 1 H), 7.24 (d, *J* = 2.00, 1 H);
7-methyl-4-(3-phenylpropyl)-2-chloro-5,6,7,8-tetrahydro-4H-thieno[2',3':4,5]pyrrolo[3,2-c]pyridine **2.12(2):** LCMS (ESI): *m*/*z* 345 (M+H)⁺. ¹H NMR (CDCl₃, 400 MHz), δ, ppm (*J,* Hz): 2.08 (d, *J* = 6.50, 2H), 2.43 (m, 1 H), 2.50 (m, 4H), 2.64 (t, *J*₁ = 13.50, *J*₂ = 7.07, 2H), 2.90 (m, 1 H), 2.97 (m, 1 H), 3.46 (m, 1 H), 3.65 (m, 1 H), 3.72 (d, *J =* 14.00, 2H), 7.05 (s, 1H), 7.14 (m, 5H);
7-methyl-4-[3-(6-methylpyridin-3-yl)propyl]-3-chloro-5,6,7,8-tetrahydro-4H-thieno[2',3':4,5]pyrrolo[3,2-c]pyridine **2.12(5)** LCMS (ESI): *m*/*z* 360 (M+H)⁺. ¹H NMR (CDCl₃, 400 MHz), δ, ppm (*J,* Hz): 2.04 (d, *J* = 6.50, 2H), 2.45 (m, 4H), 2.50 (m, 4H), 2.66 (t, *J*₁ = 13.47, *J*₂ = 7.07, 2H), 2.89 (m, 1 H), 2.96 (m, 1H), 3.46 (m, 1 H), 3.65 (m, 1 H), 3.67 (d, *J* = 14.00, 2H), 6.58 (s, 1 H), 7.07 (t, *J*₁ = 8.10, *J*₂ = 0.77, 1 H), 7.18 (d, *J =* 1.90, 1 H), 8.22 (s, 1 H);
7-methyl-4-[3-(3-fluorophenyl)propyl]-2-chloro-5,6,7,8-tetrahydro-4H-thieno[2',3':4,5]pyrrolo[3,2-c]pyridine **2.12(6):** LCMS (ESI): *m*/*z* 363 (M+H)⁺. ¹H NMR (CDCl₃, 400 MHz), δ, ppm (*J,* Hz): 2.06 (d, *J* = 6.50, 2H), 2.42 (m, 1 H), 2.50 (m, 4H), 2.69 (t, *J*₁ = 13.50, *J*₂ = 7.07, 2H), 2.90 (m, 1 H), 2.97 (m, 1 H), 3.46 (m, 1H), 3.67 (m, 1H), 3.67 (d, *J* = 14.00, 2H), 6.75 (d, *J* = 1.10, 1 H), 6.90 (d, *J* = 1.90, 1 H), 7.01 (t, *J*₁ = 8.12, *J*₂ = 0.77, 2H), 7.08 (s, 1 H);
(3,5-dimethyl-8-(3-phenylpropyl)-4,5,6,7-tetrahydro-5H-thieno[3',2':4,5]pyrrolo[3,2-c]pyridine **3.12(1):** LCMS (ESI): *m*/*z* 325 (M+H)⁺. ¹H NMR (CDCl₃, 400 MHz), δ, ppm (*J,* Hz): 1.91 (d, *J* = 6.50, 2H), 2.44 (m, 1 H), 2.49 (m, 1 H), 2.53 (d, *J* = 2.88, 3H), 2.64 (t, *J*₁ = 13.50, *J*₂ = 7.07, 2H), 2.86 (m, 1 H), 2.92 (t, *J*₁ = 13.35, *J*₂ = 0.99, 4H), 3.68 (m, 1 H), 3.80 (d, *J =* 14.00, 3H), 6.70 (s, 1 H), 7.17 (m, 5H);
(2,5-dimethyl-8-[3-(6-methylpyridin-3-yl)propyl]-4,5,6,7-tetrahydro-5H-thieno[3',2':4,5]pyrrolo[3,2-c]pyridine **3.12(3):** LCMS (ESI): *m*/*z* 340 (M+H)⁺. ¹H NMR (CDCl₃, 400 MHz), δ, ppm (*J,* Hz): 1.87 (d, *J* = 6.50, 2H), 2.42 (m, 1 H), 2.48 (m, 4H), 2.65 (d, *J* = 2.88, 5H), 2.87 (m, 1 H), 2.91 (t, *J*₁ = 13.35, *J*₂ = 0.99, 3H), 2.97 (m, 1H), 3.68 (m, 1H), 3.76 (m, 1 H), 3.79 (s, 2H), 6.09 (s, 1 H), 7.08 (t, *J*₁ = 8.10, *J*₂ = 0.70, 1 H), 7.18 (t, *J*₁ = 2.03, *J*₂ = 1.90, 1 H), 8.22 (s, 1 H);
(3,4,5,6-tetramethyl-8-[3-(3-chlorophenyl)propyl]-4,5,6,7-tetrahydro-5H-thieno[3',2':4,5]pyrrolo[3,2-c]pyridine **3.12(4):** LCMS (ESI): *m*/*z* 387 (M+H)⁺. ¹H NMR (CDCl₃, 400 MHz), δ, ppm (*J,* Hz): 1.09 (s, 1 H), 1.49 (t, *J*₁ = 6.68, *J*₂ = 1.13, 3H), 1.91 (d, *J* = 6.50, 2H), 2.06 (t, *J*₁ = 13.35, *J*₂ = 0.99, 3H), 2.44 (d, *J* = 2.88, 3H), 2.60 (t, *J*₁ = 13.50, *J*₂ = 7.07, 2H), 2.92 (m, 2H), 3.16 (d, *J* = 7.93, 1 H), 3.96 (d, *J* = 14.00, 2H), 4.23 (m, 1 H), 6.68 (s, 1 H), 7.04 (t, *J*₁ = 7.86, *J*₂ = 0.77, 2H), 7.12 (d, *J* = 1.96, 1 H), 7.24 (d*, J =* 2.00, 1 H);
(5-methyl-8-(3-phenylpropyl)-2-chloro-4,5,6,7-tetrahydro-5H-thieno[3',2':4,5]pyrrolo[3,2-c]pyridine **3.12(2):** LCMS (ESI): *m*/*z* 345 (M+H)⁺. ¹H NMR (CDCl₃, 400 MHz), δ, ppm (*J,* Hz): 1.91 (d, *J* = 6.50, 2H), 2.42 (m, 1 H), 2.50 (m, 1 H), 2.64 (t, *J*₁ = 13.50, *J*₂ = 7.07, 2H), 2.91 (t, *J*₁ = 13.35, *J*₂ = 0.99, 4H), 2.98 (m, 1 H), 3.67 (m, 1 H), 3 76 (m, 1 H), 3.79 (d, *J* = 14.00, 2H), 6.62 (s, 1 H), 7.20 (m, 5H);
5-methyl-8-[(*p*-tolyl)propyl]-3-chloro-4,5,6,7-tetrahydro-5H-thieno[3',2':4,5]pyrrolo[3,2-c]pyridine **3.12(5):** LCMS (ESI): *m*/*z* 359 (M+H)⁺. ¹H NMR (CDCl₃, 400 MHz), δ, ppm (*J,* Hz): 1.91 (d, *J* = 6.50, 2H), 2.25 (t, *J*₁ = 2.88, *J*₂ = 0.34, 3H), 2.41 (m, 1H), 2.50 (m, 1 H), 2.64 (t, *J*₁ = 13.50, *J*₂ = 7.07, 2H), 2.91 (t, *J*₁ = 13.35, *J*₂ = 0.99, 4H), 2.99 (m, 1 H), 3.66 (m, 1 H), 3.71 (m, 1 H), 3.79 (d, *J* = 14.00, 2H), 6.58 (s, 1 H), 6.94 (m, H);
5-methyl-8-[(3-fluorophenyl)propyl]-2-chloro-4,5,6,7-tetrahydro-5H-thieno[3',2':4,5]pyrrolo[3,2-c]pyridine **3.12(6):** LCMS (ESI): *m*/*z* 362 (M+H)⁺. ¹H NMR (CDCl₃, 400 MHz), δ, ppm (*J,* Hz): 1.91 (d, *J* = 6.50, 2H), 2.43 (m, 1 H), 2.50 (m, 1 H), 2.68 (t, *J*₁ = 13.50, *J*₂ = 7.07, 2H), 2.90 (m, 1 H), 2.91 (t, *J*₁ = 13.35, *J*₂ = 0.99, 3H), 2.94 (m, 1 H), 3.67 (m, 1 H), 3.73 (m, 1 H), 3.82 (m, 1 H), 6.62 (s, 1 H), 6.72 (d, *J =* 1.10, 1 H), 6.92 (d, *J =* 1.90, 1 H), 7.03 (m, 2H).

### Example 14.

Profiles of pharmacological activity of thieno-pyrrolo[3,2-c]pyridines of the general formula **1.** Biological activity test of compounds of the general formula **1** was carried out in the setting of competitive radioligand binding [I. Okun, S. Tkachenko, A. Khvat, et al., Current Alzheimer Research, 7(2), 97-112 (2010)], using a wide range of therapeutic targets including G-protein coupled receptors (GPCR), ion channels and neuromediator transporters. The tested activity was determined by quantitative measurement of radio-labelled ligand displacement by tested compounds of the general formula **1** at their concentration of 1 µM. Test results for some thieno-pyrrolo[3,2-c]pyridines of the general formula **1** are shown in the Table.

**Table.**

| Profiles of pharmacological activity of thieno-pyrrolo[3,2-c]pyridines of the general formula **1** at their concentration of 1 µM in the setting of competitive radioligand binding. | | | | | | |
|---|---|---|---|---|---|---|
| Therapeutic targets | | **2.5(1)·H Cl** | **3.5(1)·HCl** | **2.6(1)·HCl** | **3.6(1)·H Cl** | **3.7(1)·HC l** |
| Adrenergic | α1A | 42 | 77 | 83 | 97 | 79 |
| Adrenergic | α1B | 10 | 57 | 90 | 99 | 89 |
| Adrenergic | α1D | 21 | 44 | 64 | 92 | 86 |
| Adrenergic | α2A | 34 | 64 | 80 | 100 | 91 |
| Dopaminergic | D1 | 5 | 5 | 15 | 70 | 47 |
| Dopaminergic | D2L | 8 | 14 | 76 | 87 | 35 |
| Dopaminergic | D2S | -6 | 23 | 73 | 86 | 29 |
| Dopaminergic | D3 | 16 | 19 | 62 | 73 | 23 |
| Dopaminergic | D4.2 | -3 | -5 | 14 | 71 | 33 |
| Histaminergic | H1 | 96 | 96 | 97 | 98 | 89 |
| Histaminergic | H2 | 44 | 91 | 56 | 96 | 57 |
| Histaminergic | H3 | -4 | 18 | -15 | -17 | -3 |
| Serotoninergic | 5-HT1A | 3 | 42 | 16 | 48 | 18 |
| Serotoninergic | 5-HT1B | 6 | 10 | 18 | 47 | 30 |
| Serotoninergic | 5-HT2A | 93 | 100 | 99 | 101 | 97 |
| Serotoninergic | 5-HT2B | 91 | 90 | 79 | 92 | 63 |
| Serotoninergic | 5-HT2C | 84 | 97 | 89 | 99 | 94 |
| Serotoninergic | 5-HT6 | 55 | 85 | 98 | 103 | 99 |
| Serotoninergic | 5-HT7 | 92 | 95 | 98 | 99 | 96 |

| Therapeutic targets | | **2.4(3)·H Cl poor** | **2.5(4)·HCl satisfactory** | **3.11(2)· HCl satisfactory** | **3.12(1)· HCl good** | **3.12(5)· HCl good** |
|---|---|---|---|---|---|---|
| Adrenergic | α1A | 17 | 51 | 55 | 98 | 79 |
| Adrenergic | α1B | 10 | 38 | 60 | 99 | 89 |
| Adrenergic | α1D | 28 | 29 | 42 | 92 | 86 |
| Adrenergic | α2A | 14 | 42 | 53 | 101 | 91 |
| Dopaminergic | D1 | 5 | 3 | 10 | 57 | 47 |
| Dopaminergic | D2L | 4 | 11 | 50 | 82 | 35 |
| Dopaminergic | D2S | 3 | 17 | 61 | 89 | 29 |
| Dopaminergic | D3 | 11 | 15 | 49 | 75 | 23 |
| Dopaminergic | D4.2 | 3 | -1 | 13 | 73 | 33 |
| Histaminergic | H1 | 38 | 70 | 81 | 98 | 89 |
| Histaminergic | H2 | 29 | 83 | 50 | 99 | 57 |
| Histaminergic | H3 | 1 | 9 | 7 | 11 | -3 |
| Serotoninergic | 5-HT1A | 3 | 35 | 21 | 43 | 18 |
| Serotoninergic | 5-HT1B | 5 | 28 | 39 | 54 | 30 |
| Serotoninergic | 5-HT2A | 41 | 91 | 93 | 99 | 97 |
| Serotoninergic | 5-HT2B | 39 | 78 | 89 | 91 | 63 |
| Serotoninergic | 5-HT2C | 33 | 91 | 93 | 97 | 94 |
| Serotoninergic | 5-HT6 | 26 | 85 | 95 | 101 | 99 |
| Serotoninergic | 5-HT7 | 43 | 87 | 85 | 98 | 96 |

| Therapeutic targets | | **3.12(6)· HCl good** | **3.13(1)· HCl satisfactory** | **2.10(3)· HCl poor** | **2.12(5)· HCl good** | **3.6(4)·HC l good** |
|---|---|---|---|---|---|---|
| Adrenergic | α1A | 84 | 61 | 21 | 98 | 89 |
| Adrenergic | α1B | 96 | 69 | 5 | 97 | 91 |
| Adrenergic | α1D | 83 | 43 | 11 | 93 | 89 |
| Adrenergic | α2A | 91 | 65 | 17 | 101 | 93 |
| Dopaminergic | D1 | 63 | 40 | 4 | 75 | 56 |
| Dopaminergic | D2L | 81 | 37 | 20 | 87 | 45 |
| Dopaminergic | D2S | 77 | 25 | 31 | 89 | 72 |
| Dopaminergic | D3 | 69 | 21 | 23 | 71 | 63 |
| Dopaminergic | D4.2 | 68 | 31 | 4 | 69 | 54 |
| Histaminergic | H1 | 97 | 88 | 65 | 99 | 95 |
| Histaminergic | H2 | 91 | 70 | 33 | 96 | 63 |
| Histaminergic | H3 | -1 | 12 | -10 | 12 | 8 |
| Serotoninergic | 5-HT1A | 50 | 19 | 6 | 57 | 29 |
| Serotoninergic | 5-HT1B | 43 | 20 | 9 | 62 | 41 |
| Serotoninergic | 5-HT2A | 97 | 95 | 43 | 99 | 97 |
| Serotoninergic | 5-HT2B | 91 | 90 | 39 | 95 | 78 |
| Serotoninergic | 5-HT2C | 98 | 81 | 31 | 99 | 96 |
| Serotoninergic | 5-HT6 | 84 | 67 | 35 | 100 | 99 |
| Serotoninergic | 5-HT7 | 95 | 79 | 33 | 99 | 99 |

### Example 15.

Nootropic action (enhancement of memory disturbed by Scopolamine) of compounds of the general formula 1 in test "Passive Avoidance of mice in Shuttle Chamber". Shuttle chamber (Ugo Basile, Italy) consisted of two sections was used. Walls of one section were opaque, while the other section had a transparent cover. Sections were connected through a hole which could be overlapped by vertical door. The floor was made of transverse metal bars on which DC current impulses could be fed. Experiments were carried out in aged male mice of BALB/c line weighing 20-24 g.

On the first day of experiment 30 minutes before training mice were injected intraperitoneally with physiological solution, (0,3 mg/kg) or Scopolamine in combination with compound **3.6(1)·HCl,** or Scopolamine in combination with compound **2.5(1)·HCl.** Each group consisted of 8 animals. Animals were placed in light section, and latent period of the first entry into the dark chamber was registered. Then the vertical door was closed and the animal was punished by 0.6 mA DC current for 3 seconds. After that the animal was taken back to its home cage. In 22-24 hours the same animal was placed again in light section of shuttle chamber and latent period of its first entry into dark section, total time of its stay in light section and number of entries into dark section was registered. Each monitoring lasted for 5 minutes.

Experiments were carried out during light period of animal's diurnal in isolated laboratory room, level of white noise made up 70 dB above normal threshold of audibility.

Scopolamine causes training disturbance (memory disturbance), which was expressed in prolongation of latent period of its first entry into dark section, duration of its stay in light section and decreasing the number of entries into dark section.

The ability of compounds **2.5(1)·HCl** and **3.6(1)·HCl** to improve training disturbed by Scopolamine is regarded as evidence of their nootropic action.

### Example 16.

Antipsychotic activity of compounds of general formula 1 in "Prepulse inhibition of startle response in mice" test. Mice of SHK line weighing about 24-30g were used in the test. Experiments were carried out during light period of animal's diurnal. Apomorphine hydrochloride and Haloperidol were received from Sigma Chemicals Company, (USA). Apomorphine hydrochloride was dissolved in 0.1% solution of ascorbic acid prepared with sterilized water; it was administered subcutaneously 15 minutes before the test. Haloperidol was dissolved in sterilized water using emulsifier Twin 80, it was administered intraperitoneally 60 minutes before the test. Compounds **2.5(1)·HCl** and **3.6(1)·HCl** were dissolved in sterilized water and administered subcutaneously 60 minutes before the test. Injection volume was 10 ml/kg. Solution of ascorbic acid, prepared with sterilized water and Twin 80, were injected to control group of animals.

The test instrument consisted of a chamber made of transparent plexiglass (manufacturer - Columbia Instruments Company, USA) and placed on a platform; the latter was lodged inside the sound insulating chamber. High frequency sound colomn transmitting acoustic stimuluses was located 2 cm away from the platform. Startle of animal resulted in vibrations of platform, which were detected by analog converter and registered by computer. Level of background noise made up 65 dB. Each animal received 4 stimuli of single testing (pulse) stimulus of 50 ms duration and 105 dB or prepulsory stimulus (pre-pulse) of 20 ms duration and 85 dB, after which in 30 ms pulse stimulus of 50 ms duration and 105 dB followed. Time interval between repeated pulse or prepulse in combination with pulse stimuli made up 10 s. Inhibition of the startle in reply to prepulse-plus-pulse stimulus was calculated in percentage towards amplitude of startle in response to isolated pulse stimulus. Administration of Apomorphine, which is used in experiments on animals for modelling psychoto-like conditions, caused reduction of prepulse inhibition of startle, which reflected the lowering of CNS ability to filter sensory stimulus.

Results of the experiment show that Haloperidol (1 mg/kg) and tested compounds **2.5(1)·HCl** and **3.6(1)·HCl** (1mg/kg) prevented disturbance of prepulse inhibition of startle caused by Apomorphine.

### Example 17.

Antidepressant action of antagonists of the general formula **1** in Porsolt's Forced Swim Test. Test apparatus represented a plastic vessel filled with water up to height of 18 cm at 20-22°C. Experiments were carried out in aged male mice of BALB/c line weighing 20-24 g.

Mice were placed in water and for 15 minutes duration of immobile hanging in water - so called behavioural despaire, which is considered to be a measure of depressively-like state, was registered. The last 5 minutes of the test were used for analysis. Automated computerized detection of motion with videosystem and Any-maze programm were used in the test. The ability of compounds **2.5(1)·HCl** and **3.6(1)·HCl** after 4 days injections in dose 1 mg/kg to decrease this index is regarded as evidence of their antidepressant action.

### Example 18.

Antidepressant action of compounds of the general formula 1 in test "Mice behavior in tail suspension test". Experiments were carried out in aged male mice of BALB/c line weighing 20-24 g.

Mice were suspended by tail with scotch tape on holder over horizontal surface at height of about 40 cm, and for 6 minutes of total duration of complete immobility episodes which is considered to be a measure of depressively-like state was registered. Automated computerized detection of motion with videosystem and Any-maze programm were used in test. The ability of compounds **2.5(1)·HCl** and **3.6(1)·HCl** after 4 days injections in dose of 0,1 mg/kg to decrease the duration of complete immobility of mice is regarded as evidence of their antidepressant action.

### Example 19.

Preparation of tablets comprising 100 mg of active ingredient. Starch (1600 mg), ground lactose (1600 mg), talk (400 mg) and compound **3.6.2(1)HCl** (1000 mg) were mixed together and pressed into bar. The resultant bar was comminuted into granules and sifted through sieve to collect granules of 14-16 mesh. The granules thus obtained were shaped into tablets of suitable form weighing 560 mg each.

### Example 20.

According to the invention capsules comprising compound **3.6(1)·HCl** (200 mg) were prepared by careful mixing of compound **3.6(1)·HCl** with lactose powder in ratio 2 : 1. The resultant powdery mixture was packed into gelatin capsules of suitable size by 200 mg to a capsule.

### Example 21.

Injection composition for intramuscular, intraperitoneal or subcutaneous injections could be prepared by mixing together compound **3.6(1)·HCl** (500 mg), chlorobutanol (300 mg), propylene glycol (2 ml), and injectable water (100 ml). The resultant solution was filtered and placed into 1 ml ampoules, which were sealed and sterilized in autoclave.

### Industrial applicability

The invention could be used in medicine, veterinary, biochemistry.

## Claims

1. Substituted tetrahydro-4H-thieno-pyrrolo[3,2-c]pyridines of the general formula **1,** geometrical isomers, mixtures of geometrical isomers, and pharmaceutically acceptable salts thereof, 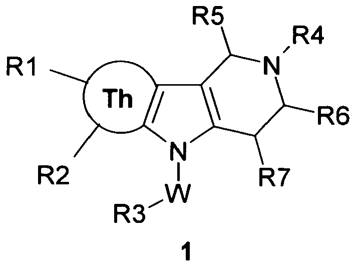 wherein:
Th represents annelated thienic cycle;
**W** represents ordinary bond (in this case R3 is bound directly to N-atom of pyrrole cycle), methylene, 1,2-ethylene, 1,2-vinyl, 1,2-ethynylene, 1,3-propanediyl or 1,3-propenylene, optionally substituted with hydroxy group;
**R1** and **R2** represent hydrogen, C₁-C₄alkyl, halogen or CH₂OH;
**R3** represents hydrogen, optionally substituted phenyl or optionally substituted azaheteroaryl;
**R4** represents C₁-C₄alkyl, CO₂C₂H₅ or CO₂C(CH₃)₃;
**R5, R6, R7** independently of each other represent hydrogen or C₁-C₄alkyl, or
**R5** and **R6** form together ethylene bridge, and R7 represents hydrogen, or R5 and
**R7** form together ethylene bridge, and R6 represents hydrogen.

2. Compounds according to claim 1 reprsenting substituted 5,6,7,8-tetrahydro-4*H-*[2',3':4,5]pyrrolo[3,2-c]pyridines of the general formula **2** and substituted **4**,5,6,7-tetrahydro-5*H*-[3',2':4,5]pyrrolo[3,2-c]pyridines of the general formula **3,** 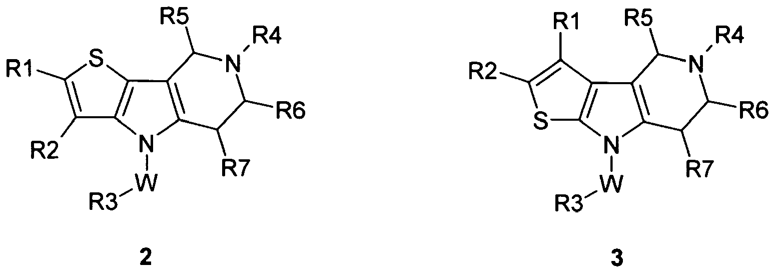 wherein:
**R1, R2, R3, R4, R5, R6, R7** and **W** have the above meanings.

3. Compounds according to claim 1 representing substituted tetrahydro-4*H*-thieno-pyrrolo[3,2-c]pyridines of the general formulas **1.1** - **1.14, 2.1 - 2.14, 3.1 - 3.14,** 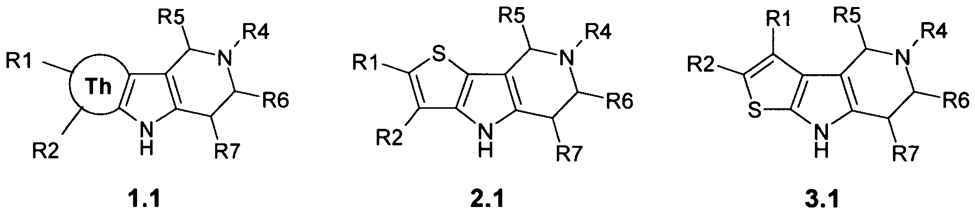 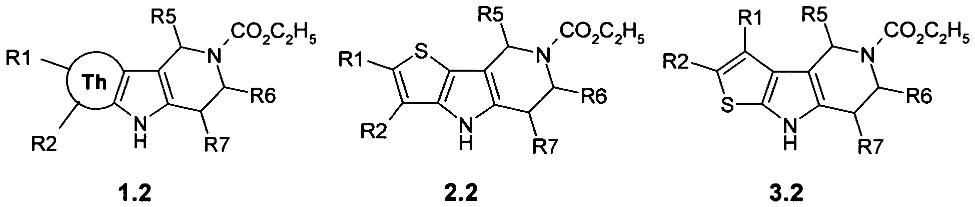 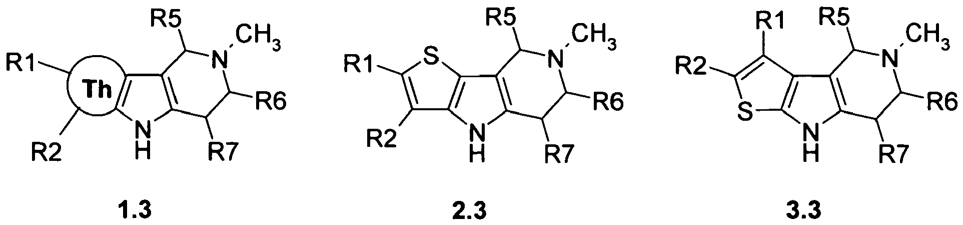 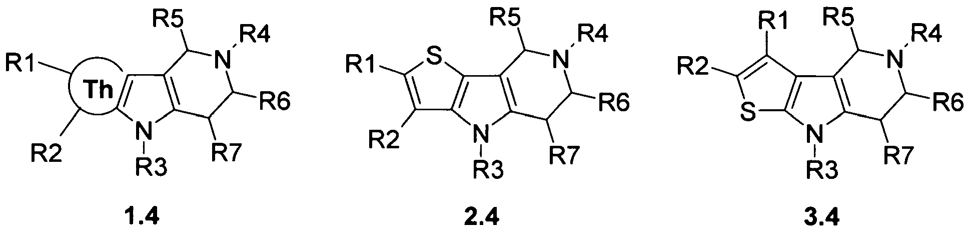 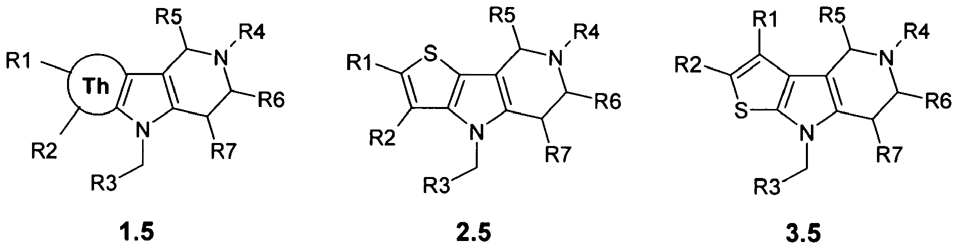 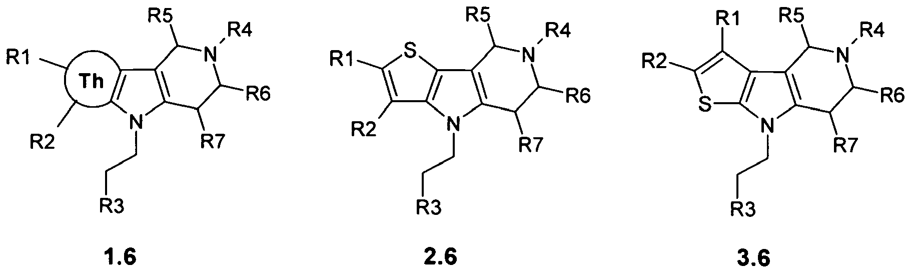 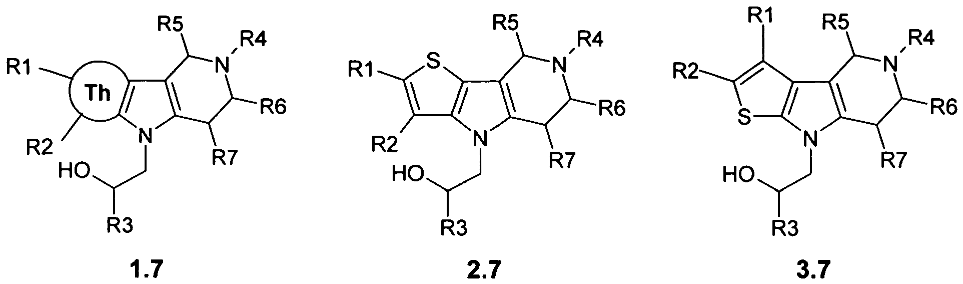 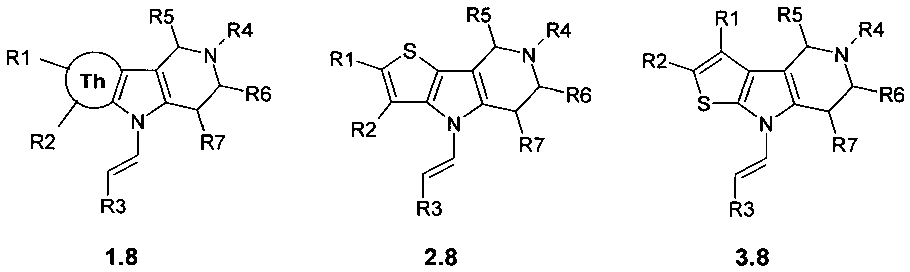 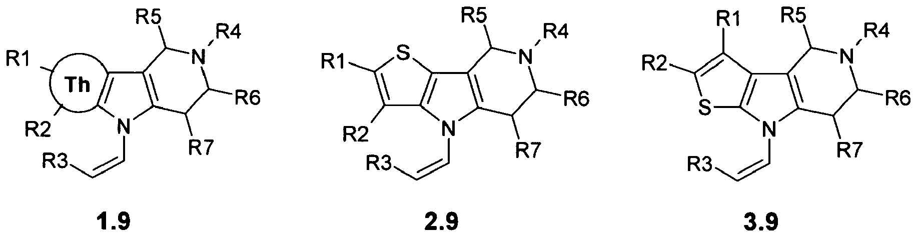 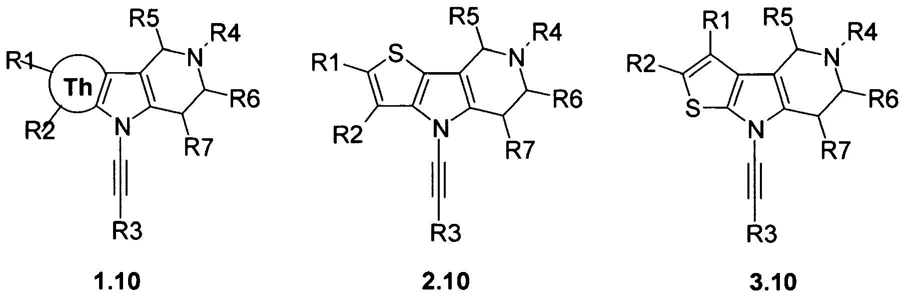 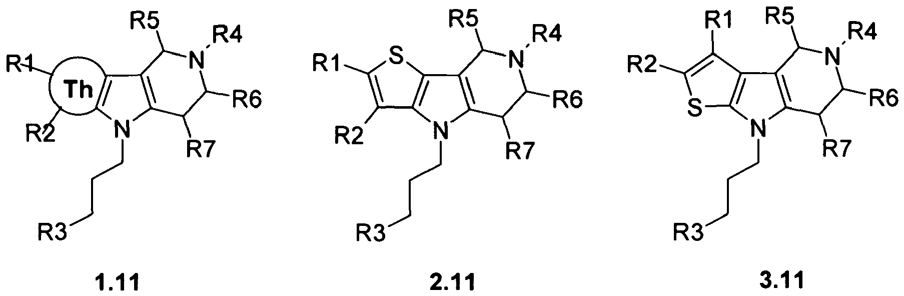 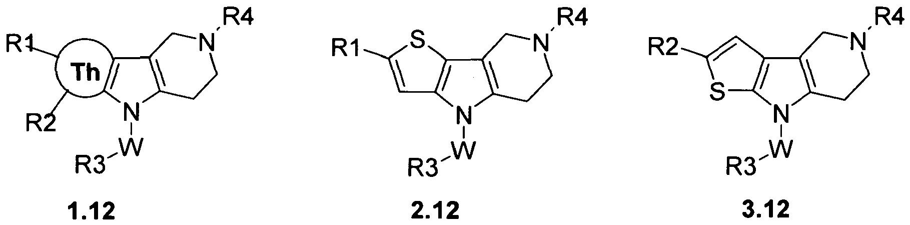 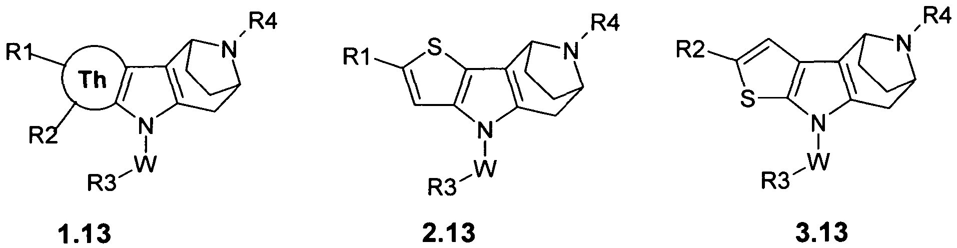 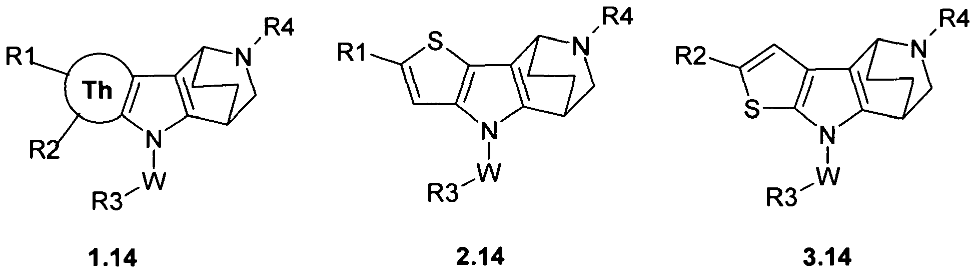 wherein:
**R1, R2, R3, R4, R5, R6, R7** and **W** have the above meanings.

4. Compounds according to claims 1, 2, selected from the group consisting of
2,7-dimethyl-4-(pyridyl-4-yl)-5,6,7,8-tetrahydro-4H-thieno[2',3':4,5]pyrrolo[3,2-c]pyridine **2.4(1),**
4-benzyl-2,7-dimethyl-5,6,7,8-tetrahydro-4H-thieno[2',3':4,5]pyrrolo[3,2-c]pyridine **2.5(1),**
2,7-dimethyl-4-phenethyl-5,6,7,8-tetrahydro-4H-thieno[2',3':4,5]pyrrolo[3,2-c]pyridine **2.6(1),**
(7-methyl-4-phenethyl-5,6,7,8-tetrahydro-4H-thieno[2',3':4,5]pyrrolo[3,2-c]pyridine-2-yl)-methanol **2.6(2),**
2,7-dimethyl-4-(3-fluorophenyl)-5,6,7,8-tetrahydro-4H-thieno[2',3':4,5]pyrrolo[3,2-c]pyridine **2.4(3),**
4-(3-methylbenzyl)-3-methyl-7-benzyl-5,6,7,8-tetrahydro-4H-thieno[2',3':4,5]pyrrolo[3,2-c]pyridine **2.5(3),**
3,5,7-trimethyl-4-[2-(pyridin-3-yl)ethyl]-5,6,7,8-tetrahydro-4H-thieno[2',3':4,5]pyrrolo[3,2-c]pyridine **2.6(4),**
2-(2,7-dimethyl-5,6,7,8-tetrahydro-4H-thieno[2',3':4,5]pyrrolo[3,2-c]pyridin-4-yl)-1-phenyl-ethanol **2.7(1),**
(*E*)-2,7-dimethyl-4-styryl-5,6,7,8-tetrahydro-4H-thieno[2',3':4,5]pyrrolo[3,2-c]pyridine **2.8(1),**
(*Z*)-2,7-dimethyl-4-styryl-5,6,7,8-tetrahydro-4H-thieno[2',3':4,5]pyrrolo[3,2-c]pyridine **2.9(1),**
2-(2,5,7-trimethyl-5,6,7,8-tetrahydro-4H-thieno[2',3':4,5]pyrrolo[3,2-c]pyridin-4-yl)-1-(6-methylpyridin-3-yl)-ethanol **2.7(3),**
(*E*)-2-methyl-7-ethyl-4-(3-fluorostyryl)-5,6,7,8-tetrahydro-4H-thieno[2',3':4,5]pyrrolo[3,2-c]pyridine **2.8(3),**
(*Z*)-2-methyl-7-ethyl-4-(3-fluorostyryl)-5,6,7,8-tetrahydro-4H-thieno[2',3':4,5]pyrrolo[3,2-c]pyridine **2.9(3),**
2,7-dimethyl-4-phenylethyl-5,6,7,8-tetrahydro-4H-thieno[2',3':4,5]pyrrolo[3,2-c]pyridine **2.10(1),**
2-methyl-7-(3-fluorobenzyl)-4-cinnamyl-5,6,7,8-tetrahydro-4H-thieno[2',3':4,5]pyrrolo[3,2-c]pyridine **2.11(1)**
(*E*)-3,7-dimethyl-4-[3-(*n*-tolyl)allyl]-5,6,7,8-tetrahydro-4H-thieno[2',3':4,5]pyrrolo[3,2-c]pyridine **2.11(2),**
(*E*)-2,7-dimethyl-4-[3-(3-chlorophenyl)allyl]-5,6,7,8-tetrahydro-4H-thieno[2',3': 4,5]pyrrolo[3,2 -c]pyridine **2.11(3),**
2,7-dimethyl-4-(3-phenylpropyl)-5,6,7,8-tetrahydro-4H-thieno[2',3':4,5]pyrrolo[3,2-c]pyridine **2.12(1),**
3,7-dimethyl-4-[3-(6-methylpyridin-3-yl)propyl)]-5,6,7,8-tetrahydro-4H-thieno[2',3': 4,5]pyrrolo[3,2 -c]pyridine **2.12(3),**
2,6,7,8-tetramethyl-4-[3-(3-chlorophenyl)propyl]-5,6,7,8-tetrahydro-4H-thieno[2',3': 4,5]pyrrolo[3,2-c]pyridine **2.12(4),**
7-methyl-4-(pyridyl-4-yl)-5,6,7,8-tetrahydro-4H-thieno[2',3':4,5]pyrrolo[3,2-c]pyridine **2.4(2),**
4-benzyl-7-methyl-2-chloro-5,6,7,8-tetrahydro-4H-thieno[2',3':4,5]pyrrolo[3,2-c]pyridine **2.5(2),**
7-methyl-4-phenethyl-2-chloro-5,6,7,8-tetrahydro-4H-thieno[2',3':4,5]pyrrolo[3,2-c]pyridine **2.6(3),**
2-(7-methyl-2-chloro-5,6,7,8-tetrahydro-4H-thieno[2',3':4,5]pyrrolo[3,2-c]pyridin-4-yl)-1-phenyl-ethanol **2.7(2),**
7-methyl-4-*π*-tolyl-2-chloro-5,6,7,8-tetrahydro-4H-thieno[2',3':4,5]pyrrolo[3,2-c]pyridine **2.4(4),**
4-(6-methylpyridin-3-ylmethyl)-6,7,8-trimethyl-3-chloro-5,6,7,8-tetrahydro-4H-thieno[2',3':4,5]pyrrolo[3,2-c]pyridine **2.5(4),**
7-methyl-4-[2-(pyridin-4-yl)ethyl]-2-chloro-5,6,7,8-tetrahydro-4H-thieno[2',3': 4,5]pyrrolo[3,2-c]pyridine **2.6(5),**
2-(5,7-dimethyl-2-chloro-5,6,7,8-tetrahydro-4H-thieno[2',3':4,5]pyrrolo[3,2-c]pyridin-4-yl)-1-(3-chlorophenyl)-ethanol **2.7(4),**
(*E*)-7-methyl-4-styryl-2-chloro-5,6,7,8-tetrahydro-4H-thieno[2',3':4,5]pyrrolo[3,2-c]pyridine **2.8(2),**
(*Z*)-7-methyl-4-styryl-2-chloro-5,6,7,8-tetrahydro-4H-thieno[2',3':4,5]pyrrolo[3,2-c]pyridine **2.9(2),**
7-methyl-4-phenylethynyl-2-chloro-5,6,7,8-tetrahydro-4H-thieno[2',3':4,5]pyrrolo[3,2-c]pyridine **2.10(2),**
(*E*)-7-methyl-4-[3-(*m*-tolyl)allyl]-3-chloro-5,6,7,8-tetrahydro-4H-thieno[2',3':4,5]pyrrolo[3,2-c]pyridine **2.11(4),**
(*E*)-7-methyl-4-(3-methylstyryl)-2-chloro-5,6,7,8-tetrahydro-4H-thieno[2',3':4,5]pyrrolo[3,2-c]pyridine **2.8(4),**
(*Z*)-7-methyl-4-(3-methylstyryl)-2-chloro-5,6,7,8-tetrahydro-4H-thieno[2',3':4,5]pyrrolo[3,2-c]pyridine **2.9(4),**
6,7,8-trimethyl-4-[(3-fluorophenyl)ethynyl]-2-chloro-5,6,7,8-tetrahydro-4H-thieno[2',3':4,5]pyrrolo[3,2-c]pyridine **2.10(3),**
(*E*)-7-methyl-4-[3-(*p*-tolyl)allyl]-2-chloro-5,6,7,8-tetrahydro-4H-thieno[2',3':4,5]pyrrolo[3,2-c]pyridine **2.11(5),**
7-methyl-4-(3-phenylpropyl)-2-chloro-5,6,7,8-tetrahydro-4H-thieno[2',3':4,5]pyrrolo[3,2-c]pyridine **2.12(2),**
7-methyl-4-[3-(6-methylpyridin-3-yl)propyl]-3-chloro-5,6,7,8-tetrahydro-4H-thieno[2',3': 4,5]pyrrolo[3,2-c]pyridine **2.12(5),**
7-methyl-4-[3-(3-fluorophenyl)propyl]-2-chloro-5,6,7,8-tetrahydro-4H-thieno[2',3': 4,5]pyrrolo[3,2-c]pyridine **2.12(6),**
2,10-dimethyl-4-(pyridin-3-ylmethyl)-4,5,6,7,8,9-hexahydro-6,9-epiminocyclohepta[b]thieno[2,3-d]pyrrole **2.13(1),**
9-benzyl-4-(3-fluorobenzyl)-2-methyl-5,6,7,8,-tetrahydro-4H-8,5-(epiminomethano)thieno[3,2-b]indole **2.14(1),**
2-chloro-9-methyl-4-(2-phenylethyl)-5,6,7,8-tetrahydro-4H-8,5-(epiminomethano)thieno[3,2-b]indole **2.14(2).**

5. Compounds according to claims 1, 2, selected from the group consisting of
2,5-dimethyl-8-(pyridin-4-yl)-4,5,6,7-tetrahydro-5H-thieno[3',2':4,5]pyrrolo[3,2-c] pyridine **3.4(1)**,
8-benzyl-2,5-dimethyl-4,5,6,7-tetrahydro-5H-thieno[3',2':4,5]pyrrolo[3,2-c]pyridine **3.5(1),**
2,5-dimethyl-8-phenethyl-4,5,6,7-tetrahydro-5H-thieno[3',2':4,5]pyrrolo[3,2-c]pyridine **3.6(1),**
(5-methyl-8-phenethyl-4,5,6,7-tetrahydro-5H-thieno[3',2':4,5]pyrrolo[3,2-c]pyridin-2-yl)-methanol **3.6(2),**
2,5-dimethyl-8-(3-fluorophenyl)-4,5,6,7-tetrahydro-5H-thieno[3',2':4,5]pyrrolo[3,2-c]pyridine **3.4(3)**,
8-(pyridin-4-ylmethyl)-3-methyl-5-benzyl-4,5,6,7-tetrahydro-5H-thieno[3',2':4,5]pyrrolo[3,2-c]pyridine **3.5(3),**
2,5,7-trimethyl-8-(4-chlorophenethyl)-4,5,6,7-tetrahydro-5H-thieno[3',2':4,5]pyrrolo[3,2-c]pyridine **3.6(4),**
2-(2,5-dimethyl-4,5,6,7-tetrahydro-5H-thieno[3',2':4,5]pyrrolo[3,2-c]pyridin-8-yl)-1-phenyl-ethanol **3.7(1)**,
(*E*)-2,5-dimethyl-8-styryl-4,5,6,7-tetrahydro-5H-thieno[3',2':4,5]pyrrolo[3,2-c]pyridine **3.8(1),**
(Z)-2,5-dimethyl-8-styryl-4,5,6,7-tetrahydro-5H-thieno[3',2':4,5]pyrrolo[3,2-c]pyridine **3.9(1),**
2-(2,5,7-trimethyl-4,5,6,7-tetrahydro-5H-thieno[3',2':4,5]pyrrolo[3,2-c]pyridin-8-yl)-1-*p*-tolyl-ethanol **3.7(3),**
(*E*)-2-methyl-5-ethyl-8-(pyridin-3-ylvinyl)-4,5,6,7-tetrahydro-5H-thieno[3',2':4,5]pyrrolo[3,2-c]pyridine **3.8(3),**
(*Z*)-2-methyl-8-(pyridin-3-ylvinyl)-4,5,6,7-tetrahydro-5H-thieno[3',2':4,5]pyrrolo[3,2-c] pyridine **3.9(3),**
2,5-dimethyl-8-phenylethynyl-4,5,6,7-tetrahydro-5H-thieno[3',2':4,5]pyrrolo[3,2-c]pyridine **3.10(1),**
(2-methyl-5-(3-fluorobenzyl)-8-cinnamyl-4,5,6,7-tetrahydro-5H-thieno[3',2':4,5]pyrrolo[3,2-c]pyridine **3.11(1),**
(2,5-dimethyl-8-[3-(*p*-tolyl)allyl]-4,5,6,7-tetrahydro-5H-thieno[3',2':4,5]pyrrolo[3,2-c] pyridine **3.11(2),**
(2,5-dimethyl-8-[3-(3-chlorophenyl)allyl]-4,5,6,7-tetrahydro-5H-thieno[3',2':4,5]pyrrolo[3,2-c]pyridine **3.11(3),**
(3,5-dimethyl-8-(3-phenylpropyl)-4,5,6,7-tetrahydro-5H-thieno[3',2':4,5]pyrrolo[3,2-c] pyridine **3.12(1),**
(2,5-dimethyl-8-[3-(6-methylpyridin-3-yl)propyl]-4,5,6,7-tetrahydro-5H-thieno[3',2':4,5]pyrrolo[3,2-c]pyridine **3.12(3),**
(3,4,5,6-tetramethyl-8-[3-(3-chlorophenyl)propyl]-4,5,6,7-tetrahydro-5H-thieno[3',2':4,5]pyrrolo[3,2-c]pyridine **3.12(4),**
5-methyl-8-(pyridin-4-yl)-2-chloro-4,5,6,7-tetrahydro-5H-thieno[3',2':4,5]pyrrolo[3,2-c]pyridine **3.4(2),**
8-benzyl-5-methyl-2-chloro-4,5,6,7-tetrahydro-5H-thieno[3',2':4,5]pyrrolo[3,2-c]pyridine **3.5(2),**
5-methyl-8-phenethyl-2-chloro-4,5,6,7-tetrahydro-5H-thieno[3',2':4,5]pyrrolo[3,2-c]pyridine **3.6(3),**
2-(5-methyl-2-chloro-4,5,6,7-tetrahydro-5H-thieno[3',2':4,5]pyrrolo[3,2-c]pyridin-8-yl)-1-phenyl-ethanol **3.7(2),**
5-methyl-8-(6-methylpyridin-3-yl)-2-chloro-4,5,6,7-tetrahydro-5H-thieno[3',2':4,5]pyrrolo[3,2-c]pyridine **3.4(4),**
8-(3-fluorobenzyl)-4,5,6-trimethyl-3-chloro-4,5,6,7-tetrahydro-5H-thieno[3',2':4,5]pyrrolo[3,2-c]pyridine **3.5(4),**
5-methyl-8-[2-(pyridin-4-yl)ethyl]-2-chloro-4,5,6,7-tetrahydro-5H-thieno[3',2':4,5]pyrrolo[3,2-c]pyridine **3.6(5),**
2-(5,7-dimethyl-3-chloro-4,5,6,7-tetrahydro-5H-thieno[3',2':4,5]pyrrolo[3,2-c]pyridin-8-yl)-1-(3-chlorophenyl)-ethanol **3.7(4),**
(*E*)-5-methyl-8-styryl-2-chloro-4,5,6,7-tetrahydro-5H-thieno[3',2':4,5]pyrrolo[3,2-c]pyridine **3.8(2),**
(*Z*)-5-methyl-8-styryl-2-chloro-4,5,6,7-tetrahydro-5H-thieno[3',2':4,5]pyrrolo[3,2-c]pyridine **3.9(2),**
5-methyl-8-phenylethynyl-2-chloro-4,5,6,7-tetrahydro-5H-thieno[3',2':4,5]pyrrolo[3,2-c]pyridine **3.10(2),**
(*E*)-5-methyl-8-[(3-m-tolyl)allyl]-3-chloro-4,5,6,7-tetrahydro-5H-thieno[3',2':4,5]pyrrolo[3,2-c]pyridine **3.11(4),**
(*E*)-5-methyl-8-(3-methylstyryl)-2-chloro-4,5,6,7-tetrahydro-5H-thieno[3',2':4,5]pyrrolo[3,2-c]pyridine **3.8(4),**
(*Z*)-5-methyl-8-(3-methylstyryl)-2-chloro-4,5,6,7-tetrahydro-5H-thieno[3',2':4,5]pyrrolo[3,2-c]pyridine **3.9(4),**
4,5,6-trimethyl-8-[(3-fluorophenyl)ethynyl]-2-chloro-4,5,6,7-tetrahydro-5H-thieno[3',2':4,5]pyrrolo[3,2-c]pyridine **3.10(3),**
(*E*)-5-methyl-8-[3-(6-methylpyridin-3-yl)allyl]-2-chloro-4,5,6,7-tetrahydro-5H-thieno[3',2':4,5]pyrrolo[3,2-c]pyridine **3.11 (5),**
(5-methyl-8-(3-phenylpropyl)-2-chloro-4,5,6,7-tetrahydro-5H-thieno[3',2':4,5]pyrrolo[3,2-c]pyridine **3.12(2),**
5-methyl-8-[(*p*-tolyl)propyl]-3-chloro-4,5,6,7-tetrahydro-5H-thieno[3',2':4,5]pyrrolo[3,2-c]pyridine **3.12(5),**
5-methyl-8-[(3-fluorophenyl)propyl]-2-chloro-4,5,6,7-tetrahydro-5H-thieno[3',2':4,5]pyrrolo[3,2-c]pyridine **3.12(6),**
9-benzyl-2,10-dimethyl-4,5,6,7,8,9-hexahydro-4,7-epiminocyclohepta[b]thieno[3,2-d]pyrrole **3.13(1),**
10-benzyl-8-(3-fluorobenzyl)-2-methyl-5,6,7,8-tetrahydro-4H-4,7-(epiminomethano)thieno[2,3-b]indole **3.14(1),**
2-chloro-10-methyl-8-(2-phenylethyl)-5,6,7,8-tetrahydro-4H-4,7-(epiminomethano)thieno[2,3-b]indole **3.14(2).**

6. Ligands exhibiting receptor activity towards α-adrenoceptors, dopamine receptors, histamine receptors and serotonin receptors representing substituted tetrahydro-4*H*-thieno-pyrrolo[3,2-c]pyridines of the general formula 1, their geometrical isomers, mixtures of their geometrical isomers, and their pharmaceutically acceptable salts according to any of claims 1 - 5.

7. Active component for pharmaceutical compositions and medicaments representing at least one of ligands according to claim 6.

8. Pharmaceutical composition for treatment and prophylaxis of conditions and diseases of CNS pathogenesis of which is associated with receptor activity of α-adrenoceptors, dopamine receptors, histamine receptors and serotonin receptors comprising pharmaceutically effective amount of active component according to claim 7.

9. Pharmaceutical composition according to claim 8 in the form of tablets, capsules or injections, placed in pharmaceutically acceptable packing.

10. Therapeutic kit for prophylaxis and treatment of various diseases of central nervous system at humans and animals comprising an active component according to claim 7 or pharmaceutical composition according to any of claims 8, 9.

11. Method for prophylaxis and treatment of diseases of central nervous system pathogenesis of which is associated with receptor activity of α-adrenoceptors, dopamine receptors, histamine receptors and serotonin receptors consisting in introduction to patient of an active component according to claim 7 or pharmaceutical composition according to claims 8 or 9, or therapeutic kit according to claim 10.

12. Substituted tetrahydro-4H-thieno-pyrrolo[3,2-c]pyridines of the general formula **1,** geometrical isomers, mixtures of geometrical isomers, and pharmaceutically acceptable salts thereof according to any of claims 1 - 5 for investigation of peculiarities of physiologically active compounds exhibiting biological activity towards α-adrenoceptors, dopamine receptors, histamine receptors and serotonin receptors.
